(19) Europäisches Patentamt
European Patent Office
Office européen des brevets

(11) EP 2 281 897 A2

(12) **EUROPEAN PATENT APPLICATION**

(43) Date of publication:
**09.02.2011 Bulletin 2011/06**

(21) Application number: **10186056.7**

(22) Date of filing: **17.11.2004**

(51) Int Cl.:
*C12N 15/869* (2006.01)
*C12N 7/01* (2006.01)
*C12N 15/11* (2006.01)
*A61K 35/76* (2006.01)
*C07K 14/82* (2006.01)
*A61K 48/00* (2006.01)

(84) Designated Contracting States:
**AT BE BG CH CY CZ DE DK EE ES FI FR GB GR HU IE IS IT LI LU MC NL PL PT RO SE SI SK TR**

(30) Priority: **17.11.2003 GB 0326798**
**03.02.2004 US 541308 P**

(83) **Declaration under Rule 32(1) EPC (expert solution)**

(62) Document number(s) of the earlier application(s) in accordance with Art. 76 EPC:
**04798619.5 / 1 694 852**

(71) Applicants:
- **Crusade Laboratories Limited**
  **Glasgow**
  **Strathclyde G51 4WF (GB)**
- **Sloan-Kettering Institute For Cancer Research**
  **New York,**
  **New York 10021 (US)**

(72) Inventors:
- **Brown, Susanne Moira**
  **Glasgow, Strathclyde G51 4WF (GB)**
- **Dunn, Paul**
  **Glasgow, Strathclyde G51 4WF (GB)**
- **Singh, Bhuvanesh**
  **New York, NY 11568 (US)**
- **Ganly, Ian**
  **New York, NY 10021 (US)**

(74) Representative: **Clegg, Richard Ian**
**Mewburn Ellis LLP**
**33 Gutter Lane**
**London**
**Greater London EC2V 8AS (GB)**

Remarks:
This application was filed on 01-10-2010 as a divisional application to the application mentioned under INID code 62.

(54) **Oncolytic mutant Herpes Simplex virus**

(57) An herpes simplex virus wherein the herpes simplex virus genome comprises nucleic acid encoding an antisense to the squamous cell carcinoma related oncogene (asSCCRO); and an herpes simplex virus wherein the herpes simplex virus genome comprises nucleic acid encoding a short interfering ribonucleic acid (siRNA) molecule that is capable of repressing or silencing expression of squamous cell carcinoma related oncogene (SCCRO) nucleic acid or polypeptide are disclosed together with methods for generation and applications of such viruses.

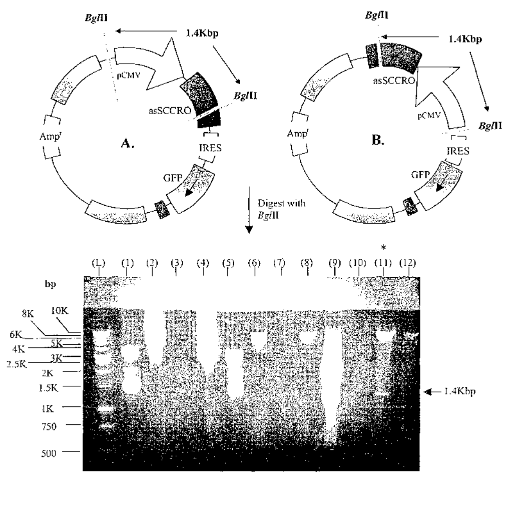


Figure 11

EP 2 281 897 A2

Printed by Jouve, 75001 PARIS (FR)

## Description

### Field of the Invention

The present invention relates to materials and methods relating to the squamous cell carcinoma related oncogene (SCCRO) and to mutant herpes simplex viruses.

### Background to the Invention

The herpes simplex virus (HSV) genome comprises two covalently linked segments, designated long (L) and short (S). Each segment contains a unique sequence flanked by a pair of inverted terminal repeat sequences. The long repeat (RL or $R_L$) and the short repeat (RS or $R_S$) are distinct.

The HSV ICP34.5 (also γ34.5) gene, which has been extensively studied[1,6,7,8], has been sequenced in HSV-1 strains F[9] and syn17+[3] and in HSV-2 strain HG52[4]. One copy of the ICP34.5 gene is located within each of the RL repeat regions. Mutants inactivating both copies of the ICP34.5 gene (i.e. null mutants), e.g. HSV-1 strain 17 mutant 1716[2] (HSV1716) or the mutants R3616 or R4009 in strain F[5], are known to lack neurovirulence, i.e. be avirulent, and have utility as both gene delivery vectors or in the treatment of tumours by oncolysis. HSV-1 strain 17 mutant 1716 has a 759bp deletion in each copy of the ICP34.5 gene located within the BamHI s restriction fragment of each RL repeat.

ICP34.5 null mutants such as 1716 are, in effect, first-generation oncolytic viruses. Most tumours exhibit individual characteristics and the ability of a broad spectrum first generation oncolytic virus to replicate in or provide an effective treatment for all tumour types is not guaranteed.

HSV 1716 is described in EP 0571410 and WO 92/13943 and has been deposited on 28 January 1992 at the European Collection of Animal Cell Cultures, Vaccine Research and Production Laboratories, Public Health Laboratory Services, Porton Down, Salisbury, Wiltshire, SP4 0JG, United Kingdom under accession number V92012803 in accordance with the provisions of the Budapest Treaty on the International Recognition of the Deposit of Microorganisms for the Purposes of Patent Procedure (herein referred to as the 'Budapest Treaty').

Squamous cell carcinoma of the head and neck afflicts an estimated 125,000 patients annually in Europe, North America and the Far East. Primary therapy for localized disease is surgery and adjuvant radiotherapy. Tumours recur in approximately one-third of patients. Once the cancer has recurred and/or metastasized, the patient is considered incurable. Combination chemotherapy induces responses in 30-50% of patients but there is no clear impact on survival. There remains an urgent need for more effective therapies [12,13].

There has been much interest in the use of novel therapies in this disease with particular focus on oncolytic viruses by direct intratumoural injection. The use of oncolytic viruses to selectively kill tumours while leaving normal cells unaffected is a very attractive concept as it has the potential to limit the toxicity which occurs with conventional modalities. Recent research has been carried out using intratumoural injections of a selectively replicating adenovirus (Onyx-015) for the local control of recurrent disease. Phase I/II studies involving virus alone and in combination with chemotherapy have produced encouraging results 14,15,16

Selectively replicating Herpes simplex viruses HSV may have better efficacy due to its more potent replication and oncolytic potential. HSV1716 [17] is a deletion mutant of herpes simplex virus which fails to synthesise the virulence protein ICP34.5. It has been shown that HSV1716 replicates in actively dividing cells but not in resting or terminally differentiated cells[18,19]. In vivo, HSV1716 administration has been carried out in mouse models of a range of cancers including melanoma, teratocarcinoma, glioma, medulloblastoma and mesothelioma. Animals showed improved survival and tumour regression following administration of HSV1716 [20,21,22,23,24,25] with no evidence of replication in normal tissue and no toxicity. HSV1716 has been used in Phase 1 trials in patients with glioblastoma multeforme (GBM)[26], melanoma and head and neck cancer. No toxicity has been experienced and patients who were seropositive pre HSV1716 seroconverted and evidence of virus replication contained within tumours has been obtained.

It has been shown that the novel oncogene SCCRO (Squamous cell carcinoma related oncogene (also called Oncoseq and sometimes called SCRO)) is amplified in 30% of mucosal squamous cell cancers and that overexpression is associated with poor prognosis in head and neck cancer patients.

The Oncoseq nucleic acid sequence was described in US 10/361,725 having publication number US 2004/0009541, published on 15 January 2004. This document is incorporated herein in its entirety by reference. A polynucleotide sequence including an open reading frame of 780 nucleotides for Oncoseq and the amino acid sequence of the 259-residue polypeptide encoded thereby was reported.

US 2004/0009541 describes Oncoseq alleles to be oncogenes identified in primary squamous cell carcinoma tissues as being colocalised with the highest gene duplication peak within the 3q26.3 locus using a positional cloning approach with Oncoseq being highly duplicated in those carcinomas. Overexpression of Oncoseq is described to be correlated with gene duplication, aggressive clinical behaviour and malignant transformation in vitro, making it a strong candidate as the target for 3q amplification. The gene is described to be highly oncogenic and to have a basic region-helix-loop-helix-leucine zipper motif, suggesting it may function as a transcription factor.

RNAi

RNAi utilises small double-stranded RNA molecules (dsRNA) to target messenger RNA (mRNA), the precursor molecule that cells use to translate the genetic code into functional proteins. During the natural process of RNAi, dsRNA is processed into short-interfering RNA (siRNA) duplexes of 21 nucleotides in length, and it is these molecules which recognise and target homologous (endogenous) mRNA sequences for enzymatic degradation (by complementary base-pair binding), resulting in gene silencing.

The advantages of RNAi over other gene-targeting strategies such as anti-sense oligonucleotides include its relative specificity, its enhanced efficacy (only nanomolar quantities of siRNA are required for efficient gene-silencing), and the fact that siRNA treatment feeds into a natural RNAi pathway that is inherent to all cells.

The success of gene-silencing by siRNA can be highly variable depending on the gene target and cell type being targeted.

## Summary of the Invention

The inventors have used plasmid RL1.dIRES-GFP to generate a shuttle vector, designated RL1.dCMV-asSCCRO-GFP, containing the human antisense squamous cell carcinoma related oncogene (SCCRO) arranged in an orientation downstream of a CMV IE promoter to produce antisense RNA transcripts for use in antisense therapeutic methods. Using this shuttle vector the inventors have provided a novel second generation mutant HSV, designated HSV1716/CMV-asSCCRO/GFP (also called HSV1716asSCCRO). The genome of this mutant HSV comprises the nucleic acid encoding heterologous (i.e. non-HSV originating) antisense SCCRO inserted at one or each ICP34.5 locus, disrupting the ICP34.5 protein coding sequence such that the ICP34.5 gene is non-functional and cannot express a functional ICP34.5 gene product. The generated HSV is capable of expressing an antisense RNA transcript under control of the CMV IE promoter which is capable of inhibiting the action of the SCCRO gene by binding to sense SCCRO nucleotide sequences, e.g. SCCRO mRNA or genomic SCCRO. This virus retains the oncolytic activity of HSV-1 strain 17 mutant 1716 and can be used in targeted antisense nucleotide delivery strategies and therapeutic methods.

In an alternative arrangement, instead of integrating a nucleic acid encoding an antisense, the inventors have integrated an siRNA in the genome of a herpes simplex virus. This siRNA is preferably heterologous to the herpes simplex virus and may be expressed from the herpes simplex virus genome. In one preferred embodiment the integrated nucleic acid encodes an siRNA capable of targeting and repressing or inhibiting expression of the functional SCCRO gene product. When expressed, the siRNA operates to silence, wholly or in part, expression of the functional SCCRO gene product.

The heterologous asSCCRO expressed by an herpes simplex virus according to the present invention may be useful in RNA based antisense therapeutic techniques for repression or silencing of the SCCRO gene product or of it's expressed function.

The siRNA expressed by an herpes simplex virus according to the present invention may be useful in siRNA based therapeutic techniques for tissue specific repression or silencing of the SCCRO gene product or of it's expressed function.

At its most general the present invention relates to (i) materials and methods relating to the squamous cell carcinoma related oncogene; and (ii) mutant herpes simplex viruses.

In one embodiment of the present invention, there is provided an attenuated replication competent HSV expressing antisense SCCRO, namely, HSV1716asSCCRO, which may be used in the treatment of squamous cell cancer, particularly head and neck squamous cell cancer.

Accordingly the present invention further provides a pharmaceutical composition comprising HSV1716asSCCRO and the use of such virus and/or composition in the treatment of cancer.

According to one aspect of the present invention there is provided an herpes simplex virus wherein the herpes simplex virus genome comprises nucleic acid encoding an antisense to the squamous cell carcinoma related oncogene (asSCCRO).

Said nucleic acid may encode an antisense to a mammalian squamous cell carcinoma related oncogene, more preferably an antisense to a human squamous cell carcinoma related oncogene.

In one arrangement said nucleic acid may encode a nucleotide sequence complementary to:

(i) the polynucleotide sequence of SEQ ID No.s 1 or 3 or its complement;
(ii) the mRNA transcript of SEQ ID No.s 1 or 3; or
(iii) to a fragment of said polynucleotide sequence, complement or mRNA transcript.

In another arrangement said nucleic acid may encode a nucleotide sequence having at least 60% sequence identity to the nucleotide sequence complementary to:

(i) the polynucleotide sequence of SEQ ID No.s 1 or 3 or its complement;
(ii) the mRNA transcript of SEQ ID No.s 1 or 3; or
(iii) to a fragment of said polynucleotide sequence or mRNA transcript.

More preferably said degree of sequence identity may be at least 70%, 80%, 90%, 95%, 96%, 97%, 98% or 99%. The fragment referred to at (iii) may comprise at least 20 nucleotides and may be limited to no more than 900 nucleotides. Identity of sequences is determined across the entire length of a given nucleotide sequence. Where sequences are of different length, sequence identity of the shorter sequence is determined over the entire length of the longer sequence.

In another arrangement said nucleic acid may be selected as one that hybridises to:

(i) the polynucleotide sequence of SEQ ID No.s 1 or 3 or its complement;
(ii) the mRNA transcript of SEQ ID No.s 1 or 3; or
(iii) to a fragment of said polynucleotide sequence or mRNA transcript

under high or very high stringency conditions.

The genome of Herpes simplex viruses according to the present invention may further comprises a regulatory sequence operably linked to said nucleic acid encoding an antisense to the squamous cell carcinoma related oncogene, wherein said regulatory sequence has a role in controlling transcription of said siRNA.

In a further aspect of the present invention there is provided an herpes simplex virus wherein the herpes simplex virus genome comprises nucleic acid encoding a short interfering ribonucleic acid (siRNA) molecule that is capable of repressing or silencing expression of the squamous cell carcinoma related oncogene (SCCRO) nucleic acid or polypeptide.

Said siRNA may repress or silence expression of a mammalian SCCRO, more preferably of a human SCCRO.

Said nucleic acid encoding siRNA may comprise a nucleic acid of between 10 and 50 nucleotides in length and may have the sequence of SEQ ID No.5 or the complement thereof.

In another arrangement said nucleic acid encoding siRNA may comprise a nucleic acid of between 10 and 50 nucleotides in length and may have at least 70%, 80%, 90%, 95%, 96%, 97%, 98% or 99% identity to SEQ ID No.5 or the complement thereof. Identity of sequences is determined across the entire length of a given nucleotide sequence. Where sequences are of different length, sequence identity of the shorter sequence is determined over the entire length of the longer sequence.

In another arrangement said nucleic acid encoding siRNA may be selected as one that hybridises to the nucleic acid of SEQ ID No. 5 or its complement under high or very high stringency conditions.

The genome of said herpes simplex virus may further comprise a regulatory sequence operably linked to said siRNA, wherein said regulatory sequence has a role in controlling transcription of said siRNA.

The nucleic acid encoding asSCCRO or said siRNA may be located in at least one RL1 locus of the herpes simplex virus genome. Suitably it may be located in, or overlap, at least one of the ICP34.5 protein coding sequences of the herpes simplex virus genome. The nucleic acid may be located in both (usually this is all) copies of the RL1 locus or ICP34.5 protein coding sequence.

The herpes simplex virus is preferably a mutant and may be a mutant of HSV-1 or HSV-2, more preferably of one of HSV-1 strains 17, F or HSV-2 strain HG52. The herpes simplex virus may be a further mutant of HSV-1 strain 17 mutant 1716.

In certain arrangements the herpes simplex virus may be a gene specific null mutant, such as an ICP34.5 null mutant.

In other arrangements the herpes simplex virus may lack at least one expressible ICP34.5 gene.

In yet another arrangement the herpes simplex virus may lack only one expressible ICP34.5 gene.

In yet another arrangement the herpes simplex virus may be non-neurovirulent.

In herpes simplex viruses of the present invention the nucleic acid encoding the asSCCRO or said siRNA may form part of a nucleic acid cassette permanently integrated in the herpes simplex virus genome, said cassette comprising nucleic acid encoding :

(a) said asSCCRO or said siRNA; and nucleic acid encoding:
(b) a ribosome binding site or a first regulatory nucleotide sequence; and
(c) a marker,

wherein the nucleic acid encoding said asSCCRO or siRNA is arranged upstream (5') of the ribosome binding site or first regulatory nucleotide sequence and the ribosome binding site or first regulatory nucleotide sequence is arranged upstream (5') of the marker. Said first regulatory sequence may have a role in controlling transcription of said marker.

A second regulatory nucleotide sequence may be located upstream (5') of the nucleic acid encoding asSCCRO or said siRNA, wherein the regulatory nucleotide sequence has a role in controlling and regulating transcription of the nucleic acid encoding the asSCCRO or siRNA and hence expression of the resulting transcript. The regulatory sequences may comprise selected promoter or enhancer elements known to the person skilled in the art, e.g. the CytoMegalovirus (CMV) or phosphoglycerokinase (PGK) promoters.

The components of the cassette are preferably arranged in a predetermined order.

In one preferred arrangement, the nucleic acid encoding the asSCCRO is arranged upstream (i.e. 5') of the ribosome binding site and the ribosome binding site is arranged upstream (i.e. 5') of the marker. During transcription a single transcript may be produced from the cassette comprising a first cistron comprising the asSCCRO and a second cistron encoding the marker wherein the ribosome binding site is located between the cistrons.

A transcription product of this cassette may be a bi- or poly- cistronic transcript comprising a first cistron encoded by the nucleic acid encoding the asSCCRO and a second cistron encoding the marker nucleic acid wherein the ribosome binding site is located between said first and second cistrons.

In another preferred arrangement, the nucleic acid encoding the siRNA is arranged upstream (i.e. 5') of a first regulatory nucleotide sequence and the first regulatory nucleotide sequence is arranged upstream (i.e. 5') of the marker.

The cassette may disrupt a protein coding sequence of the herpes simplex virus genome resulting in inactivation of the respective gene product.

Nucleic acid encoding a selected antisense DNA, that is DNA corresponding to a gene component (e.g. regulatory sequence, 5' UTR, 3'UTR or protein coding sequence) or fragment of a gene component, is inserted in the cassette in an orientation such that upon transcription an antisense RNA is obtained. Thus the expressed product of the cassette may ultimately be an antisense nucleic acid, preferably RNA.

One suitable ribosome binding site comprises a ribosome entry site permitting entry of a ribosome to the transcribed mRNA encoded by the nucleic acid of the cassette such that the ribosome binds to the translation start signal. Preferably, the ribosome entry site is an internal ribosome entry site (IRES), more preferably an encephalomyocarditis virus IRES, permitting cap-independent initiation of translation. The IRES thus enables translation of a coding sequence located internally of a bi- or poly- cistronic mRNA, i.e. of a cistron located downstream of an adjacent cistron on a single transcript.

Preferably the marker is a defined nucleotide sequence coding for a polypeptide which can be expressed in a cell line (e.g. BHK cells) infected with mutant herpes simplex virus into which the cassette has been recombined. The function of the marker is to enable identification of virus plaques containing mutant virus transformed with the cassette.

The marker is preferably a detectable marker, more preferably an expressible marker polypeptide or protein comprising at least the coding sequence for the selected polypeptide or protein. The nucleic acid encoding the marker may further comprise regulatory sequence upstream and/or downstream of the coding sequence having a role in control of transcription of the marker mRNA. Preferred markers include the Green Fluorescent Protein (GFP) protein coding sequence or gene, preferably the enhanced Green Fluorescent Protein (EGFP) protein coding sequence or gene.

In other arrangements the marker may comprise a defined nucleotide sequence which can be detected by hybridisation under high stringency conditions with a corresponding labelled nucleic acid probe, e.g. using a fluorescent- or radio-label.

The cassette may also comprise nucleic acid encoding a polyadenylation ("polyA") sequence, which sequence is preferably located downstream (3') of the nucleic acid encoding the marker. One preferred polyA sequence is the Simian Virus 40 (SV40) polyadenylation sequence. The preferred location of the polyA sequence within the cassette is immediately downstream (i.e. 3') of the marker.

By antisense nucleic acid is meant a nucleic acid:

(i) having substantial sequence identity to the nucleic acid formed by the sequence of complementary bases to the single strand of a target nucleic acid; and/or

(ii) a nucleic acid which hybridises to the target nucleic acid under intermediate, high or very high stringency conditions.

In accordance with aspects of the present invention, the target nucleic acid may be an SCCRO polynucleotide sequence (e.g. gene sequence), the polynucleotide coding sequence for the SCCRO polypeptide or protein, or a part/ fragment of the gene or polypeptide coding sequence. Thus, the antisense nucleic acid may be useful in binding the target nucleic acid (e.g. the SCCRO genomic coding sequence or mRNA transcript) and may be used as an inhibitor to prevent or disrupt the normal expression, activity, folding or binding of the target nucleic acid. The substantial sequence identity is preferably at least 50% sequence identity, more preferably one of at least 60, 70, 75, 80, 85, 90, 92, 94, 95, 96, 97, 98, 99 or 100% identity. Identity of sequences is determined across the entire length of a given nucleotide sequence. Where sequences are of different length, sequence identity of the shorter sequence is determined over the entire length of the longer sequence.

The antisense nucleic acid may comprise all or a fragment of the antisense to the squamous cell carcinoma related oncogene (asSCCRO), preferably it is an antisense to the human SCCRO.

The nucleic acid encoding the asSCCRO which may form part of the inserted cassette may encode a full length transcript of the antisense nucleotide sequence to the SCCRO. That full length antisense transcript may be a sequence complementary to one of the polynucleotide sequences of SEQ ID No.1 or SEQ ID No.3 or their complementary sequences. Alternatively, the nucleic acid may encode one or more fragments of the full length antisense transcript.

A fragment may comprise a nucleotide sequence encoding at least 10% of the corresponding full length sequence, more preferably the fragment comprises at least 20, 30, 40, 50, 60, 70, 80, 85, 90, 95, 96, 97, 98 or 99% of the corresponding full length sequence. Preferably, the fragment comprises at least, i.e. has a minimum length of, 20 nucleotides, more preferably at least 30, 40, 50, 100, 150, 200, 300, 400, 500, 600, 700, 800, 900, 1000, 1100, 1200, 1300, 1400, 1500, 1600, 1700, 1800, 1900 2000, 2100, 2200, 2300, 2400, 2500, 2600, 2700, 2800, 2900, 3000, 3100, 3200, 3300, 3400, 3500, 3600, 3700, 3800, 3900 or 4000 nucleotides. The fragment may have a maximum length, i.e. be no longer than, 20 nucleotides, more preferably no longer than 30, 40, 50, 100, 150, 200, 300, 400, 500, 600, 700, 800, 900, 1000, 1100, 1200, 1300, 1400, 1500, 1600, 1700, 1800, 1900 2000, 2100, 2200, 2300, 2400, 2500, 2600, 2700, 2800, 2900, 3000, 3100, 3200, 3300, 3400, 3500, 3600, 3700, 3800, 3900 or 4000. The fragment length may be anywhere between said minimum and maximum length.

In the case of an antisense to the SCCRO, a full length transcript comprises a minimum of the contiguous sequence of nucleotides forming an antisense strand to the corresponding complete nucleotide sequence encoding the full amino acid sequence of the SCCRO gene product or to is compliment. The complete nucleotide sequence of the SCCRO gene product may comprise the region of SEQ ID No.1 or SEQ ID No.3 respectively encoding the polypeptide of SEQ ID No. 2 or SEQ ID No.4.

Preferred antisense nucleic acids may single stranded and may be DNA or RNA.

Preferred siRNA may be single or double stranded and may comprise single stranded nucleic acids capable of forming duplex structures by stem-loop formation and self-binding of complementary nucleotides. Preferred siRNA may include RNA molecules having a sequence encoded by SEQ ID No. 5 or its complement and nucleic acids having a sequence identity of at least 60% to SEQ ID No. 5 or a complementary sequence thereof, and more preferably having at least 70, 80, 85, 90, 95% or 100% sequence identity. Identity of sequences is determined across the entire length of a given nucleotide sequence. Where sequences are of different length, sequence identity of the shorter sequence is determined over the entire length of the longer sequence.

Preferred siRNA or nucleic acid encoding preferred siRNA may comprise nucleotide sequences heterologous to the selected HSV strain being modified, i.e. the siRNA or nucleic acid sequence encoding the siRNA does not occur in or originate from the parental, unmodified wild-type, virus.

Furthermore Herpes simplex viruses according to aspects of the present invention may contain nucleic acid, encoding siRNA molecules, which hybridise with SEQ ID No 5 or its complement under very high, high or intermediate stringency conditions.

siRNA molecules encoded by nucleic acid molecules integrated in the genome of Herpes simplex viruses according to the present invention may be of any length, but preferred siRNA molecules are small and may comprise at least 10 nucleotides and no more than 50 nucleotides. Herpes simplex virus according to the present invention may encode an siRNA which is a fragment of the siRNA encoded by SEQ ID No.5. Particularly suitable siRNA will have a single strand length in the range 10 to 30 nucleotides and more suitably in the range 15 to 25 nucleotides. Selected siRNA molecules may be any of 15, 16, 17, 18, 19, 20, 21, 22, 23, 24 or 25 nucleotides in length. SiRNA molecules which fold to form their own duplex structures, e.g. by stem-loop formation will thus have an unfolded single strand length of about two times the number or range recited above and siRNA and nucleic acid encoding such siRNA may be particularly preferred.

Herpes simplex viruses according to aspects of the present invention may contain nucleic acid encoding siRNA molecules having one or more of these sequences.

Mutant herpes simplex viruses of the present invention may be generated by site directed insertion of a nucleic acid cassette into the viral genome, more preferably by homologous recombination. However, the viruses of the invention are not limited to Herpes simplex viruses obtained in this way.

In other aspects of the present invention herpes simplex viruses according to the present invention are provided for use in a method of medical treatment. Suitably they are provided for use in the treatment of disease. Preferably they are provided for use in the treatment of cancer. Suitably they may be provided for use in the oncolytic treatment of cancer/a tumour. The use of herpes simplex viruses according to the present invention in the manufacture of a medicament for the treatment of cancer is also provided.

In another aspect of the present invention medicaments comprising herpes simplex virus mutants according to the present invention for use in oncotherapy and methods of treating tumours comprising administering to a patient in need of treatment an effective amount of a mutant HSV or a medicament comprising or derived from such HSV are also provided. Methods of lysing or killing tumour cells in vitro or in vivo comprising the step of administering to a patient in need of treatment an amount of an Herpes simplex virus according to the present invention are also provided.

A medicament, pharmaceutical composition or vaccine comprising an Herpes simplex virus according to the present invention is also provided. The medicament, pharmaceutical composition or vaccine may further comprise a pharmaceutically acceptable carrier, adjuvant or diluent.

The present invention may also include the following aspects which may be provided in combination with any of the other aspects and features described.

In another aspect of the present invention there is provided an herpes simplex virus, wherein the genome of said virus comprises a nucleic acid sequence encoding an antisense to the squamous cell carcinoma related oncogene (asSCCRO) in at least one of the long repeat regions ($R_L$).

In another aspect of the present invention there is provided an herpes simplex virus, wherein the genome of said virus comprises a nucleic acid sequence encoding an antisense to the squamous cell carcinoma related oncogene (asSCCRO) and wherein the herpes simplex virus is non-neurovirulent.

In another aspect of the present invention there is provided an herpes simplex virus for use in the treatment of a tumour, wherein the genome of said virus comprises a nucleic acid sequence encoding an antisense to the squamous cell carcinoma related oncogene (asSCCRO) in at least one of the long repeat regions ($R_L$).

In another aspect of the present invention there is provided an herpes simplex virus for use in the treatment of a tumour, wherein the genome of said virus comprises a nucleic acid sequence encoding an antisense to the squamous cell carcinoma related oncogene (asSCCRO) and wherein the herpes simplex virus is non-neurovirulent.

In another aspect of the present invention the use of an herpes simplex virus, wherein the genome of said virus comprises a nucleic acid sequence encoding an antisense to the squamous cell carcinoma related oncogene (asSCCRO) in at least one of the long repeat regions ($R_L$), in the manufacture of a medicament for the treatment of cancer is provided.

In another aspect of the present invention the use of an herpes simplex virus, wherein the genome of said virus comprises a nucleic acid sequence encoding an antisense to the squamous cell carcinoma related oncogene (asSCCRO) and wherein the herpes simplex virus is non-neurovirulent, in the manufacture of a medicament for the treatment of cancer is provided.

In another aspect of the present invention there is provided a method for the treatment of a tumour comprising the step of administering to a patient in need of treatment an effective amount of an herpes simplex virus, wherein the genome of said virus comprises a nucleic acid sequence encoding an antisense to the squamous cell carcinoma related oncogene (asSCCRO) in at least one of the long repeat regions ($R_L$).

In another aspect of the present invention there is provided a method for the treatment of a tumour comprising the step of administering to a patient in need of treatment an effective amount of an herpes simplex virus, wherein the genome of said virus comprises a nucleic acid sequence encoding an antisense to the squamous cell carcinoma related oncogene (asSCCRO) and wherein the herpes simplex virus is non-neurovirulent.

Suitably, in the methods of treatment of a tumour the herpes simplex virus is capable of killing tumour cells.

In another aspect of the present invention there is provided a method of expressing in vitro or in vivo an antisense to the squamous cell carcinoma related oncogene (asSCCRO), said method comprising the step of infecting at least one cell or tissue of interest with a herpes simplex virus, wherein the genome of said virus comprises a nucleic acid sequence encoding asSCCRO in at least one of the long repeat regions ($R_L$), said asSCCRO operably linked to a transcription regulatory sequence.

In another aspect of the present invention there is provided a method of expressing in vitro or in vivo an antisense to the squamous cell carcinoma related oncogene (asSCCRO), said method comprising the step of infecting at least one cell or tissue of interest with a non-neurovirulent herpes simplex virus, wherein the genome of said virus comprises a nucleic acid sequence encoding asSCCRO, said asSCCRO operably linked to a transcription regulatory sequence.

In another aspect of the present invention there is provided an herpes simplex virus, wherein the genome of said virus comprises a nucleic acid sequence encoding a short interfering ribonucleic acid (siRNA) molecule that is capable of repressing or silencing expression of squamous cell carcinoma related oncogene (SCCRO) nucleic acid or polypeptide in at least one of the long repeat regions ($R_L$) .

In another aspect of the present invention there is provided an herpes simplex virus, wherein the genome of said virus comprises a nucleic acid sequence encoding a short interfering ribonucleic acid (siRNA) molecule that is capable of repressing or silencing expression of squamous cell carcinoma related oncogene (SCCRO) nucleic acid or polypeptide and wherein the herpes simplex virus is non-neurovirulent.

In another aspect of the present invention there is provided an herpes simplex virus for use in the treatment of a tumour, wherein the genome of said virus comprises a nucleic acid sequence encoding a short interfering ribonucleic acid (siRNA) molecule that is capable of repressing or silencing expression of squamous cell carcinoma related oncogene (SCCRO) nucleic acid or polypeptide in at least one of the long repeat regions ($R_L$) .

In another aspect of the present invention there is provided an herpes simplex virus for use in the treatment of a tumour, wherein the genome of said virus comprises a nucleic acid sequence encoding a short interfering ribonucleic acid (siRNA) molecule that is capable of repressing or silencing expression of squamous cell carcinoma related oncogene (SCCRO) nucleic acid or polypeptide and wherein the herpes simplex virus is non-neurovirulent.

In another aspect of the present invention the use of an herpes simplex virus, wherein the genome of said virus comprises a nucleic acid sequence encoding a short interfering ribonucleic acid (siRNA) molecule that is capable of repressing or silencing expression of squamous cell carcinoma related oncogene (SCCRO) nucleic acid or polypeptide in at least one of the long repeat regions ($R_L$), in the manufacture of a medicament for the treatment of cancer is provided.

In another aspect of the present invention the use of an herpes simplex virus, wherein the genome of said virus comprises a nucleic acid sequence encoding a short interfering ribonucleic acid (siRNA) molecule that is capable of repressing or silencing expression of squamous cell carcinoma related oncogene (SCCRO) nucleic acid or polypeptide and wherein the herpes simplex virus is non-neurovirulent, in the manufacture of a medicament for the treatment of cancer is provided.

In another aspect of the present invention there is provided a method for the treatment of a tumour comprising the step of administering to a patient in need of treatment an effective amount of an herpes simplex virus, wherein the genome of said virus comprises, in at least one of the long repeat regions ($R_L$), a nucleic acid sequence encoding a short interfering ribonucleic acid (siRNA) molecule that is capable of repressing or silencing expression of squamous cell carcinoma related oncogene (SCCRO) nucleic acid or polypeptide.

In another aspect of the present invention there is provided a method for the treatment of a tumour comprising the step of administering to a patient in need of treatment an effective amount of an herpes simplex virus, wherein the genome of said virus comprises a nucleic acid sequence encoding a short interfering ribonucleic acid (siRNA) molecule that is capable of repressing or silencing expression of squamous cell carcinoma related oncogene (SCCRO) nucleic acid or polypeptide and wherein the herpes simplex virus is non-neurovirulent.

Suitably, in the methods of treatment of a tumour the herpes simplex virus is capable of killing tumour cells.

In another aspect of the present invention there is provided a method of expressing in vitro or in vivo a short interfering ribonucleic acid (siRNA) molecule that is capable of repressing or silencing expression of squamous cell carcinoma related oncogene (SCCRO) nucleic acid or polypeptide, said method comprising the step of infecting at least one cell or tissue of interest with a herpes simplex virus, wherein the genome of said virus comprises a nucleic acid sequence encoding said siRNA in at least one of the long repeat regions ($R_L$), wherein said nucleic acid sequence encoding said siRNA is operably linked to a transcription regulatory sequence.

In another aspect of the present invention there is provided a method of expressing in vitro or in vivo a short interfering ribonucleic acid (siRNA) molecule that is capable of repressing or silencing expression of squamous cell carcinoma related oncogene (SCCRO) nucleic acid or polypeptide, said method comprising the step of infecting at least one cell or tissue of interest with a non-neurovirulent herpes simplex virus, wherein the genome of said virus comprises a nucleic acid sequence encoding said siRNA, wherein said nucleic acid sequence encoding said siRNA is operably linked to a transcription regulatory sequence.

siRNA according to the invention preferably repress the function of the squamous cell carcinoma related oncogene (SCCRO) protein.

In another aspect of the present invention a method is provided for repressing the cellular expression of the squamous cell carcinoma related oncogene (SCCRO) in vitro comprising the step of: in vitro, contacting a cell with an herpes simplex virus of the present invention or pharmaceutical composition containing such virus.

In one preferred aspect of the invention the herpes simplex virus is HSV1716/CMV-asSCCRO/GFP, deposited as 'HSV1716asSCCRO', in the name of Crusade Laboratories Limited having an address at Department of Neurology Southern General Hospital 1345 Govan Road Govan Glasgow G51 5TF Scotland on 19 May 2004 at the European Collection of Cell Cultures (ECACC), Health Protection Agency, Porton Down, Salisbury, Wiltshire, SP4 0JG, United Kingdom under accession number 04051901 in accordance with the provisions of the Budapest Treaty on the International Recognition of the Deposit of Microorganisms for the Purposes of Patent Procedure (herein referred to as the 'Budapest Treaty').

In yet another aspect of the present invention a cell, in vitro, in which expression of the squamous cell carcinoma related oncogene (SCCRO) protein or nucleic acid is repressed or silenced is provided. The cell may be a mammalian cell, preferably a human cell.

Suitably, the administration of said herpes simplex virus may comprise parenteral administration. Preferably administration of the herpes simplex virus is by injection, more preferably injection to the tumour which is to be treated. Alternatively injections may be intravenous.

In a further aspect of the present invention in vitro or in vivo methods are provided for delivery of nucleic acid encoding asSCCRO or siRNA to at least one cell or to a tissue of interest said method comprising the step of infecting said cell(s) or tissue with a herpes simplex virus according to the invention.

In another aspect of the present invention a method of making or producing a modified herpes simplex virus of the invention is provided comprising the step of introducing a nucleic acid sequence encoding asSCCRO or siRNA at a selected or predetermined insertion site in the genome of a selected herpes simplex virus.

As described, the nucleic acid sequence encoding the asSCCRO or siRNA may form part of a nucleic acid cassette which is inserted in the genome of a selected herpes simplex virus by homologous recombination. Whether part of a cassette or not, the site of insertion may be in any genomic location selected. One preferred insertion site is in one or both of the long repeat regions ($R_L$), and one copy of the cassette is preferably inserted in each copy of the long repeat ($R_L$) More preferably the insertion site is in at least one (preferably both) RL1 locus and most preferably it is inserted in at least one (preferably both) of the ICP34.5 protein coding sequences of the HSV genomic DNA. It is preferred that the insertion occurs in identical or substantially similar positions in each of the two repeat regions, RL1 loci or ICP34.5 protein coding sequences.

Insertion may be such as to produce a modified virus which is a non-neurovirulent mutant capable of expressing the encoded asSCCRO or siRNA upon transfection into mammalian, more preferably human, cells in vivo and in vitro. The non-neurovirulent mutant may be an ICP34.5 null mutant.

The nucleic acid cassette may be of any size, e.g. up to 5, 10, 15, 20, 25, 30, 35, 40, 45 or 50Kbp in length.

Preferably, the herpes simplex virus contains at least one copy of the nucleic acid encoding the asSCCRO or siRNA in each long repeat region ($R_L$), i.e. in the terminal and internal long repeat ($TR_L$ and $IR_L$) regions. In a preferred arrangement each exogenous sequence or cassette is located in an RL1 locus of the herpes simplex virus genome, more preferably in the DNA of the herpes simplex virus genome encoding the ICP34.5 gene or protein coding sequence. The herpes simplex virus thereby lacks neurovirulence.

The parent herpes simplex virus, from which a virus of the invention is derived may be of any kind, e.g. HSV-1 or HSV-2. In one preferred arrangement the herpes simplex virus is a variant of HSV-1 strain 17 and may be obtained by modification of the strain 17 genomic DNA. Suitable modifications include the insertion of the exogenous asSCCRO or siRNA nucleic acid sequences or exogenous/ heterologous cassette comprising said sequence into the herpes simplex virus genomic DNA. The insertion may be performed by homologous recombination of the exogenous nucleic acid sequence into the genome of the selected herpes simplex virus.

Although the non-neurovirulent phenotype of the herpes simplex virus of the invention may be the result of insertion of the exogenous nucleic acid sequence in the RL1 locus, herpes simplex viruses according to the present invention may be obtained by utilising a non-neurovirulent parent strain, e.g. HSV1716 deposited under the Budapest Treaty at the European Collection of Animal Cell Cultures (ECACC), Health Protection Agency, Porton Down, Salisbury, Wiltshire, United Kingdom under accession number V92012803, and inserting the exogenous nucleic acid sequence at another location of the genome by standard genetic engineering techniques, e.g. homologous recombination. In this aspect the location of the herpes simplex virus genome selected for insertion of the asSCCRO or siRNA nucleic acid sequence or cassette containing said sequence may be a neutral location.

Herpes simplex viruses of the present invention may be variants of a known 'parent' strain from which the herpes simplex virus of the invention has been derived. A particularly preferred parent strain is HSV-1 strain 17. Other parent strains may include HSV-1 strain F or HSV-2 strain HG52. A variant comprises an HSV in which the genome substantially resembles that of the parent, contains the asSCCRO or siRNA encoding nucleic acid sequence or cassette containing said sequence and may contain a limited number of other modifications, e.g. one, two or three other specific mutations, which may be introduced to disable the pathogenic properties of the herpes simplex virus, for example a mutation in the ribonucleotide reductase (RR) gene, the 65K trans inducing factor (TIF) and/or a small number of mutations resulting from natural variation, which may be incorporated naturally during replication and selection in vitro or in vivo. Otherwise the genome of the variant will be that of the parent strain.

Herpes simplex viruses of the invention may be used in a method of medical treatment. This may involve treatment of diseases associated with or involving the proliferation of cells, or cancers or tumours of any kind. Treatment may involve the selective lysis of dividing cells. This may be oncolysis, i.e. lysis of tumour cells. Tumours to be treated may be of any kind, may comprise cancers, neoplasms or neoplastic tissue and may be in any animal or human patient.

Herpes simplex viruses of the invention may be used in 'gene delivery' methods in vitro or in vivo. Non-neurovirulent herpes simplex viruses of the invention are expression vectors and may be used to infect selected cells or tissues in order to express the asSCCRO or siRNA encoded by the herpes simplex virus genome.

In one arrangement, cells may be taken from a patient, a donor or from any other source, infected with a herpes simplex virus of the invention, optionally screened for expression and/or function of the encoded asSCCRO or siRNA, and optionally returned/introduced to a patient's body, e.g. by injection.

Delivery of herpes simplex viruses of the invention to the selected cells may be performed using naked virus or by encapsulation of the virus in a carrier, e.g. nanoparticles, liposomes or other vesicles.

In vitro cultured cells, preferably human or mammalian cells, transformed with viruses of the present invention and preferably cells expressing the asSCCRO or siRNA as well as methods of transforming such cells in vitro with said viruses form further aspects of the present invention.

Cancer/tumour types to be treated may include primary and/or secondary (metastatic) tumours. These may be carcinomas of the head and/or neck. They may be squamous cell carcinomas, which may be of mucosal origin and may show a predilection for duplication of the 3q locus. Preferred squamous cell carcinomas to be treated may be those of the head and/or neck. Squamous cell carcinomas to be treated may include those originating from the lung, head neck, oesophagus and cervix.

Other tumour types which may be treated may be primary or secondary (metastatic) tumours. Tumours to be treated may be nervous or non-nervous system tumours. Nervous system tumours may originate either in the central or peripheral nervous system, e.g. glioma, medulloblastoma, meningioma, neurofibroma, ependymoma, Schwannoma, neurofibrosarcoma, astrocytoma and oligodendroglioma. Non-nervous system tumours may originate in any other non-nervous tissue, examples include melanoma, mesothelioma, lymphoma, hepatoma, epidermoid carcinoma, prostate carcinoma, breast cancer cells, lung cancer cells or colon cancer cells. HSV mutants of the present invention may be used to treat metastatic tumours of the central or peripheral nervous system which originated in a non-nervous system tissue.

In this specification, a mutant herpes simplex virus is a non-wild type herpes simplex virus and may be a recombinant herpes simplex virus. Mutant herpes simplex viruses may comprise a genome containing modifications relative to the wild type. A modification may include at least one deletion, insertion, addition or substitution.

Medicaments and pharmaceutical compositions according to aspects of the present invention may be formulated for administration by a number of routes, including but not limited to, parenteral, intravenous, intramuscular, intratumoural, oral and nasal. The medicaments and compositions may be formulated in fluid or solid (e.g. tablet) form. Fluid formulations may be formulated for administration by injection to a selected region of the human or animal body.

In this specification, non-neurovirulence is defined by the ability to introduce a high titre of virus (approx $10^6$ plaque forming units (pfu)) to an animal or patient[22, 23] without causing a lethal encephalitis such that the $LD_{50}$ in animals, e.g. mice, or human patients is in the approximate range of ≥106 pfu[21].

Where all copies of the ICP34.5 gene present in the herpes simplex virus genome (two copies are normally present) are disrupted such that the herpes simplex virus is incapable of producing a functional ICP34.5 gene product, the virus is considered to be an ICP34.5 null mutant.

A regulatory sequence (e.g. promoter) that is operably linked to a nucleotide sequence may be located adjacent to that sequence or in close proximity such that the regulatory sequence can effect and/or control expression of a product of the nucleotide sequence. The encoded product of the nucleotide sequence may therefore be expressible from that regulatory sequence.

SCCRO

The polynucleotide sequence of SEQ ID No.1, positions 43-918 and the polynucleotide of SEQ ID No.2 are disclosed in GenBank Accession No. AF456425 (GI:18700655) released to the public as of 19 February 2002.

A second Oncoseq (Oncoseq2) polypeptide is encoded by the polynucleotide sequence of SEQ ID No.3, which together with the polypeptide thereby encoded (SEQ ID No.4) are disclosed in GenBank Accession No. AF456426 (GI: 18700657) released to the public as of 19 February 2002.

The GenBank database may be accessed at http://www.ncbi.nlm.nih.gov/.

**Therapeutic strategies**

The following therapeutic strategies are provided by way of example only. The invention is not limited to a theory of operation of a given antisense or siRNA.

Antisense

Herpes simplex viruses according to the present invention may express an antisense nucleic acid, e.g. single stranded RNA that targets and binds, by complementary sequence binding, to the target mRNA thereby blocking translation of that mRNA and expression of the gene product.

Expressed antisense nucleic acid may also be arranged to bind sense genomic nucleic acid and inhibit transcription of a target nucleotide sequence.

siRNA

Herpes simplex viruses according to the present invention may encode nucleic acid designed such that on transcription an RNA having internal complementary sequence is provided and which may bind to form a short hairpin siRNA duplex having a stem-loop structure. Preferably, the hairpin siRNA mediates specific repression and/or silencing of gene expression by RNA interference.

Alternatively, two siRNA molecules may be encoded which are designed to bind by complementary sequence binding and form a functionally active duplex molecule.

Repression and silencing

siRNA and antisense molecules provided under the invention are designed to repress or silence the expression of a target nucleic acid, peptide, polypeptide or protein or to repress a function of such nucleic acid, peptide, polypeptide or protein.

A repression of expression results in a decrease in the quantity or expressed function of the target. For example, in a given cell the repression of SCCRO by expression of an siRNA or antisense may result in a decrease in either the quantity of the SCCRO gene product or the expressed function of the SCCRO gene product relative to an untreated cell.

Repression of a function may involve the decrease in transcription of an mRNA, or translation of a peptide or polypeptide.

Repression may be partial. Preferred degrees of repression are at least 50%, more preferably one of at least 60, 70, 80, 85 or 90%. A level of repression between 90% and 100% is considered a 'silencing' of expression or function.

**Hybridisation stringency**

In accordance with the present invention, nucleic acid sequences may be identified by using hybridization and washing conditions of appropriate stringency.

Complementary nucleic acid sequences will hybridise to one another through Watson-Crick binding interactions. Sequences which are not 100% complementary may also hybridise but the strength of the hybridisation usually decreases with the decrease in complementarity. The strength of hybridisation can therefore be used to distinguish the degree of complementarity of sequences capable of binding to each other.

The "stringency" of a hybridization reaction can be readily determined by a person skilled in the art.

The stringency of a given reaction may depend upon factors such as probe length, washing temperature, and salt concentration. Higher temperatures are generally required for proper annealing of long probes, while shorter probes may be annealed at lower temperatures. The higher the degree of desired complementarity between the probe and hybridisable sequence, the higher the relative temperature which can be used. As a result, it follows that higher relative temperatures would tend to make the reaction conditions more stringent, while lower temperatures less so.

For example, hybridizations may be performed, according to the method of Sambrook et al., ("Molecular Cloning, A Laboratory Manual, Cold Spring Harbor Laboratory Press, 1989) using a hybridization solution comprising: 5X SSC, 5X Denhardt's reagent, 0.5-1.0% SDS, 100 μg/ml denatured, fragmented salmon sperm DNA, 0.05% sodium pyrophosphate and up to 50% formamide. Hybridization is carried out at 37-42°C for at least six hours. Following hybridization, filters are washed as follows: (1) 5 minutes at room temperature in 2X SSC and 1% SDS; (2) 15 minutes at room temperature in 2X SSC and 0.1% SDS; (3) 30 minutes-1 hour at 37°C in 1X SSC and 1% SDS; (4) 2 hours at 42-65°C in 1X SSC and 1% SDS, changing the solution every 30 minutes.

One common formula for calculating the stringency conditions required to achieve hybridization between nucleic acid molecules is to calculate the melting temperature $T_m$ (Sambrook et al., 1989):

$$T_m = 81.5°C + 16.6Log\,[Na+] + 0.41(\%\ G+C) - 0.63\ (\%\ formamide) - 600/n$$

where n is the number of bases in the oligonucleotide.

As an illustration of the above formula, using [Na+] = [0.368] and 50% formamide, with GC content of 42% and an average probe size of 200 bases, the $T_m$ is 57°C. The $T_m$ of a DNA duplex decreases by 1 - 1.5°C with every 1% decrease in sequence complementarity.

Accordingly, nucleotide sequences can be categorised by an ability to hybridise to a target sequence under different hybridisation and washing stringency conditions which can be selected by using the above equation. The $T_m$ may be used to provide an indicator of the strength of the hybridisation.

The concept of distinguishing sequences based on the stringency of the conditions is well understood by the person skilled in the art and may be readily applied.

Sequences exhibiting 95-100% sequence complementarity may be considered to hybridise under very high stringency conditions, sequences exhibiting 85-95% complementarity may be considered to hybridise under high stringency conditions, sequences exhibiting 70-85% complementarity may be considered to hybridise under intermediate stringency conditions, sequences exhibiting 60-70% complementarity may be considered to hybridise under low stringency conditions and sequences exhibiting 50-60%% complementarity may be considered to hybridise under very low stringency conditions.

The invention includes the combination of the aspects and preferred features described except where such a combination is clearly impermissible or expressly avoided.

Aspects and embodiments of the present invention will now be illustrated, by way of example, with reference to the accompanying figures. Further aspects and embodiments will be apparent to those skilled in the art. All documents mentioned in this text are incorporated herein by reference.

## Brief Description of the Figures

**Figure 1.** Generation of plasmid RL1.dIRES-GFP from plasmids pNAT-IRES-GFP and RL1.del.

**Figure 2.** Agarose gel electrophoresis of *Hpa*I digested, CIP treated, RL1.del. RL1.del was digested with *Hpa*I. The digested DNA was then treated with Calf Intestinal Phosphatase (CIP) to prevent the vector re-annealing to itself in subsequent ligation reactions. A sample of the digested/CIP treated DNA was electrophoresed, beside a 1Kbp DNA ladder (Promega), on a 1% agarose gel. *Hpa*I linearises the vector at 8.6 Kbp.

Figure 3. Agarose gel electrophoresis of *Nsi*I/*Ssp*I digested pNAT-IRES-GFP (A) and purified/blunt-ended pCMV-NAT-IRES-GFP-PolyA (B). Four *Nsi*I/*Ssp*I digestions of pNAT-IRES-GFP were electrophoresed, beside a 1Kbp DNA Ladder (Promega) on a 1% agarose gel. The 5.4Kbp fragments (pCMV-NAT-IRES-GFP-PolyA) were purified from the gel. The purified DNA was blunt ended using Klenow polymerase and a sample electrophoresed on an agarose gel to check its concentration.

**Figure 4.** Identification of RL1.del clones containing the pCMV-NAT-IRES-GFP-PolyA insert. Ligation reactions were set up with the purified, blunt ended pCMV-NAT-IRES-GFP-PolyA fragment and *Hpa*I digested, CIP treated RL1.del. Bacteria were transformed with samples from the ligation reactions and plated out onto LBA ($Amp^r$) plates. Colonies were picked and plasmid DNA was extracted and digested with *Afl*II. Digested samples were electrophoresed, beside a 1Kbp DNA ladder (L) (Promega), on a 1% agarose gel.
*Clones 5 and 8 contained the pCMV-NAT-IRES-GFP-PolyA insert as two fragments of the predicted size - 4.8Kbp and 9.2Kbp - were generated from *Afl*II digestion. Clones without inserts would not be digested with *Afl*II as there is no AflII site in RL1.del. N.B. Inserts could have been cloned in two orientations, both of which were acceptable.

**Figure 5.** Determination of the orientation of pCMV-NAT-IRES-GFP-PolyA in clone 5 (RL1.dCMV-NAT-GFPb). pCMV-NAT-IRES-GFP-PolyA (blunt ended) could have been cloned into the *Hpa*I site of RL1.del in two orientations. To determine the orientation of the insert in clone 5, the plasmid was digested with *Xho*I and the digested DNA electrophoresed, beside a 1Kbp DNA ladder (Promega), on a 1% agarose gel. If the insert had been cloned in the orientation shown in A, two fragments of 10.2Kbp and 3.8Kbp would be generated from *Xho*I digestion. If it had been cloned in the opposite orientation (B), two fragments of 12.4Kbp and 1.6Kbp would be generated. The presence of two fragments of 10.2Kbp and 3.8Kbp in the gel confirmed that the insert had been cloned in the orientation shown in A.
*This *Xho*I site was present in the initial cloning vector (RL1.del), upstream of the *Hpa*I site into which pCMV-NAT-IRES-GFP-PolyA was cloned.

**Figure 6.** Removal of pCMV-NAT from clone 5 (A) and large scale plasmid preparation of RL1.dIRES-GFP (B). Four samples of clone 5 were digested with *Xho*I and electrophoresed, beside a 1Kbp DNA ladder (L) (Promega), on a 1% agarose gel (A). The larger fragment of DNA generated from this digestion (10.2Kbp) was purified from the gel and ligated back together, at the *Xho*I sites, to form a single *Xho*I site in a new plasmid, designated RL1.dIRES-GFP. A large-scale plasmid preparation was grown up and the preparation checked by digesting with *Xho*I. 1μl and 4μl of the digested DNA was electrophoresed, beside a 1Kbp DNA ladder (L) (Promega), on a 1% agarose gel (B). The DNA should produce a single fragment of 10.2Kbp when digested with *Xho*I. The ClaI, *Bgl*II, *Nru*I and *Xho*I sites of RL1.dIRES-GFP are all unique.
*Clone 5 is the RL1.del plasmid into which has been cloned the 5.4Kbp pCMV-NAT-IRES-GFP-PolyA fragment from pNAT-IRES-GFP.

**Figure 7.** Generation, detection and purification of ICP34.5 null HSV-1 expressing a gene product of interest.

**Figure 8.** Strategy used to clone pCMV-asSCCRO, from pUSEamp-asSCCRO, into RL1.dIRES-GFP. (1) Digest pUSEamp-asSCCRO with *Ssp*1 and *Xho*I and purify the 1.96Kbp pCMV-asSCCRO fragment; (2) Digest RL1.dIRES-GFP with *Bgl*II, blunt end using Klenow polymerase and treat with Calf Intestinal Phosphatase (CIP). (3) Clone the blunt ended pCMV-asSCCRO fragment (1.96Kbp) into *Bgl*II digested/blunt ended/CIP treated RL1.dIRES-GFP. (*pUSEamp-asSCCRO was provided by Memorial Sloan-Kettering Cancer Centre, New York.)

**Figure 9.** Agarose gel electrophoresis of *Bgl*II digested, blunt ended, CIP treated RL1.dIRES-GFP. RL1.dIRES.GFP was digested with *Bgl*II. The digested plasmid was then blunt ended using Klenow polymerase and treated with Calf Intestinal Phosphatase (CIP) to prevent the vector re-annealing to itself in subsequent ligation reactions. A sample of the digested/blunt ended/CIP treated DNA was electrophoresed, beside a 1Kbp DNA ladder (Promega), on a 1% agarose gel to check its concentration. pCMV-asSCCRO was subsequently cloned into this digested/CIP treated vector.

**Figure 10.** Agarose gel electrophoresis of *Ssp*I/*Xho*I digested pUSEamp-asSCCRO (A) and the purified pCMV-asSCCRO fragment (B). Four samples of pUSEamp-asSCCRO were digested with *Ssp*I and *Xho*I then electrophoresed, beside a 1Kbp DNA ladder (L) (Promega), on a 1% agarose gel. The 1.96Kbp fragments, consisting of DNA antisense to the squamous cell carcinoma related oncogene (asSCCRO) downstream of the CMV IE promoter (pCMV), were purified from the gel, blunt ended using Klenow polymerase, purified again and a sample of the purified DNA electrophoresed on an agarose gel to check its concentration.

**Figure 11.** Identification of RL1.dIRES-GFP clones containing the pCMV-asSCCRO insert. Ligation reactions were set up with the purified, blunt ended pCMV-asSCCRO fragment and *Bgl*II digested, blunt ended, CIP treated RL1.dIRES-GFP. Bacteria were transformed with samples from the ligation reactions and plated onto LBA ($Amp^r$) plates. Colonies were picked and plasmid DNA was extracted and digested with *Bgl*II. Digested samples were electrophoresed, beside a 1Kbp DNA ladder (L) (Promega), on a 1% agarose gel.
*Clone 11 contained the pCMV-asSCCRO insert as two fragments of the predicted size - 1.4Kbp and 10.8Kbp were generated from *Bgl*II digestion. Clones without the insert would not produce a fragment of 1.4Kbp when digested with *Bgl*II.

**Figure 12.** Determination of the orientation of pCMV-asSCCRO in clone 11. The presence of an *Nru*I site, -320bp into the cloned pCMV-asSCCRO fragment, was utilized to determine the orientation of pCMV-asSCCRO. Clone 11 was digested with *Nru*I and electrophoresed, beside a 1Kbp DNA ladder (L) (Promega), on a 1% agarose gel. If pCMV-asSCCRO was in the desired orientation (A), *Nru*I digestion would produce a fragment of 1.64Kbp. If in the opposite orientation (B), no 1.64Kbp fragment would be generated from this digestion. The presence of a fragment at 1.64Kbp in the gel confirmed that pCMV-asSCCRO was in the desired orientation. (*This *Nru*I site was already present in the initial cloning vector (i.e. RL1.dIRES-GFP)).

**Figure 13.** Agarose gel electrophoresis of ScaI digested clone 11 (A) and HSV1716/CMV-asSCCRO/GFP virus titre (B). Clone 11 (RL1.dCMV-asSCCRO-GFP) was digested with ScaI, the digested DNA purified and 5μl electrophoresed, beside a 1Kbp DNA ladder (Promega), on a 1% agarose gel, to check its concentration. 80% confluent BHK cells were then co-transfected with 10μl $HSV17^+$ DNA and an appropriate volume of the remaining digested clone 11. The cells were incubated at 37°C for 3 days until cpe was evident. Recombinant viral plaques were picked under the fluorescent microscope, purified and a virus stock, named HSV1716/CMV-asSCCRO/GFP, grown up. HSV1716/CMV-asSCCRO/GFP was titrated on BHK cells.

**Figure 14.** Cytotoxicity assay for cell lines SCC15 and 584 after infection with HSV1716 or HSV1716asSCCRO at MOI of 1pfu/cell and 5pfu/cell.

**Figure 15.** Cytotoxicity assay for cell lines 1483 and 1986 after infection with HSV1716 or HSV1716asSCCRO at MOI of 1 pfu/cell and 5pfu/cell.

**Figure 16.** Cytotoxicity assay for cell line 1186 and 1386 after infection with HSV1716 or HSV1716asSCCRO at MOI of 1 pfu/cell and 5pfu/cell.

**Figure 17.** Viral proliferation assays for head and neck squamous cell carcinoma cell lines after infection with HSV1716 or HSV1716asSCCRO at MOI 1pfu/cell.

**Figure 18.** Infectivity assay- gfp expression 6 hours post infection with 1716gfp virus.

**Figure 19.** Western blot results of the cell line SCC15 showing downregulation of SCCRO protein at 12 hours with HSV1716asSCCRO but not in 584.

**Figure 20.** Nude mice xenograft growth curves in SCC15 and 584 following single intratumoural injection of HSV1716 or HSV1716asSCCRO.

**Figure 21.** Nude mice xenograft growth curves in SCC15 following single intratumoural injection of PBS, HSV1716 or HSV1716asSCCRO.

**Figure 22.**

(A) SEQ ID No. 1- a Human SCCRO nucleic acid sequence. Also showing the amino acid sequence of the encoded polypeptide (an SCCRO gene product);
(B) SEQ ID No. 2 - Amino acid sequence of the polypeptide encoded by SEQ ID No.1;
(C) SEQ ID No. 3 - a Human SCCRO nucleic acid sequence. Also showing the amino acid sequence of the encoded polypeptide (an SCCRO gene product);
(D) SEQ ID No. 4 - Amino acid sequence of the polypeptide encoded by SEQ ID No.3.

**Figure 23**. **(A)** DNA nucleotide sequence encoding the siRNA construct designed to target expression of the SCCRO gene (SEQ ID No. 5); and (B) nucleotide sequence encoding control siRNA (SEQ ID No 6). Sequences either side of the central nucleotides are respectively complimentary enabling the transcribed RNA to form a hairpin structure (stem-loop) by binding of complementary nucleotides.

**Detailed Description of the Best Mode of the Invention**

Specific details of the best mode contemplated by the inventors for carrying out the invention are set forth below, by way of example. It will be apparent to one skilled in the art that the present invention may be practiced without limitation to these specific details.

<u>Vectors Useful for Generation of Herpes Simplex virus Mutants</u>

Mutant herpes simplex viruses of the invention may be generated by use of nucleic acid vectors.
One such vector useful for generation of mutant herpes simplex viruses according to the present invention is a nucleic acid vector comprising, consisting or consisting essentially of:

first and second nucleotide sequences corresponding to nucleotide sequences flanking an insertion site in the genome of a selected herpes simplex virus; and a cassette located between said first and second nucleotide sequences comprising nucleic acid encoding:

a) one or a plurality of insertion sites; and
b) a ribosome binding site; and
c) a marker.

Another vector useful for generation of mutant herpes simplex viruses according to the present invention is a nucleic acid vector comprising, consisting or consisting essentially of:

first and second nucleotide sequences corresponding to nucleotide sequences flanking an insertion site in the genome of a selected herpes simplex virus; and a cassette located between said first and second nucleotide sequences comprising nucleic acid encoding:

a) one or a plurality of insertion sites; and
b) a first regulatory nucleotide sequence; and
c) a marker.

The first and second nucleotide sequences may correspond to nucleotide sequences flanking an insertion site formed in, or comprising all or a part of, the ICP34.5 protein coding sequence of the genome of a selected herpes simplex virus.
The cassette may comprise a plurality of insertion sites, each insertion site preferably formed by nucleic acid encoding a specific restriction endonuclease site ('restriction site'). Together the restriction sites may form a multiple cloning site (MCS) comprising a series of overlapping or distinct restriction sites, preferably a series of distinct restriction sites comprising one or more of the ClaI, BglII, NruI, XhoI restriction sites.

The encoded components of the cassette may be arranged in a predetermined order. In one arrangement, the one or plurality of insertion sites is/are arranged upstream (i.e. 5') of the ribosome binding site/first regulatory sequence and the ribosome binding site/first regulatory sequence is arranged upstream (i.e. 5') of the marker.

The first and second nucleotide sequences may comprise nucleotide sequences having identity to regions of the genome surrounding the insertion site in the selected herpes simplex virus (the 'viral insertion site'). These sequences enable the cassette to be incorporated at the viral insertion site by homologous recombination between the first and second nucleotide sequences and their respective corresponding sequences in the viral genome.

Thus the first and second nucleotide sequences are flanking sequences for homologous recombination with corresponding sequences of a selected viral genome, such homologous recombination resulting in insertion of the cassette at the viral insertion site.

The first and second nucleotide sequences may correspond to nucleotide sequences flanking an insertion site in the RL1 locus of the HSV genome, more preferably in the ICP34.5 protein coding sequence of the HSV genome.

The first and second nucleotide sequences may each be at least 50bp in length, more preferably at least 100, 150, 200, 300, 400, 500, 600, 700, 800, 900, 1000, 1100, 1200, 1300, 1400, 1500, 1600, 1700, 1800, 1900 2000, 2100, 2200, 2300, 2400, 2500, 2600, 2700, 2800, 2900, 3000, 3100, 3200, 3300, 3400, 3500, 3600, 3700, 3800, 3900 or 4000bp in length. Each of the first and second nucleotide sequences may have at least 50% sequence identity to their corresponding sequence in the viral genome, more preferably at least 60%, 70%, 75%, 80%, 85%, 90%, 92%, 94%, 95%, 96%, 97%, 98% 99% or 100% identity. Identity of sequences is determined across the entire length of a given nucleotide sequence. Where sequences are of different length, sequence identity of the shorter sequence is determined over the entire length of the longer sequence.

The first and second nucleotide sequences may be characterised by the ability of one strand of a given sequence to hybridise with the corresponding single-stranded complement of the HSV genome under varying hybridisation stringency conditions. Suitably, the first and second nucleotide sequences will hybridise with their corresponding complement under very low, low or intermediate stringency conditions, more preferably at high or very high stringency conditions.

The viral insertion site is the position between the genomic nucleotide sequences corresponding to the first and second nucleotide sequences of the vector (the 'genomic' and 'vector flanking sequences' respectively) at which homologous recombination will occur and may be predetermined by selection of the vector flanking sequences. Where the genomic flanking sequences are immediately adjacent, the insertion site is the position between the peripheral and immediately adjacent bases of the two genomic flanking sequences, such that insertion of the cassette separates the genomic flanking sequences. Where the genomic flanking sequences are separated by one or a plurality of bases in the viral genome, the insertion site is formed by said one or a plurality of bases which are excised from the genome by the homologous recombination event.

The position of the viral insertion site may be accurately selected by careful selection and construction of the vector flanking sequences. Accordingly, the vector may be constructed such that homologous insertion of the cassette results in disruption of a chosen protein coding sequence and inactivation of the respective gene product or such that the cassette is inserted at a nonprotein coding region of the viral genome. The complete genome sequences of several herpes simplex virus strains have been reported and are publicly available. The complete genome sequence for HSV-1 strain 17syn+ was reported by Dolan et al[3] (incorporated herein by reference) and the complete genome sequence of HSV-2 strain HG52 was reported by Dolan et al[4] (incorporated herein by reference) and is available from the EMBL database under accession code Z86099. Using this information, the vector of the present invention may preferably be designed for use in generating mutant HSV-1 (e.g. in strain 17 or F) or mutant HSV-2 (e.g. in strain HG52).

The first and second nucleotide sequences (vector flanking sequences) may each comprise sequence corresponding to the RL terminal repeat region of the genome of the selected HSV (e.g. HSV-1 strains 17 or F or HSV-2 strain HG52). The vector flanking sequences may comprise, consist or consist essentially of nucleotide sequences of the RL repeat region which flank the ICP34.5 protein coding sequence. In flanking the ICP34.5 coding sequence, one or both of the selected sequences may, in the corresponding HSV genome, overlap, i.e. extend into, the ICP34.5 protein coding sequence or one or both sequences may be selected so as to not overlap the ICP34.5 protein coding sequence. In a similar manner, the selected sequences may be chosen to overlap completely or partially other important encoded signals, e.g. transcription initiation site, polyadenylation site, defined promoters or enhancers. In this preferred arrangement the insertion site will thus comprise all or a part of the ICP34.5 protein coding sequence and/or be such that the inserted cassette disrupts the ICP34.5 protein coding sequence.

The vectors described, comprising first and second nucleotide sequences corresponding to regions of the RL repeat region flanking and/or overlapping the ICP34.5 protein coding sequence, may be used in the generation of ICP34.5 null mutants wherein all or a portion of the ICP34.5 protein coding sequence is excised and replaced during the homologous recombination event such that both copies of the ICP34.5 coding sequence are disrupted. The recombination may result in an insertion of nucleic acid within the ICP34.5 protein coding sequence thereby disrupting that sequence. In that case, successfully transformed virus are thus mutants incapable of generating the ICP34.5 active gene product from at least one copy, and preferably from both copies, of the ICP34.5 gene.

Successfully transformed virus are thus mutants incapable of generating the ICP34.5 active gene product.

Each component of the cassette may be positioned substantially adjacent the neighbouring component such that a single bicistronic transcript comprising or consisting essentially of the mRNA encoding the nucleotide sequence of interest, ribosome binding site and marker is obtainable.

The vectors described may further comprise, consist, or consist essentially of a nucleic acid encoding a selectable marker such as a polypeptide or protein conferring antibiotic resistance e.g. kanamycin resistance or ampicillin resistance.

The vectors described are preferably DNA vectors, particularly dsDNA vectors. The vector may be provided as a linear or circular (plasmid) DNA vector. The vector preferably contains nucleotide sequences, e.g. restriction endonuclease site(s), permitting transition between the two forms by use of DNA ligation and restriction materials (e.g. enzymes) and techniques known to the person skilled in the art. To achieve homologous recombination with a selected HSV, the vector is preferably provided in linear form.

One such vector provided by the inventors is plasmid RL1.dIRES-GFP deposited in the name of Crusade Laboratories Limited having an address at Department of Neurology Southern General Hospital 1345 Govan Road Govan Glasgow G51 5TF Scotland on 03 September 2003 at the European Collection of Cell Cultures (ECACC), Health Protection Agency, Porton Down, Salisbury, Wiltshire, SP4 0JG, United Kingdom under accession number 03090303 in accordance with the provisions of the Budapest Treaty on the International Recognition of the Deposit of Microorganisms for the Purposes of Patent Procedure (herein referred to as the 'Budapest Treaty').

RL1.dIRES-GFP provides a platform for generating a plurality of 'shuttle vectors' which can exploit the process of homologous recombination to transfer a nucleotide sequence of interest (downstream of a selected promoter) into the disabling RL1 locus of HSV-1, generating easily identifiable, oncolytic, ICP34.5 null HSV-1 mutants expressing the products of the nucleotide sequence of interest, e.g. an RNA transcript or a polypeptide, and GFP. RL1.dIRES-GFP thus provides for ease of generation and purification of ICP34.5 null HSV.

RL1.dIRES-GFP is a useful vector for making second-generation oncolytic viruses having enhanced cytotoxic potential and which may express the product(s) of selected gene(s) to enhance the oncolytic and/or therapeutic effect of the administered virus.

The RL1.dIRES-GFP plasmid incorporates a multi-cloning sequence (MCS), upstream of an internal ribosome entry site (IRES), the GFP gene and SV40 polyadenylation sequences flanked by HSV-1 RL1 sequences. Incorporation of the encephalomyocarditis virus IRES (EMCV IRES) permits translation of two open reading frames from a single transcribed mRNA.

Following generation of a specific shuttle vector by cloning of the nucleotide sequence of interest (and the selected promoter) into RL1.dIRES-GFP, recombinant HSV-1 expressing the desired nucleic acid transcript or protein, can be generated and purified within 2 weeks. This compares with 2-3 months using prior art protocols.

In the ICP34.5 null HSV generated using the RL1.dIRES-GFP plasmid provided by the inventors transcription of both the nucleotide sequence of interest and GFP as a single transcript is controlled by the same promoter upstream of the nucleotide sequence of interest, the transcribed IRES directing cap-independent translation of GFP. The generated ICP34.5 null HSV are non-neurovirulent. By modifying the RL1.dIRES-GFP plasmid to incorporate appropriate flanking sequences surrounding the cassette other gene-specific HSV null mutants expressing GFP can be generated.

RL1.dIRES-GFP is promoterless, thus enabling a promoter of choice to be incorporated in the homologously recombined shuttle vector for controlling expression of the nucleotide sequence of interest from the inserted cassette.

Plasmid RL1.dIRES-GFP or modified plasmid shuttle vectors thereof further comprising nucleotide sequence encoding a nucleic acid transcript or polypeptide of interest may be provided in isolated or purified form.

The vector may be a variant of plasmid RL1.dIRES-GFP.

As the plasmid RL1.dIRES-GFP is designed for tandem expression of a sequence of interest and the marker gene encoding green fluorescent protein (GFP). The sequence of interest is cloned into RL1.dIRES-GFP along with its promoter (e.g. CMV) such that the promoter drives transcription of an mRNA for the sequence of interest along with the IRES-GFP. Translation results in expression of the GFP from the internal ribosomal entry site and the gene of interest and promoter must be cloned into RL1.dIRES-GFP in the correct orientation to achieve this. There are a number of instances where this tandem expression arrangement may be unsuitable and a variation of the cassette design is favourable.

One example is the expression of siRNAs as short hairpin RNAs using RNA polIII promoters such as H1 or U6. These promoters are unable to drive the additional tandem expression of the IRES-GFP as the RNApolIII expression cassette is designed only to produce short transcripts. Additionally, sequences of interest derived from genomic DNA with strong mRNA shut-off signals in their 3' untranslated regions may not support IRES-GFP expression.

Thus in some cases a cassette may be provided in which the sequence of interest and marker are expressed separately from independent promoters.

One variant contains a cassette in which the ribosome binding site of plasmid RL1.dIRES-GFP is replaced with a regulatory nucleotide sequence, preferably a strong, constitutive promoter such as the Phosphoglycerokinase promoter. The marker is thereby expressed under the control of this (the 'first') regulatory sequence. The nucleotide sequence of interest (e.g. antisense or siRNA) is expressed under the control of a second regulatory sequence upstream (5') of the nucleotide sequence of interest, e.g. the CMV promoter. This vector variant is particularly suitable for expression of siRNA where a weak promoter may be used for expression of the siRNA molecule or the nucleic acid encoding the siRNA may have a strong termination signal making it difficult to produce a single bi- or poly-cistronic transcript containing the transcribed siRNA and marker sequence. In this arrangement the transformed virus containing the cassette integrated in the viral genome produces two separate transcripts under the control of the first and second promoters.

This cassette was constructed in the following manner. The 1.3kbp blunt-ended EcoRI/AflII fragment that contains the PGK promoter/GFP gene was obtained by restriction digestion followed by Klenow treatment from the vector pSNRG and cloned into the RL1-del vector cut with the restriction enzyme NruI that generates blunt ends. Successful insertion of the PGK/GFP DNA was confirmed by BamHI digestion and the orientation of the inserted DNA identified using the unique XhoI site in RL1-de1 and the BsrGI site at the 3' end of PGK/GFP. Plasmids with PGK/GFP in both forward and reverse orientation were obtained and the plasmids were designated RL1-dPGK/GFPfor and RL1-dPGK/GFPrev. Expression of GFP was confirmed in BHK cells transfected with the forward and reverse orientation plasmids.

Thus, sequences of interest along with their own promoters (although it is preferred that the PGK promoter is not also used for this purpose) can then be cloned into either RL1-dPGK/GFPfor or RL1-dPGK/GFPrev in either orientation using the remaining unique BglII, XhoI or HpaI unique restriction enzyme sites. The resulting plasmid can be used to derive recombinant HSV in which the marker GFP gene and the gene of interest are expressed independently from their own promoters.

The vectors described may be constructed for use in generating engineered HSV-1 or HSV-2 by insertion of a nucleic acid cassette through a mechanism of homologous recombination between nucleotide sequences flanking the cassette and corresponding sequences in the selected herpes simplex virus genome.

The vectors described may comprise and have use as:

i) gene delivery (gene therapy) vectors for delivery of a selected nucleotide sequence, e.g. antisense nucleic acid or siRNA, to a specific locus of the HSV genome; and/or

ii) expression vectors for expression of the delivered nucleotide sequence of i) from the HSV genome under the control of a selected regulatory element; and/or

iii) vectors for the generation of HSV gene-specific null mutants wherein the cassette is inserted at a selected genomic location to disrupt the protein coding sequence of a selected HSV gene such that the gene product is inactive in the resultant mutant virus.

The vectors described may be used in the manufacture of engineered gene specific HSV null mutants, i.e. HSV mutants incapable of expressing an active gene product of a selected gene. They may be used in the manufacture of engineered viruses which express a selected protein from only one gene copy the other gene copy being disrupted or modified such that it cannot express a functional gene product. Such vectors may also be used in the manufacture of a medicament, preferably comprising said gene specific HSV null mutant, for use in treating cancer and tumours, preferably by the oncolytic treatment of the tumour.

The vectors described may also be used in the manufacture of engineered HSV mutants wherein the genome of the mutant HSV comprises a nucleotide sequence which has been inserted in the HSV genome by homologous recombination of the cassette such that the nucleotide sequence is arranged to be transcribed from the HSV genome under the control of a regulatory element e.g. promoter, preferably a regulatory element forming part of the inserted cassette, to produce an antisense transcript or siRNA. Preferably the antisense nucleotide sequence is an exogenous/ heterologous (i.e. non-HSV originating) sequence. Such vectors may be used in the manufacture of a medicament, preferably comprising the engineered HSV mutant, for use in the treatment of disease, including the oncolytic treatment of tumours.

The vectors described may also be used in the manufacture of an engineered HSV mutant wherein the genome of the mutant HSV comprises a nucleotide sequence which has been inserted in a protein coding sequence of the HSV genome by homologous recombination of the cassette such that the mutant HSV is incapable of expressing the active gene encoded by said protein coding sequence and wherein the inserted nucleotide sequence is expressed under the control of a regulatory element to produce an antisense transcript or siRNA. Preferably, the regulatory element forms part of the cassette. Such vectors may be used in the manufacture of a medicament, preferably comprising the engineered HSV mutant, for use in the treatment of disease, including the oncolytic treatment of tumours.

The vectors described may be used to generate mutant HSV by inserting the cassette into the genome of a selected HSV, the method of generation may comprise providing a vector described above, where the vector is a plasmid, linearising the vector; and co-transfecting a cell culture with the linearised vector and genomic DNA from said HSV.

The co-transfection may be carried out under conditions effective for homologous recombination of said cassette into an insertion site of the viral genome.

The method may further comprise one or more of the steps of:

1) screening said co-transfected cell culture to detect mutant HSV expressing said marker; and/or
2) isolating said mutant HSV; and/or
3) screening said mutant HSV for expression of the nucleotide sequence of interest or the RNA or polypeptide thereby encoded; and/or
4) screening said mutant HSV for lack of an active gene product; and/or
5) testing the oncolytic ability of said mutant HSV to kill tumour cells in vitro.

**Example 1**

**Construction of plasmid RL1.dIRES-GFP**

General Approach

Plasmid RL1.dIRES-GFP was generated in three stages, illustrated in Figure 1.

1. The DNA sequences containing the CMV IE promoter (pCMV), the NAT gene, the internal ribosome entry site (IRES), the GFP reporter gene and the SV40 polyadenylation sequences were excised from pNAT-IRES-GFP using *Nsi*I and *Ssp*I and purified.

2. The purified pCMV-NAT-IRES-GFP-PolyA DNA fragment was cloned into RL1.del to form a new plasmid designated RL1.dCMV-NAT-GFP.

3. The pCMV-NAT DNA sequences of RL1.dCMV-NAT-GFP were excised using *Xho*I and the remainder of the plasmid religated to form a novel plasmid designated RL1.dIRES-GFP. This novel plasmid contained a multi-cloning site (all sites shown are unique) upstream of an IRES, the GFP gene and the SV40 polyA sequences all within the HSV-1 RL1 flanking sequences. Recombinant ICP34.5 null HSV-1, expressing a gene of interest in the RL1 locus, can be generated by cloning the gene of interest (downstream of a suitable promoter) into the multi-cloning site and co-transfecting BHK cells with the plasmid and HSV-1 DNA. Recombinant virus expressing the target gene can be identified using GFP fluorescence.

Removal of the CMV promoter and noradrenaline transporter gene (pCMV-NAT) from RL1.dCMV-NAT-GFP, followed by religation of the remainder of the plasmid, resulted in a novel plasmid (RL1.dIRES-GFP) containing a multi-cloning site (MCS), upstream of the encephalomyocarditis virus internal ribosome entry site (EMCV IRES), the GFP reporter gene and the SV40 PolyA sequences, all within RL1 flanking sequences. This novel arrangement of DNA sequences or 'smart cassette' allows ICP34.5 null HSV-1, expressing a gene of interest in the RL1 locus, to be easily generated by simply inserting the desired transgene (downstream of a suitable promoter) into the MCS and co-transfecting BHK cells with the plasmid and HSV-1 DNA. The IRES situated between the GFP gene and the MCS permits expression of two genes from the same promoter and so recombinant virus expressing the gene of interest also expresses GFP and can therefore be easily identified under a fluorescence microscope and purified.

Materials and Methods

1μg of RL1.del* was digested with 10units HpaI (Promega) in a suitable volume of 10x buffer (Promega) and nuclease free water (Promega) at 37°C for 16hrs. The digested plasmid was then purified using the QIAquick PCR purification kit (Qiagen), treated with 10 units of Calf Intestinal Phosphatase (Promega), in a suitable volume of 10x CIP buffer and nuclease free water for 4hrs at 37°C, before being purified again using a Qiaquick PCR purification kit. 5μl of the purified DNA was electrophoresed on a 1% agarose gel to check its concentration (Figure 2).

4 x 1μg of pNAT-IRES-GFP** was digested with 10 units of *Nsi*I and 10 units of *Ssp*I in a suitable volume of 10x buffer (Promega) and nuclease free water (Promega) at 37°C for 16hrs. The reaction mixture was electrophoresed in a 1% agarose gel for 1hr at 110 volts. The 5.4Kbp DNA fragment consisting of the CMV IE promoter (pCMV), upstream of the noradrenaline transporter gene (NAT), the encephalomyocarditis virus internal ribosome entry site (IRES), the gene for green fluorescent protein (GFP) and the SV40 polyadenylation sequences (SV40 Poly A), was excised using a sterile scalpel and the DNA purified from the gel using a QIAquick Gel Extraction kit (Qiagen). The eluted DNA was blunt ended using 3 units Klenow Polymerase (Promega) in accordance with the manufacturers instructions and the DNA purified using a QIAquick PCR purification kit (Qiagen). 5μl of the purified DNA fragment was electrophoresed on a 1% agarose gel to check its concentration (Figure 3).

Ligation reactions were carried out in small eppendorf tubes containing 5 units T4 DNA Ligase (Promega), a suitable volume of 10X DNA Ligase Buffer (Promega), nuclease free water (Promega) and various volumes of the HpaI digested/CIP treated RL1.del and blunt ended pCMV-NAT-IRES-GFP-SV40 Poly A DNA, at 16°C overnight. Competent JM109 bacterial cells (Promega) were then transformed with various aliqouts of the ligation reactions***. Colonies formed on the plates were picked, had their plasmid DNA extracted using a Qiagen Plasmid Mini kit and screened for inserts using *Afl*II (New England Biolabs) restriction enzyme analysis. Plasmid DNA containing the insert would produce two fragments of 4.8Kbp and 9.2Kbp following digestion with *Afl*II. Two clones (clone 5 and 8) contained the insert (Figure 4). The orientation of the insert in clone 5 (RL1.dCMV-NAT-GFP) was determined using *Xho*I restriction enzyme analysis (Figure 5).

To generate RL1.dIRES-GFP from clone 5, the CMV-NAT portion of the CMV-NAT-IRES-GFP-SV40 PolyA insert was removed by digesting 4 x 500ng of clone 5 with 10 units of *Xho*I in a suitable volume of buffer and water (Promega), overnight at 37°C. The digested DNA was electrophoresed on a 1% agarose gel at 110 volts for 1hr (Figure 6A). The 10.2Kbp fragment consisting of the IRES, the GFP gene, the SV40 PolyA sequences and RL1 flanking sequences in a pGEM3Zf(-) (Promega) backbone, was excised using a sterile scalpel and the DNA purified from the gel using a QIAquick Gel Extraction kit.

Ligation reactions were performed in small eppendorf tubes containing 100ng - 500ng purified DNA, 3 units T4 DNA Ligase (Promega), a suitable volume of 10X DNA Ligase Buffer (Promega) and nuclease free water (Promega) overnight at 16°C. Competent JM109 bacterial cells (Promega) were then transformed with various aliquots of the ligation reactions***. Colonies formed on the plates were picked, had their plasmid DNA extracted using a Qiagen Plasmid Mini kit and screened using *Xho*I (Promega) restriction enzyme analysis. Colonies containing plasmid DNA from which CMV-NAT had been removed would produce one fragment of 10.2Kbp when digested with *Xho*I. Several positive clones were found, one was isolated, and a large-scale plasmid preparation undertaken using Promega's Wizard Plus Maxipreps kit. The large-scale plasmid preparation was checked by digesting with *Xho*I (Figure 6B). This plasmid DNA was subsequently named 'RL1.dIRES-GFP'.

Plasmid RL1.dIRES-GFP has been deposited in the name of Crusade Laboratories Limited having an address at Department of Neurology Southern General Hospital 1345 Govan Road Govan Glasgow G51 5TF Scotland on 03 September 2003 at the European Collection of Cell Cultures (ECACC), Health Protection Agency, Porton Down, Salisbury, Wiltshire, SP4 0JG, United Kingdom under accession number 03090303 in accordance with the provisions of the Budapest Treaty.

*RL.1.del*

*RL1.del was provided by Dr.E.McKie and is the pGEM-3Zf(-) plasmid (Promega) into which has been cloned an HSV-1 fragment (123459-129403) consisting of the RL1 gene and its flanking sequences. The 477bp *Pfl*MI-*Bst*EII fragment of the RL1 gene (125292-125769) has been removed and replaced with a multi-cloning site (MCS) to form RL1.del.

*pNAT-IRES-GFP*

** pNAT-IRES-GFP was supplied by Dr. Marie Boyd (CRUK Beatson Laboratories) and is the pIRES2-EGFP plasmid (BD Biosciences Clontech) into which she has cloned the bovine noradrenaline transporter (NAT) gene (3.2Kbp), at the NheI and *Xho*I sites.

****Transformation of Bacterial Cells*

10μl of a glycerol E.coli stock was added to 10ml 2YT medium in a 20ml griener tube. This was placed in a 37°C shaking incubator for 16-24hrs until a saturated culture was obtained. 1ml of this culture was then added to 100ml of 2YT in a 500ml sterile glass bottle and placed in the 37°C shaking incubator for 3hrs. The bacterial cells were pelleted by centrifugation at 2,000rpm for 10 minutes (Beckman). The cells were then resuspended in 1/10$^{th}$ volume of transformation and storage buffer (10mM $MgCl_2$, 10mM $Mg(SO)_4$, 10% (w/v) PEG 3,500, 5% (v/v) DMSO). The cells were placed on ice for between 10 minutes and 2hrs, after which time they were considered competent for transformation.

1-10μl of DNA was mixed with 100μl of competent bacteria in eppendorf tubes, and the tubes placed on ice for 30 minutes. After this, the samples were 'heat shocked' by incubating the tubes in a 42°C water bath for exactly 45 seconds before placing them on ice for a further 2 minutes. 1ml of L-Broth was added, the tube inverted 2-3 times, and the bacteria incubated for 1hr at 37°C. 100μl of the transformed bacteria was plated out onto L-broth agar plates containing 100μg/ml of the appropriate antibiotic (usually ampicillin or kanamycin). Plates were allowed to dry at room temperature, before incubating in an inverted position at 37°C overnight.

**Example 2**

**Generation of ICP34.5 null HSV-1 expressing a gene product of interest and GFP using plasmid RL1.dIRES-GFP.**

General Approach

Generation of ICP34.5 null HSV-1 expressing a gene product of interest requires insertion of nucleotide sequence encoding the gene product (polypeptide) of interest and desired promoter at the MCS of RL1.dIRES.GFP followed by co-transfection of BHK cells with the linearised plasmid, containing the gene of interest, and HSV DNA. Following homologous recombination viral plaques expressing GFP are identified. Figure 7 illustrates the method steps involved.

Referring to Figure 7A plasmid DNA, containing the gene of interest and the desired promoter (X), is digested with restriction endonucleases to release the promoter/gene fragment.

The promoter/gene fragment is purified and cloned into the multi-cloning site (MCS) of RL1.dIRES.GFP forming a shuttle vector suitable for generating oncolytic HSV-1 (Figure 7B). This vector contains HSV-1 sequences that flank the essential RL1 gene but does not contain the RL1 gene. The plasmid also contains the gene for Green Fluorescent Protein (GFP) downstream of an internal ribosome entry site (IRES). The IRES permits expression of both the gene of interest and the GFP gene from the same upstream promoter.

BHK cells are then co-transfected with linearised RL1.dIRES.GFP, now containing the gene of interest, and HSV-1 DNA (Figure 7C). Following homologous recombination, designer virus, expressing the gene of interest and GFP, is generated and can be distinguished from wild type virus (also generated but not expressing GFP) under a fluorescence microscope.

Viral plaques, expressing GFP (and hence the gene of interest), are picked under the fluorescence microscope and purified until all wild-type HSV-1 has been removed. The recombinant HSV-1 is considered 100% pure when all the viral plaques are expressing GFP (Figure 7D).

Once the recombinant virus is completely pure, an isolated plaque is picked and a highly concentrated stock is grown and titrated (Figure 7E). Oncolytic HSV-1, expressing a gene product of interest from a selected promoter, is then ready for characterisation and in vitro examination of its tumour killing potential.

Materials and Methods

To generate recombinant ICP34.5 null HSV-1 expressing a gene of interest and GFP, requires the gene of interest and a suitable promoter to be cloned into the MCS of RL1.dIRES-GFP in the forward orientation with respect to the GFP gene in this plasmid. Once this has been achieved the plasmid is linearised (i.e. digested with a restriction enzyme that cuts only once, usually *Ssp*I or *Sca*I) in an irrelevant region. 80% confluent BHK cells in 60 mm petri dishes are then co-transfected with HSV-1 DNA and linearised plasmid DNA as described below.

To generate replication restricted HSV-1, expressing the gene of interest and GFP, the gene of interest must be cloned into RL1dIRES-GFP downstream of a suitable promoter (e.g. CMV IE). The promoter is required upstream of the gene of interest for the production of a bicistronic mRNA transcript. The IRES sequence between the two open reading frames in the transcript functions as a ribosome binding site for efficient cap-independent internal initiation of translation. The design enables coupled transcription of both the gene of interest and GFP, followed by cap-dependent initiation of translation of the first gene (gene of interest) and IRES-directed, cap-independent translation of GFP. Coordinate gene expression is thus ensured in this configuration.

*Co-Transfection of Virus and Plasmid DNA by $CaPO_4$ and DMSO Boost*

HSV-1 ($17^+$) DNA and 0.1-1μg linearized SMART cassette containing the gene and promoter of interest is pipetted into 1.5ml eppendorf tubes containing 1μl of calf thymus DNA (10μg/ml) and an appropriate volume of distilled water to give a final volume of 165μl. The solutions are very gently mixed using a 200μl pipette tip. 388μl of HEBS, pH 7.5, (130mM NaCl, 4.9mM KCl, 1.6mM $Na_2HPO_4$, 5.5mM D-glucose, 21mM HEPES) is then added, the solution mixed, before adding 26.5μl of 2M $CaCl_2$ dropwise and flicking the eppendorf tube two or three times. The samples are left at room temperature for 10-15 minutes then added dropwise to 80% confluent BHK's in 60mm petri dishes from which the medium has been removed. Following incubation at 37°C for 45 minutes, the cells are overlaid with 5ml of ETC10 and incubated at 37°C. Three to four hours later, the media is removed and the plates washed with ETC10. For exactly 4 minutes, the cells are overlaid with 1ml 25% (v/v) DMSO in HEBS at room temperature. After the 4 minutes, the cells are immediately washed three times with 5ml ETC10 before overlaying with 5ml of ETC10 and returning to the incubator. The following day, fresh medium is added to the cells. Two days later, when cpe is evident, cells are scraped into the medium, transferred to small bijoux and sonicated thoroughly. The sample is then stored at -70°C until required (see section below on plaque purification).

**N.B.** The volume of virus DNA to add is determined by undertaking the above procedure without plasmid DNA, using a range of virus DNA volumes and choosing the volume that gives the greatest number of viral plaques on the BHK monolayer after 2 or 3 days.

*Plaque Purification*

Sonicated samples from co-transfection plates are thawed and serially diluted 10 fold in ETC10. 100μl from neat to the $10^5$ dilution is plated out on confluent BHK's in 60 mm petri dishes from which the media has been removed. After 45 minutes incubation at 37°C, the cells are overlaid with 5ml EMC10 and incubated at 37°C for 48hrs. The plates are then checked for the presence of viral plaques and those dishes with the fewest, most separated plaques are placed under a fluorescent stereomicroscope. Recombinant virus, designed to express the green fluorescent protein (GFP) in addition to the gene of interest, can clearly be distinguished from wild type virus using a GFP filter. Fluorescent plaques are picked using a 20μl pipette and placed (including the tip) into an eppendorf tube containing 1ml ETC10. The sample is thoroughly sonicated before making serial 10 fold dilutions in ETC10 and repeating the above purification procedure. The process is repeated typically 3-4 times until every plaque on the BHK monolayer is fluorescent. Once this has been achieved, 50μl of this sample is used to infect BHK's in roller bottles, in 50ml ETC10, and a virus stock grown.

*Tissue Culture Media*

BHK21/C13 cells are grown in Eagle's medium (Gibco) supplemented with 10% newborn calf serum (Gibco) and 10% (v/v) tryptose phosphate broth. This is referred to as ETC10. For virus titrations and plaque purification, EMC10 (Eagles medium containing 1.5% methylcellulose and 10% newborn calf serum) is used to overlay the cells.

**Example 3**

**Construction of HSV1716/CMV-asSCCRO/GFP**

General Approach

HSV1716/CMV-asSCCRO/GFP was generated by first digesting pUSEamp-asSCCRO with *Ssp*I and *Xho*I and purifying the 1.96Kbp fragment generated from the digestion. The 1.96kbp *Ssp*I/*Xho*I fragment comprises DNA antisense to squamous cell carcinoma related antigen (asSCCRO), downstream of the CMV IE promoter (pCMV). This fragment was cloned into the MCS of the RL1.dIRES-GFP smart cassette, in the forward orientation with respect to the GFP gene in RL1.dIRES-GFP (Figure 8). The resultant plasmid, named RL1.dCMV-asSCCRO-GFP, was then linearised and recombinant virus generated and purified as described in Example 2. The plasmid pUSEamp-asSCCRO was obtained from Bhuvanesh Singh, Memorial Sloan Kettering Cancer Center, New York.

Materials and Methods

2μg of the RL1.dIRES-GFP plasmid was then digested with 15 units of *Bgl*II (Promega), in a suitable volume of 10x buffer (Promega) and nuclease free water (Promega), at 37°C for 16hrs. The digested plasmid was then purified using the QIAquick PCR purification kit (Qiagen), treated with 5 units of Klenow polymerase (Promega) for 20 minutes at room temperature, then purified again. The purified DNA was then added to 10 units of Calf Intestinal Phosphatase (Promega), in a suitable volume of 10x CIP buffer and nuclease free water for 4hrs at 37°C, before being purified again using the QIAquick PCR purification kit. 5μl of the purified DNA was electrophoresed on a 1% agarose gel to check its concentration (Figure 9).

4 x 1μg of pUSEamp-asSCCRO was digested with 10 units of *SspI* and 10 units of *Xho*I (Promega), in a suitable volume of 10x buffer (Promega) and nuclease free water (Promega), at 37°C for 16hrs. The reaction mixture was electrophoresed in a 1% agarose gel for 1hr at 110 volts. The 1.96Kbp DNA fragment, consisting essentially of the CMV promoter upstream of DNA antisense to SCCRO (pCMV-asSCCRO), was excised using a sterile scalpel and the DNA purified from the gel using a QIAquick Gel Extraction kit (Qiagen). The purified DNA was blunt ended using 5 units of Klenow polymerase (Promega) for 20 minutes at room temperature, then purified again. 5μl of the purified DNA fragment was electrophoresed on a 1% agarose gel to check its concentration (Figure 10).

Ligation reactions were carried out in small eppendorf tubes containing 5 units T4 DNA Ligase (Promega), a suitable volume of 10X DNA Ligase Buffer (Promega), nuclease free water (Promega) and various volumes of the *Bgl*II digested/blunt ended/CIP treated RL1.dIRES-GFP plasmid and blunt ended pCMV-asSCCRO, at 16°C overnight. Competent JM109 bacterial cells (Promega) were then transformed with various aliqouts of the ligation reactions. Colonies formed on the plates were picked, had their plasmid DNA extracted using a Qiagen Plasmid Mini kit and screened for inserts using *Bgl*II (Promega) restriction enzyme analysis. RL1.dIRES-GFP plasmid DNA containing the pCMV-asSCCRO insert would produce two fragments of 10.8Kbp and 1.4Kbp following digestion with *Bgl*II. One clone (clone 11) was found to contain the insert (Figure 11). The pCMV-asSCCRO insert could have been cloned into RL1.dIRES-GFP in two orientations. To confirm that the pCMV-asSCCRO fragment had been cloned into RL1.dIRES-GFP in the desired orientation, clone 11 was digested with 10 units of NruI (Promega), in a suitable volume of 10x buffer (Promega) and nuclease free water (Promega), at 37°C for 16hrs. If the insert was in the correct orientation, a fragment of 1.64Kbp would be generated. As a 1.64Kbp fragment was generated following digestion with *NruI* (Figure 12), it was confirmed that pCMV-asSCCRO had been cloned in the desired orientation. This plasmid (clone 11) was named 'RL1.dCMV-asSCCRO-GFP'.

0.1-1μg of RL1.dCMV-asSCCRO-GFP was linearized by digesting with 10 units of *Sca*I (Promega), in a suitable volume of 10x buffer (Promega) and nuclease free water (Promega), at 37°C for 16hrs. A sample (5μl) of the digested DNA was electrophoresed on a 1% agarose gel for 1hr at 110 volts to check that it had been linearized. 80% confluent BHK cells were then co-transfected with a suitable volume of the remaining linearised DNA and HSV-1 DNA. Recombinant HSV-1, expressing GFP (and hence asSCCRO), was identified and purified using a fluorescent microscope and a virus stock, named HSV1716/CMV-asSCCRO/GFP, was grown and titrated on BHK cells (Figure 13).

HSV1716/CMV-asSCCRO/GFP has been deposited as 'HSV1716asSCCRO' in the name of Crusade Laboratories Limited having an address at Department of Neurology Southern General Hospital 1345 Govan Road Govan Glasgow G51 5TF Scotland on 19 May 2004 at the European Collection of Cell Cultures (ECACC), Health Protection Agency, Porton Down, Salisbury, Wiltshire, SP4 0JG, United Kingdom under accession number 04051901 in accordance with the provisions of the Budapest Treaty on the International Recognition of the Deposit of Microorganisms for the Purposes of Patent Procedure (herein referred to as the 'Budapest Treaty').

**Example 4**

**The use of HSV1716asSCCRO as a novel therapeutic agent for head and neck squamous cell cancer.**

The inventors believe that insertion of the antisense to SCCRO into the herpes simplex virus HSV1716 may provide a virus with a dual hit mechanism of cell kill. This would involve virus induced cell death via cytolysis in addition to cell death via downregulation of endogenous SCCRO expression.

The HSV1716asSCCRO virus was constructed, amplified and purified in accordance with the present invention. Following this, in vitro and in vivo analysis was carried out on a series of head and neck squamous cell cancer (HNSCC) cell lines. HNSCC cell lines studied were SCC15, 1483, 1186, 1386, 1986 and 584. The relative expression of SCCRO protein expression in these cell lines was initially determined by western blotting. This showed the cell lines SCC15, 1483 and 1186 had high levels of expression of SCCRO, 1386 intermediate expression and 1986, 584 low expression. All cell lines were then infected with HSV1716 or HSV1716asSCCRO viruses and cytotoxicity determined by LDH release cytotoxicity assay at MOI (multiplicity of infection) of 1 and 5pfu/cell (Figures 14, 15 and 16). Viral proliferation was determined by serial plaque assays at an MOI of 1pfu/cell (Figure 17) and infectivity determined by green fluorescent protein (gfp) using HSV1716gtp virus (Figure 18). In the cell lines with low or intermediate expression (1986,584,1386) cytotoxicity increased in a dose dependent fashion with both viruses but there was no significant difference in cytotoxicity between the 2 viruses. Viral proliferation assays (Figure 17) showed an increase in viral production over a range of $10^2$ to $10^4$ with equivalent proliferation with both viruses. In the cell lines with high expression of SCCRO the inventors found that the cell line SCC15 showed enhanced cytotoxicity with the HSV1716asSCCRO virus. This observation occurred 12 hours post viral infection which is premature for virus induced cell death by a cytolytic mechanism. In addition, virus proliferation of the 2 viruses was equivalent with an increase in virus production of $10^4$ for both viruses. These results suggested that the enhanced cell kill at 12 hours was by an alternative mechanism possibly by downregulation of the endogenous high expression of SCCRO by antisenseSCCRO expression. To investigate this hypothesis the inventors analysed the cell lines SCC15 (high expression) and 584 (low expression) post virus infection by serial protein expression over a 36 hour period. Cells were infected at an MOI of 1pfu/cell with HSV1716 or HSV1716asSCCRO and cells harvested and lysed for protein at 12, 24 and 36 hours post infection. Western blotting of the cell line SCC15 showed downregulation of SCCRO protein at 12 hours with HSV1716asSCCRO but not in 584 (see Figure 19). This suggested that this was the mechanism by which HSV1716asSCCRO had enhanced efficacy in cell line SCC15.

In vivo studies were then carried out in the cell lines SCC15 and 584. Subcutaneous tumour were grown in athymic nude mice and injected intratumorally with a single injection of HSV1716, HSV1716asSCCRO or PBS control and tumour sizes monitored at serial time points (Figure 20 and 21). In SCC15, efficacy was enhanced with HSV1716asSCCRO compared to HSV1716. All 6 mice injected with HSV1716asSCCRO showed complete responses by 21 days post infection. Inhibition of tumour growth occurred with HSV1716 with only 3/6 mice showing a complete response over a 48 day follow up period. In the cell line 584, both viruses were able to inhibit tumour growth but neither virus produced a complete response in any mouse xenograft injected. This in vivo data was further evidence that HSV1716asSCCRO was a more potent antitumour agent than HSV1716 in the cell line SCC15 with high SCCRO expression.

These results suggest that HSV1716 and HSV1716asSCCRO has great potential as useful therapeutic agents in the treatment of recurrent or locally advanced head and neck cancer by direct intratumoral injection. However, this data also suggests that HSV1716asSCCRO may augment antitumour activity in SCCRO over-expressing tumours. Since SCCRO is overexpressed in a significant number of squamous cell cancers of the head and neck this modified virus may be particularly efficacious in this disease. Therefore, the inventors believe that HSV1716asSCCRO will be an important therapeutic agent in head and neck cancer patients with locally advanced or recurrent head and neck cancer, particularly as these cancers are amenable to direct intratumoural injection.

## Example 5 - Construction of HSV1716 variants expressing siRNA

General Strategy

A plasmid that contains the siRNA construct designed to target expression of the SCCRO gene (SEQ ID No. 5) and designated 339i was provided by Dr Bhuv Singh, MSKCC, New York. A plasmid encoding a control siRNA (SEQ ID No 6), designated Coni, was also provided.

Both siRNA constructs were in the vector pSNRG and their expression is driven by the RNA polIII H1 promoter. RNA polIII only transcribes short RNA molecules and the H1 promoter would be insufficient to drive expression of IRES-gfp from the normal recombinant virus producing shuttle vector RL1-del.IRES.gfp so an alternative cloning strategy was adopted.

A cassette was constructed in the following manner. The 1.3kbp blunt-ended EcoRI/AflII fragment that contains the PGK promoter/GFP gene was obtained by restriction digestion followed by Klenow treatment from the vector pSNRG and cloned into the RL1-del vector cut with the restriction enzyme NruI that generates blunt ends. Successful insertion of the PGK/GFP DNA was confirmed by BamHI digestion and the orientation of the inserted DNA identified using the unique XhoI site in RL1-del and the BsrGI site at the 3' end of PGK/GFP. Plasmids with PGK/GFP in both forward and reverse orientation were obtained and the plasmids were designated RL1-dPGK/GFPfor and RL1-dPGK/GFPrev. Expression of GFP was confirmed in BHK cells transfected with the forward and reverse orientation plasmids.

Thus, sequences of interest along with their own promoters (in this arrangement it is preferred that a different promoter is used to drive transcription of the nucleotide sequence of interest and marker) can then be cloned into either RL1-dPGK/GFPfor or RL1-dPGK/GFPrev in either orientation using the remaining unique BglII, XhoI or HpaI unique restriction enzyme sites. The resulting plasmid can be used to derive recombinant HSV in which the marker GFP gene and the gene of interest are expressed independently from their own promoters

Materials and methods

In the pSNRG plasmid and adjacent to the H1/siRNA coding sequence is a green fluorescent protein (gfp) expression cassette comprising the gfp gene with a Phosphoglycerokinase (PGK) promoter. Using the restriction enzymes HindIII and AflII sequentially, the 1.6kbp DNA fragment that contains the H1/siRNA and PGK/EGFP expression cassettes were excised from their Coni and 339i plasmids. The 1.6kbp DNA fragment was purified from a 1% agarose gel and blunt-ended by incubation with Klenow DNA polymerase for 30 minutes at 30°C. The blunt-ended fragment was ligated into the RL1-del shuttle vector which had been digested with the restriction enzyme Nru1 that produces a blunt-ended cut. Before ligation the Nru1-cut RL1-del was gel purified and phosphatase-treated using Calf Intestinal Alkaline Phosphatase. After an overnight ligation with either the blunt-ended 339i or Coni DNA fragments with the blunt-ended RL1-del plasmid, the reaction mix was used to transform DH5alpha cells and these were plated-out on LB amp plates. After overnight incubation at 37°C, individual clones from each of the LB amp plates were grown overnight in 3ml of LB broth and plasmid DNA extracted.

To screen for recombinants, plasmids were initially digested with BamHI, as insertion of the H1/siRNA and PGK/gfp cassette increases the size of the RL1 BamHI fragment in the plasmid from 5.4kbp to 7.0kbp. For both Coni and 339i ligations 1/24 clones screened demonstrated a 7.0kbp BamHI fragment and the presence of the H1/siRNA and PGK/EGFP cassette in these plasmids was confirmed by EcoR1, EcoR1/HindIII and EcoR1/SalI digests, the inserted H1/siRNA and PGK/EGFP cassette introduces a novel EcoR1 site into the RL1-del vector.

From a glycerol stock of the positive 339i and Coni clones, additional plasmid was prepared and used to transfect BHK cells. Fifty microlitres (50μl) of plasmid was mixed with 6μl lipofectamine 2000 in a final volume of 100μl

serum free medium and used to transfect BHK cells plated out on a 13mm glass coverslip in a 24-well plate. After 48hrs of transfection the cells were washed once in PBS, incubated for 2hrs in 4% paraformaldehyde, washed once more in PBS and mounted on microscope slides using Vectashield. The presence of c5% gfp-positive cells following transfection with the RL1-del/339i and RL1-del/Coni plasmids confirmed the presence of the PGK/GFP cassette.

The RL1-del/339i and RL1-del/Coni plasmids were linearized using either of the restriction enzymes ScaI and XmnI and the linearized plasmid was used along with viral DNA to transfect BHK cells plated out to c80% confluency in 60mm dishes. To 100μl of linearized plasmid or undigested circular plasmid, 50μl of HSV-1 strain 17+ DNA was added along with 20μl lipofectamine 2000 in a final volume of 500μl serum free medium and the mix added to the BHK cells. After 4hrs of transfection, the cells were shocked with 25% DMSO in HBSS for exactly 4 minutes, washed x3 with medium and returned to 37°C incubation in 5ml of medium for 48hrs. Viral cpe was evident after 48hrs and the cells and medium were harvested together, sonicated and stored at -80°C. Undiluted medium/cells and 4x 10-fold dilutions were plated out on BHK cells and, after 48hrs, viral plaques were examined by fluorescent microscopy for gfp expression. On the undiluted plate from cells transfected with XmnI-linearized plasmid >100 gfp-positive plaques were observed for both Coni and 339i indicating a high degree of recombination. Interestingly, recombination, but at a lower frequency (c50 plaques/plate), was observed for the transfected circular plasmid but recombination with the ScaI-linearized plasmid was very low (<5 plaques/plate).

Using the highest dilution at which gfp-positive plaques were clearly visible (the PGK/GFP cassette gave a very strong fluorescent signal), two plaques each of Coni and 339i viruses were picked using a sterile pipette tip, placed in 1ml medium, sonicated for 1 minute and stored at -80°C. Plaques were then subjected to 6 rounds of plaque purification, after the 6$^{th}$ round no wild type, non-gfp expressing plaques were visible and 6 plaques each of Coni or 339i virus were picked for Southern blotting.

Each of the six plaques of Coni and 339i virus was used to infect a T175 flask of Vero cells, after 72hrs of infection virus was harvested and titred. For 3 each of the Coni and 339i viruses that gave the highest titres 0.5ml was used to infect a second T175 flask for 24hrs. Viral DNA was then harvested from each of the 6 flasks. The BamHI-digested viral DNA was Southern blotted with the Alu/Rsa RL1 probe and the band pattern compared to wild type and HSV1716 DNA digested also with BamHI. A novel c6kbp band, consistent with the insertion of the 1.6kbp H1/siRNA and PGK/GFP cassette in the RL1 locus, was clearly visible in all six viral isolates and no wild type bands were detected. Stocks of the Coni and 339i viruses that gave the strongest signal on Southern blotting were produced.

## References

1. BL Liu, M Robinson, Z-Q Han, RH Branston, C English, Preay, Y McGrath, SK Thomas, M Thornton, P Bullock, CA Love and RS Coffin; Gene Therapy (2003) 10, 292-303.

2. WO 92/13943

3. A Dolan, E Mckie, AR Maclean, DJ McGeoch; Journal of General Virology (1992) 73 971-973.

4. Aidan Dolan, Fiona E Jamieson, Charles Cunnigham, Barbara C Barnett Duncan J McGeoch; Journal of Virology Mar 1998 2010-2021.

5. Joany Chou, Earl R Kern, Richard J Whitley, Bernard Roizman; Science (1990) 250 1262-1265.

6. Coffin RS, MacLean AR, Latchman DS, Brown SM; gene therapy (1996) Oct 3(10) 886-91.

7. McKie EA, Hope RG, Brown SM, Maclean AR; Journal of General Virology, (1994) Apr 75(Pt4) 733-41.

8. McKay EM, McVey B, Marsden HS, Brown SM, MacLean AR; Journal of general Virology, (1993) Nov 74(Pt11) 2493-7.

9. Joany Chou, Bernard Roizman; Journal of Virology; (1990) Mar 1014-1020.

10. Green, N.K., Youngs, D.J, J.P. Neoptolemos, F. Friedlos, R.J. Knox, C.J. Springer, G.M. Anlezark, N.P. Michael, R.G. Melton, M.J. Ford, L.S.Young, D.J. Kerr, and P.F. Searle; Cancer Gene Therapy (1997) 4:229-238.

11. Cherry L. Estilo, Pornchai O-charoenrat, Ivan Nagai, Snehal G. Patel, Pabbathi G. Reddy, Su Dao, Ashok R.

Shaha, Dennis H. Kraus, Jay O. Boyle, Richard J. Wong, David G. Pfister, Joseph M. Huryn, Ian M. Zlotolow, Jatin P. Shah and Bhuvanesh Singh; Clinical Cancer Research (June 2003) Vol. 9 2300-2306.

12. Ganly I, Kaye SB. Recurrent head and neck cancer-current therapy and future prospects. Annals of Oncology (2000),11:1-6.

13. Ganly I, Soutar DS, Kaye SB.Current role of gene therapy in head and neck cancer. European J of Surgical Oncology (2000),26:338-343.

14. Ganly I, Kirn D, Soutar D, Eckardt G, Otto R, Robertson AG, Park O, Heise C, Von Hoff DD, Kaye SB. A phase I study of Onyx-015, an E1B attenuated adenovirus, administered intratumourally in patients with recurrent tumours of the head and neck. Clinical Cancer Research (2000), 6:798-806.

15. Khuri F, Nemunaitis J, Ganly I, Arseneau J, Tannock I, Romel L, Gore M, Ironside J, Heise C, Randley B, Gillenwater A, Bruso P, Kaye SB, Hong WK, Kirn DH. Controlled trial of intratumoural ONYX-015, a selectively replicating adenovirus, in combination with cisplatin and 5-fluorouracil in patients with recurrent head and neck cancer. Nature Medicine (2000) 6(8): 879-885.

16. Heise C, Sampson-Johannes A, Williams A, McCormick F, Von Hoff D and Kirn DH. Onyx -015 an E1B gene attenuated adenovirus causes tumour specific cytolysis and antitumoural dfficacy that canb e augmented by standard chemotherpeutic agents. Nature Medicine 3, 639-645.

17. MacLean AR, Fareed MU, Robertson L, Harland J, and Brown SM. (1991). Herpes simplex virus type 1 deletion variants 1714 and 1716 pinpoint neurovirulence related sequences in Glasgow strain 17+ between immediate early gene 1 and the 'a' sequence. Journal of General Virology 72, 631-639.

18. Brown SM, Harland J, MacLean AR, Podlech J. and Clements JB. (1994). Cell type and cell state determine differentiated in vitro growth of non-neurovirulent ICP34.5 negative herpes simplex virus. Journal of General Virology 75, 2367-2377.

19. McKie EA, MacLean AR, Lewis AD, Cruickshank G, Rampling R, Barnett SC, Kennedy PGE and Brown SM. (1996) Selective in vitro replication of herpes simplex virus type 1 (HSV1), ICP34.5 null mutants in primary human CNS tumours-evaluation of a potentially effective clinical therapy. British Journal of Cancer 74, 745-752.

20. Randazzo B, Kesari S, MacLean AR, Brown SM. and Fraser NW. (1995). Treatment of experimental intracranial murine melanoma with the neuroattenuated HSV1 mutant 1716. Virology 211, 94-101.

21. Kesari S, Randazzo BP, Valyi-Nagy T, Huang QS, Brown SM, MacLean AR, Lee VM-Y, Trojanowski JQ. and Fraser NW. (1995). A mutant herpes simplex virus replicates in brain tumours but not in neurons derived from a human embryonal carcinoma cell line. Laboratory Investigation 73, 636-648.

22. Randazzo BP, Kucharczuk JC, Litzky LA, Kaiser LR, Brown SM, MacLean AR, Albelda SM. and Fraser NW. (1996). Herpes simplex 1716 - an ICP34.5 mutant is severely replication restricted in human skin xenografts in vivo. Virology 223, 392-396.

23. Lasner TM, Kesari S, Brown SM, Lee VM-Y, Fraser NW. and Trojanowski JQ. (1996). Herpes simplex virus type 1 (HSV1) mutants for the treatment of childhood brain tumours. Journal of Neuropathology and Experimental Neurology 55,1259-1269

24. Kucharczuk JC, Randazzo B, Chang MY, Amin KM, Elshami AA, Sterman DH. Rizk NP, Molnar-Kimber KL, Brown SM, MacLean AR, Litzky LA, Fraser NW, Albelda SM. and Kaiser LR. (1997). Use of a "replication restricted" herpes virus to treat experimental human malignant mesothelioma. Cancer Research, 57, 466-471.

25. Randazzo BP, Bhat MG, Kesari S, Fraser NW. and Brown SM. (1997). Treatment of experimental subcutaneous human melanoma with a replication restricted herpes simplex virus mutant. Journal of Investigative Dermatology, 108, 933-937.

26. Rampling R, Cruickshank G, Papanastassiou V, Nicoll J, Hadley D, Petty R, Maclean A, Harland J, McKie E, Mabbs R. & Brown SM. (2000). Toxicity evaluation of replication competent herpes simplex virus (ICP 34.5 null mutant 1716) in patients with recurrent malignant glioma. Gene Therapy 7, 859-866.

The following numbered paragraphs contain statements of broad combinations of the inventive technical features herein disclosed:

1. An herpes simplex virus wherein the herpes simplex virus genome comprises nucleic acid encoding an antisense to the squamous cell carcinoma related oncogene (asSCCRO).

2. An herpes simplex virus according to paragraph 1 wherein said nucleic acid encodes a mammalian asSCCRO.

3. An herpes simplex virus according to paragraph 1 wherein said nucleic acid encodes the human asSCCRO.

4. An herpes simplex virus according to paragraph 1 wherein said nucleic acid encodes a nucleotide sequence complementary to:

(i) the polynucleotide sequence of SEQ ID No.s 1 or 3 or its complement;
(ii) the mRNA transcript of SEQ ID No.s 1 or 3; or
(iii) to a fragment of said polynucleotide sequence or mRNA transcript.

5. An herpes simplex virus according to paragraph 1 wherein said nucleic acid encodes a nucleotide sequence having at least 60% sequence identity to the nucleotide sequence complementary to:

(i) the polynucleotide sequence of SEQ ID No.s 1 or 3 or its complement;
(ii) the mRNA transcript of SEQ ID No.s 1 or 3; or
(iii) to a fragment of said polynucleotide sequence or mRNA transcript.

6. An herpes simplex virus according to paragraph 5 wherein said degree of sequence identity is at least 70%.

7. An herpes simplex virus according to any one of paragraphs 4 to 6 wherein a said fragment comprises at least 20 nucleotides and no more than 900 nucleotides.

8. An herpes simplex virus according to paragraph 1 wherein said nucleic acid hybridises to:

(i) the polynucleotide sequence of SEQ ID No.s 1 or 3 or its complement;
(ii) the mRNA transcript of SEQ ID No.s 1 or 3; or
(iii) to a fragment of said polynucleotide sequence or mRNA transcript under high stringency conditions.

9. An herpes simplex virus as claimed in any one of paragraphs 1 to 8 wherein said herpes simplex virus genome further comprises a regulatory sequence operably linked to said nucleic acid encoding an antisense to the squamous cell carcinoma related oncogene (as SCCRO), wherein said regulatory sequence has a role in controlling transcription of said asSCCRO.

10. An herpes simplex virus as claimed in any one of paragraphs 1 to 9 wherein said nucleic acid is located in at least one RL1 locus of the herpes simplex virus genome.

11. An herpes simplex virus as claimed in any one of paragraphs 1 to 10 wherein said nucleic acid is located in, or overlaps, at least one of the ICP34.5 protein coding sequences of the herpes simplex virus genome.

12. An herpes simplex virus as claimed in any one of paragraphs 1 to 11 wherein the herpes simplex virus is a mutant of one of HSV-1 strains 17 or F or HSV-2 strain HG52.

13. An herpes simplex virus as claimed in any one of paragraphs 1 to 11 wherein the herpes simplex virus is a mutant of HSV-1 strain 17 mutant 1716.

14. An herpes simplex virus as claimed in any one of paragraphs 1 to 13 which is a gene specific null mutant.

15. An herpes simplex virus as claimed in any one of paragraphs 1 to 14 which is an ICP34.5 null mutant.

16. An herpes simplex virus as claimed in any one of paragraphs 1 to 13 which lacks at least one expressible ICP34.5 gene.

17. An herpes simplex virus as claimed in any one of paragraphs 1 to 12 which lacks only one expressible ICP34.5 gene.

18. An herpes simplex virus as claimed in any one of paragraphs 1 to 17 which is non-neurovirulent.

19. An herpes simplex virus as claimed in any one of paragraphs 1 to 18 wherein said nucleic acid encoding the asSCCRO forms part of a nucleic acid cassette integrated in the genome of said herpes simplex virus, said cassette comprising nucleic acid encoding:

(a) said asSCCRO; and nucleic acid encoding:
(b) a ribosome binding site; and
(c) a marker,

wherein the nucleic acid encoding asSCCRO is arranged upstream (5') of the ribosome binding site and the ribosome binding site is arranged upstream (5') of the marker.

20. An herpes simplex virus according to paragraph 19 wherein a regulatory nucleotide sequence is located upstream (5') of the nucleic acid encoding asSCCRO, wherein the regulatory nucleotide sequence has a role in regulating transcription of said nucleic acid encoding the asSCCRO.

21. An herpes simplex virus as claimed in paragraph 19 or 20 wherein the cassette disrupts a protein coding sequence resulting in inactivation of the respective gene product.

22. An herpes simplex virus as claimed in any one of paragraphs 19 to 21 wherein a transcription product of the cassette is a bi- or poly- cistronic transcript comprising a first cistron encoding the asSCCRO and a second cistron encoding the marker nucleic acid wherein the ribosome binding site is located between said first and second cistrons.

23. An herpes simplex virus as claimed in any one of paragraphs 19 to 22 wherein the ribosome binding site comprises an internal ribosome entry site (IRES).

24. An herpes simplex virus as claimed in any one of paragraphs 19 to 22 wherein the marker is a defined nucleotide sequence encoding a polypeptide.

25. An herpes simplex virus as claimed in paragraph 24 wherein the marker comprises the Green Fluorescent Protein (GFP) protein coding sequence or the enhanced Green Fluorescent Protein (EGFP) protein coding sequence.

26. An herpes simplex virus according to any one of paragraphs 19 to 23 wherein the marker comprises a defined nucleotide sequence detectable by hybridisation under high stringency conditions with a corresponding labelled nucleic acid probe.

27. An herpes simplex virus as claimed in any one of paragraphs 19 to 26 wherein the cassette further comprises nucleic acid encoding a polyadenylation sequence located downstream (3') of the nucleic acid encoding the marker.

28. An herpes simplex virus as claimed in paragraph 27 wherein the polyadenylation sequence comprises the Simian Virus 40 (SV40) polyadenylation sequence.

29. An herpes simplex virus as claimed in any one of paragraphs 1 to 28 for use in a method of medical treatment.

30. An herpes simplex virus as claimed in any one of paragraphs 1 to 28 for use in the treatment of cancer.

31. An herpes simplex virus as claimed in any one of paragraphs 1 to 28 for use in the oncolytic treatment of a tumour.

32. Use of an herpes simplex virus as claimed in any one of paragraphs 1 to 28 in the manufacture of a medicament for the treatment of cancer.

33. A method of lysing or killing tumour cells in vitro or in vivo comprising the step of administering to a patient in need of treatment an herpes simplex virus as claimed in any one of paragraphs 1 to 28.

34. A medicament, pharmaceutical composition or vaccine comprising an herpes simplex virus as claimed in any one of paragraphs 1 to 28.

35. A medicament, pharmaceutical composition or vaccine as claimed in paragraph 34 further comprising a pharmaceutically acceptable carrier, adjuvant or diluent.

36. An herpes simplex virus, wherein the genome of said virus comprises a nucleic acid sequence encoding an antisense to the squamous cell carcinoma related oncogene (asSCCRO) in at least one of the long repeat regions ($R_L$).

37. An herpes simplex virus, wherein the genome of said virus comprises a nucleic acid sequence encoding an antisense to the squamous cell carcinoma related oncogene (asSCCRO) and wherein the herpes simplex virus is non-neurovirulent.

38. An herpes simplex virus for use in the treatment of a tumour, wherein the genome of said virus comprises a nucleic acid sequence encoding an antisense to the squamous cell carcinoma related oncogene (asSCCRO) in at least one of the long repeat regions ($R_L$).

39. An herpes simplex virus for use in the treatment of a tumour, wherein the genome of said virus comprises a nucleic acid sequence encoding an antisense to the squamous cell carcinoma related oncogene (asSCCRO) and wherein the herpes simplex virus is non-neurovirulent.

40. Use of a herpes simplex virus, wherein the genome of said virus comprises a nucleic acid sequence encoding an antisense to the squamous cell carcinoma related oncogene (asSCCRO) in at least one of the long repeat regions ($R_L$), in the manufacture of a medicament for the treatment of cancer.

41. Use of a herpes simplex virus, wherein the genome of said virus comprises a nucleic acid sequence encoding an antisense to the squamous cell carcinoma related oncogene (asSCCRO) and wherein the herpes simplex virus is non-neurovirulent, in the manufacture of a medicament for the treatment of cancer.

42. A method for the treatment of a tumour comprising the step of administering to a patient in need of treatment a herpes simplex virus, wherein the genome of said virus comprises a nucleic acid sequence encoding an antisense to the squamous cell carcinoma related oncogene (asSCCRO) in at least one of the long repeat regions ($R_L$).

43. A method for the treatment of a tumour comprising the step of administering to a patient in need of treatment a herpes simplex virus, wherein the genome of said virus comprises a nucleic acid sequence encoding an antisense to the squamous cell carcinoma related oncogene (asSCCRO) and wherein the herpes simplex virus is non-neurovirulent.

44. The method of paragraph 42 or 43 wherein said herpes simplex viruses is capable of killing tumour cells.

45. A method of expressing in vitro or in vivo an antisense to the squamous cell carcinoma related oncogene (asSCCRO), said method comprising the step of infecting at least one cell or tissue of interest with a herpes simplex virus, wherein the genome of said virus comprises a nucleic acid sequence encoding asSCCRO in at least one of the long repeat regions ($R_L$), said asSCCRO operably linked to a transcription regulatory sequence.

46. A method of expressing in vitro or in vivo an antisense to the squamous cell carcinoma related oncogene (asSCCRO), said method comprising the step of infecting at least one cell or tissue of interest with a non-neurovirulent herpes simplex virus, wherein the genome of said virus comprises a nucleic acid sequence encoding asSCCRO, said asSCCRO operably linked to a transcription regulatory sequence.

47. HSV1716asSCCRO (ECACC accession number 04051901).

48. An herpes simplex virus wherein the herpes simplex virus genome comprises nucleic acid encoding a short interfering ribonucleic acid (siRNA) molecule that is capable of repressing or silencing expression of squamous cell carcinoma related oncogene (SCCRO) nucleic acid or polypeptide.

49. An herpes simplex virus according to paragraph 48 wherein said siRNA is capable of repressing or silencing expression of a mammalian SCCRO.

50. An herpes simplex virus according to paragraph 48 wherein said siRNA is capable of repressing or silencing expression of human SCCRO.

51. An herpes simplex virus according to paragraph 48 wherein said siRNA comprises a nucleic acid of between 10 and 70 nucleotides in length and having the sequence of SEQ ID No.5 or the complement thereof.

52. An herpes simplex virus according to paragraph 48 wherein said siRNA comprises a nucleic acid of between 10 and 70 nucleotides in length and having at least 70% identity to SEQ ID No.5 or the complement thereof.

53. An herpes simplex virus according to paragraph 52 wherein said degree of sequence identity is at least 80%.

54. An herpes simplex virus as claimed in any one of paragraphs 48 to 53 wherein said herpes simplex virus genome further comprises a regulatory sequence operably linked to said siRNA, wherein said regulatory sequence has a role in controlling transcription of said siRNA.

55. An herpes simplex virus as claimed in any one of paragraphs 48 to 54 wherein said nucleic acid is located in at least one RL1 locus of the herpes simplex virus genome.

56. An herpes simplex virus as claimed in any one of paragraphs 48 to 55 wherein the said nucleic acid is located in, or overlaps, at least one of the ICP34.5 protein coding sequences of the herpes simplex virus genome.

57. An herpes simplex virus as claimed in any one of paragraphs 48 to 56 wherein the herpes simplex virus is a mutant of one of HSV-1 strains 17 or F or HSV-2 strain HG52.

58. An herpes simplex virus as claimed in any one of paragraphs 48 to 56 wherein the herpes simplex virus is a mutant of HSV-1 strain 17 mutant 1716.

59. An herpes simplex virus as claimed in any one of paragraphs 48 to 58 which is a gene specific null mutant.

60. An herpes simplex virus as claimed in any one of paragraphs 48 to 59 which is an ICP34.5 null mutant.

61. An herpes simplex virus as claimed in any one of paragraphs 48 to 58 which lacks at least one expressible ICP34.5 gene.

62. An herpes simplex virus as claimed in any one of paragraphs 48 to 57 which lacks only one expressible ICP34.5 gene.

63. An herpes simplex virus as claimed in any one of paragraphs 48 to 62 which is non-neurovirulent.

64. An herpes simplex virus as claimed in any one of paragraphs 48 to 63 wherein said nucleic acid encoding said siRNA forms part of a nucleic acid cassette integrated in the genome of said herpes simplex virus, said cassette comprising nucleic acid encoding

(a) said siRNA; and nucleic acid encoding:
(b) a first regulatory nucleotide sequence; and
(c) a marker,

wherein the nucleic acid encoding said siRNA is arranged upstream (5') of the first regulatory nucleotide sequence and the first regulatory nucleotide sequence is arranged upstream (5') of the marker, wherein said first regulatory sequence has a role in controlling transcription of said marker.

65. An herpes simplex virus according to paragraph 64 wherein a second regulatory nucleotide sequence is located upstream (5') of the nucleic acid encoding said siRNA, wherein the second regulatory nucleotide sequence has a role in regulating transcription of said nucleic acid encoding said siRNA.

66. An herpes simplex virus as claimed in paragraph 64 or 65 wherein the cassette disrupts a protein coding sequence resulting in inactivation of the respective gene product.

67. An herpes simplex virus as claimed in any one of paragraphs 64 to 66 wherein the marker is a defined nucleotide sequence encoding a polypeptide.

68. An herpes simplex virus as claimed in paragraph 67 wherein the marker comprises the Green Fluorescent Protein (GFP) protein coding sequence or the enhanced Green Fluorescent Protein (EGFP) protein coding sequence.

69. An herpes simplex virus according to any one of paragraphs 64 to 66 wherein the marker comprises a defined nucleotide sequence detectable by hybridisation under high stringency conditions with a corresponding labelled nucleic acid probe.

70. An herpes simplex virus as claimed in any one of paragraphs 64 to 69 wherein the cassette further comprises nucleic acid encoding a polyadenylation sequence located downstream (3') of the nucleic acid encoding the marker.

71. An herpes simplex virus as claimed in paragraph 70 wherein the polyadenylation sequence comprises the Simian Virus 40 (SV40) polyadenylation sequence.

72. An herpes simplex virus as claimed in any one of paragraphs 48 to 71 for use in a method of medical treatment.

73. An herpes simplex virus as claimed in any one of paragraphs 48 to 71 for use in the treatment of cancer.

74. An herpes simplex virus as claimed in any one of paragraphs 48 to 71 for use in the oncolytic treatment of a tumour.

75. Use of an herpes simplex virus as claimed in any one of paragraphs 48 to 71 in the manufacture of a medicament for the treatment of cancer.

76. A method of lysing or killing tumour cells in vitro or in vivo comprising the step of administering to a patient in need of treatment an herpes simplex virus as claimed in any one of paragraphs 48 to 71.

77. A medicament, pharmaceutical composition or vaccine comprising an herpes simplex virus as claimed in any one of paragraphs 48 to 71.

78. A medicament, pharmaceutical composition or vaccine as claimed in paragraph 77 further comprising a pharmaceutically acceptable carrier, adjuvant or diluent.

79. An herpes simplex virus, wherein the genome of said virus comprises a nucleic acid sequence encoding a short interfering ribonucleic acid (siRNA) molecule that is capable of repressing or silencing expression of squamous cell carcinoma related oncogene (SCCRO) nucleic acid or polypeptide in at least one of the long repeat regions ($R_L$).

80. An herpes simplex virus, wherein the genome of said virus comprises a nucleic acid sequence encoding a short interfering ribonucleic acid (siRNA) molecule that is capable of repressing or silencing expression of squamous cell carcinoma related oncogene (SCCRO) nucleic acid or polypeptide and wherein the herpes simplex virus is non-neurovirulent.

81. An herpes simplex virus for use in the treatment of a tumour, wherein the genome of said virus comprises a nucleic acid sequence encoding a short interfering ribonucleic acid (siRNA) molecule that is capable of repressing or silencing expression of squamous cell carcinoma related oncogene (SCCRO) nucleic acid or polypeptide in at least one of the long repeat regions ($R_L$).

82. An herpes simplex virus for use in the treatment of a tumour, wherein the genome of said virus comprises a nucleic acid sequence encoding a short interfering ribonucleic acid (siRNA) molecule that is capable of repressing or silencing expression of squamous cell carcinoma related oncogene (SCCRO) nucleic acid or polypeptide and wherein the herpes simplex virus is non-neurovirulent.

83. Use of a herpes simplex virus, wherein the genome of said virus comprises a nucleic acid sequence encoding a short interfering ribonucleic acid (siRNA) molecule that is capable of repressing or silencing expression of squamous cell carcinoma related oncogene (SCCRO) nucleic acid or polypeptide in at least one of the long repeat regions ($R_L$), in the manufacture of a medicament for the treatment of cancer.

84. Use of a herpes simplex virus, wherein the genome of said virus comprises a nucleic acid sequence encoding a short interfering ribonucleic acid (siRNA) molecule that is capable of repressing or silencing expression of squamous cell carcinoma related oncogene (SCCRO) nucleic acid or polypeptide and wherein the herpes simplex virus is non-neurovirulent, in the manufacture of a medicament for the treatment of cancer.

85. A method for the treatment of a tumour comprising the step of administering to a patient in need of treatment a herpes simplex virus, wherein the genome of said virus comprises, in at least one of the long repeat regions ($R_L$), a nucleic acid sequence encoding a short interfering ribonucleic acid (siRNA) molecule that is capable of repressing or silencing expression of squamous cell carcinoma related oncogene (SCCRO) nucleic acid or polypeptide.

86. A method for the treatment of a tumour comprising the step of administering to a patient in need of treatment a herpes simplex virus, wherein the genome of said virus comprises a nucleic acid sequence encoding a short interfering ribonucleic acid (siRNA) molecule that is capable of repressing or silencing expression of squamous cell carcinoma related oncogene (SCCRO) nucleic acid or polypeptide and wherein the herpes simplex virus is non-neurovirulent.

87. The method of paragraph 85 or 86 wherein said herpes simplex virus is capable of killing tumour cells.

88. A method of expressing in vitro or in vivo a short interfering ribonucleic acid (siRNA) molecule that is capable of repressing or silencing expression of squamous cell carcinoma related oncogene (SCCRO) nucleic acid or polypeptide, said method comprising the step of infecting at least one cell or tissue of interest with a herpes simplex virus, wherein the genome of said virus comprises a nucleic acid sequence encoding said siRNA in at least one of the long repeat regions ($R_L$), wherein said nucleic acid sequence encoding said siRNA is operably linked to a transcription regulatory sequence.

89. A method of expressing in vitro or in vivo a short interfering ribonucleic acid (siRNA) molecule that is capable of repressing or silencing expression of squamous cell carcinoma related oncogene (SCCRO) nucleic acid or polypeptide, said method comprising the step of infecting at least one cell or tissue of interest with a non-neurovirulent herpes simplex virus, wherein the genome of said virus comprises a nucleic acid sequence encoding said siRNA, wherein said nucleic acid sequence encoding said siRNA is operably linked to a transcription regulatory sequence.

## Claims

**1.** An oncolytic mutant herpes simplex virus (HSV) wherein the genome of the HSV comprises a nucleotide sequence encoding an antisense nucleic acid to a target nucleotide sequence.

**2.** An oncolytic mutant herpes simplex virus according to claim 1 wherein the HSV is configured to express the antisense nucleic acid.

**3.** An oncolytic mutant herpes simplex virus according to claim 1 or 2 wherein the target nucleotide sequence is an mRNA.

**4.** An oncolytic mutant herpes simplex virus according to any one of claims 1 to 3 wherein the antisense nucleic acid is a non-HSV originating sequence.

**5.** An oncolytic mutant herpes simplex virus according to any one of claims 1 to 4 wherein the genome of the mutant HSV further comprises a regulatory nucleotide sequence operably linked to the nucleotide sequence encoding the antisense nucleic acid

**6.** An oncolytic mutant herpes simplex virus (HSV) wherein the genome of the HSV comprises a nucleotide sequence encoding a short interfering ribonucleic acid (siRNA) molecule that is capable of repressing or silencing expression of a target nucleic acid or polypeptide.

**7.** An oncolytic mutant herpes simplex virus according to claim 7 wherein the HSV is configured to express the siRNA.

**8.** An oncolytic mutant herpes simplex virus according to claim 6 or 7 wherein the genome of the mutant HSV further comprises a regulatory nucleotide sequence operably linked to the nucleotide sequence encoding the siRNA.

**9.** An oncolytic mutant herpes simplex virus according to any one of claims 1 to 8 wherein the mutant HSV is an ICP34.5 null mutant.

**10.** An oncolytic mutant herpes simplex virus according to any one of claims 1 to 8 wherein the mutant HSV lacks at least one expressible ICP34.5 gene, or lacks only one expressible ICP34.5 gene.

**11.** An oncolytic mutant herpes simplex virus according to any one of claims 1 to 10 wherein the mutant HSV is non-neurovirulent.

**12.** An oncolytic mutant herpes simplex virus according to any one of claims 1 to 11 wherein the mutant HSV is a mutant of HSV-1, and is optionally a mutant of HSV-1 strain 17, or HSV-1 strain F, or HSV-1 strain 17 mutant 1716.

**13.** A medicament, pharmaceutical composition or vaccine comprising an oncolytic mutant herpes simplex virus according to any one of claims 1 to 12.

**14.** An oncolytic mutant herpes simplex virus according to any one of claims 1 to 12 for use in a method of treatment of cancer or of a tumour.

**15.** Use of an oncolytic mutant herpes simplex virus according to any one of claims 1 to 12 in the manufacture of a medicament for the treatment of cancer or of a tumour.

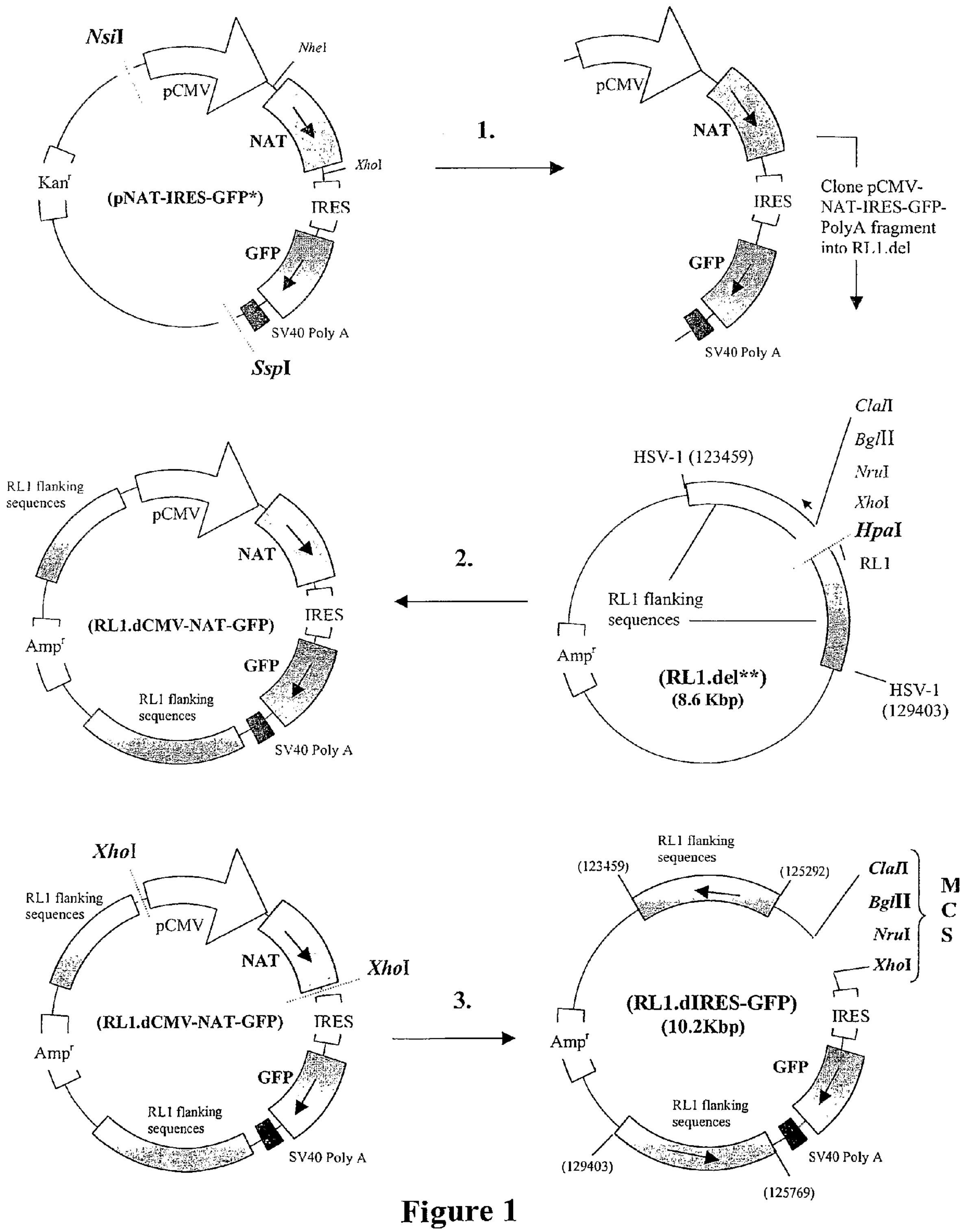
NsiI
NheI
pCMV
NAT
XhoI
Kanr
(pNAT-IRES-GFP*)
IRES
GFP
SV40 Poly A
SspI
1.
pCMV
NAT
IRES
GFP
SV40 Poly A
Clone pCMV-NAT-IRES-GFP-PolyA fragment into RL1.del
ClaII
BglII
NruI
XhoI
HpaI
HSV-1 (123459)
RL1
RL1 flanking sequences
Ampr
(RL1.del**)
(8.6 Kbp)
HSV-1 (129403)
2.
RL1 flanking sequences
pCMV
NAT
(RL1.dCMV-NAT-GFP)
IRES
Ampr
GFP
RL1 flanking sequences
SV40 Poly A
XhoI
RL1 flanking sequences
pCMV
NAT
XhoI
(RL1.dCMV-NAT-GFP)
IRES
Ampr
GFP
RL1 flanking sequences
SV40 Poly A
3.
RL1 flanking sequences
(123459)
(125292)
ClaII
BglII
NruI
XhoI
M
C
S
(RL1.dIRES-GFP)
(10.2Kbp)
IRES
Ampr
GFP
RL1 flanking sequences
SV40 Poly A
(129403)
(125769)

**Figure 1**

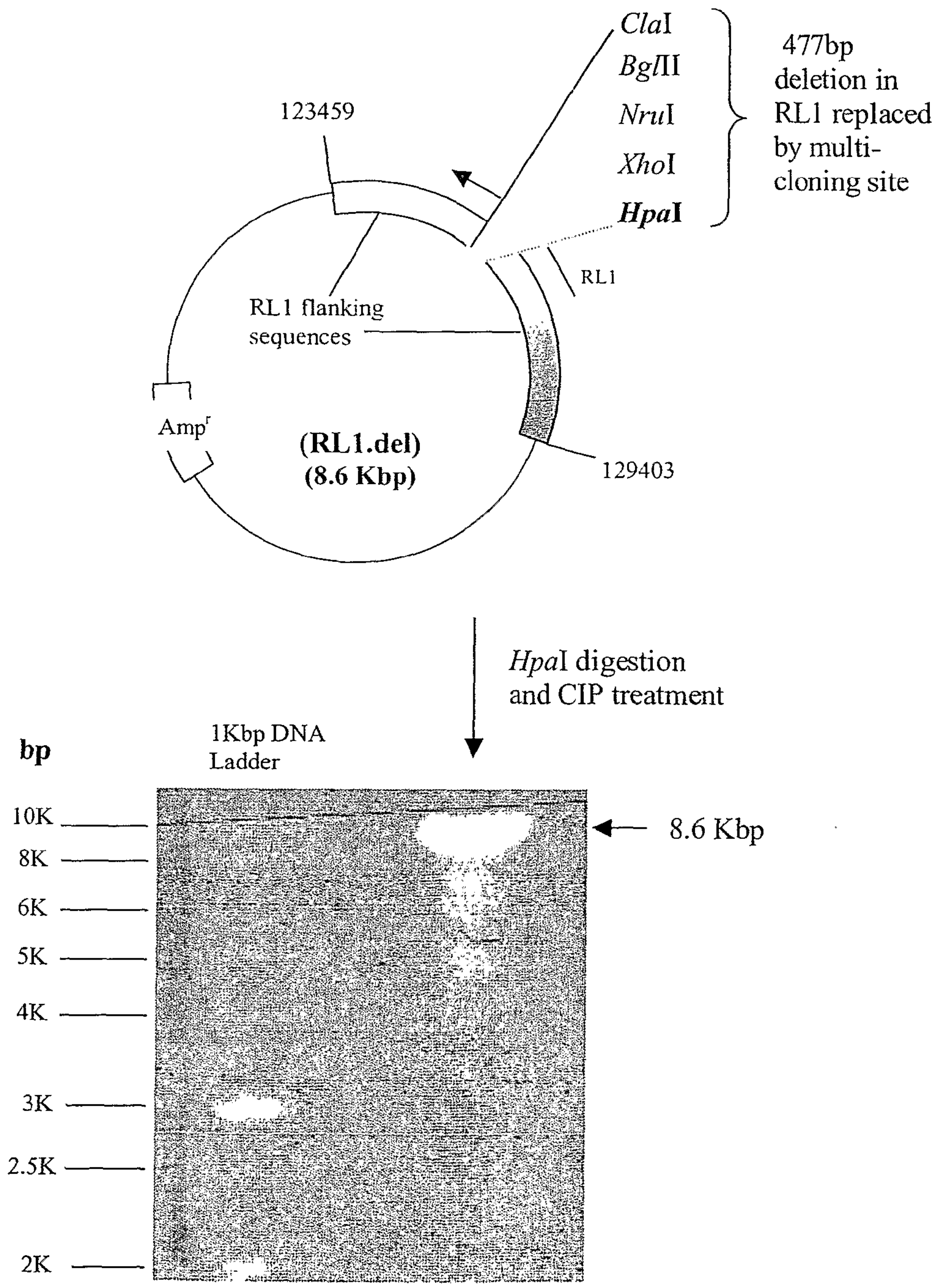
ClaI
BglII
NruI
XhoI
HpaI
477bp deletion in RL1 replaced by multi-cloning site
123459
RL1
RL1 flanking sequences
Amp^r
(RL1.del)
(8.6 Kbp)
129403
HpaI digestion and CIP treatment
bp
1Kbp DNA Ladder
10K
8K
6K
5K
4K
3K
2.5K
2K
8.6 Kbp

# Figure 2

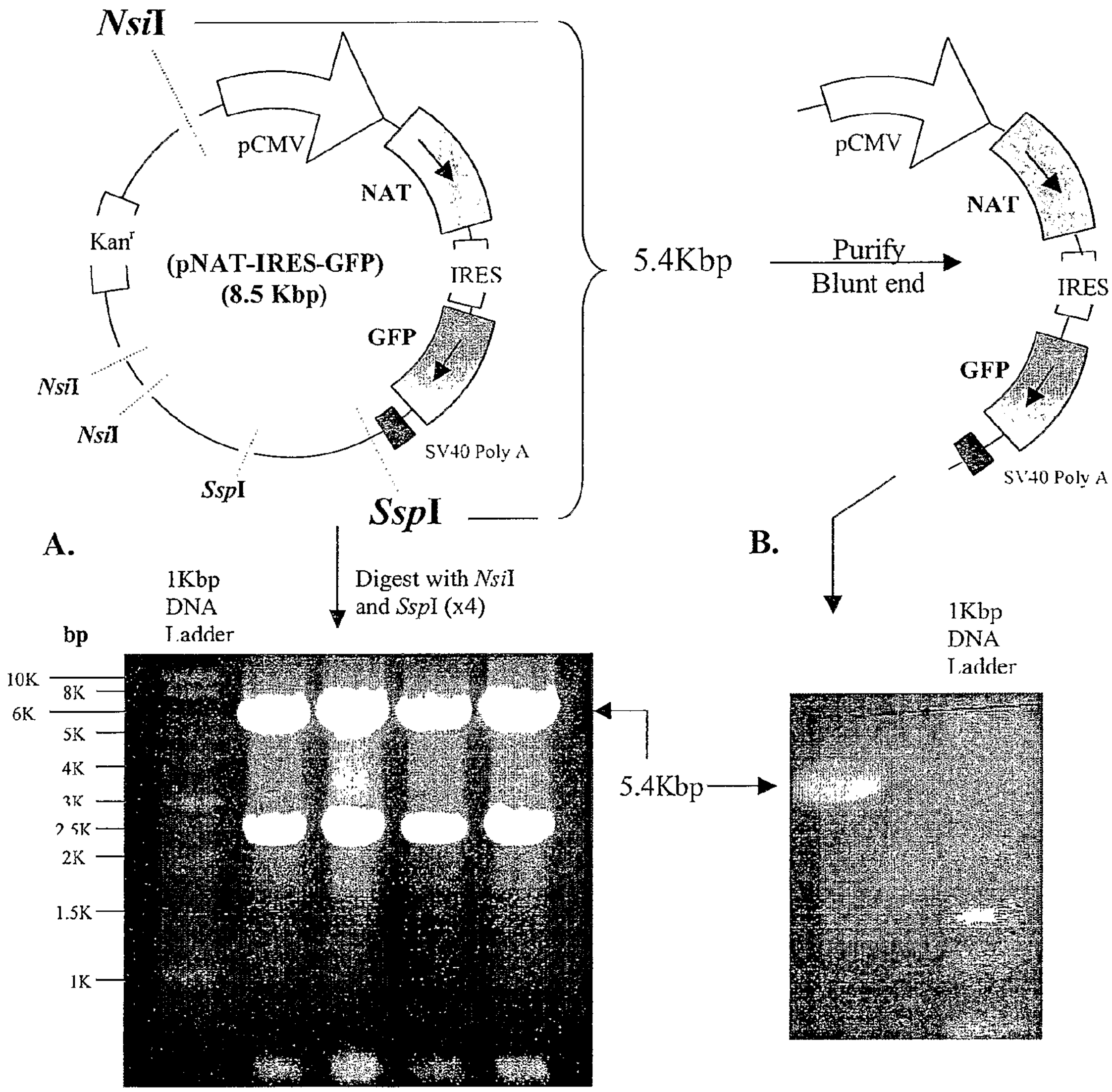
NsiI
pCMV
NAT
Kan^r
(pNAT-IRES-GFP)
(8.5 Kbp)
IRES
GFP
NsiI
NsiI
SspI
SV40 Poly A
SspI
5.4Kbp
Purify
Blunt end
pCMV
NAT
IRES
GFP
SV40 Poly A
A.
B.
1Kbp
DNA
Ladder
Digest with NsiI
and SspI (x4)
bp
10K
8K
6K
5K
4K
3K
2.5K
2K
1.5K
1K
5.4Kbp
1Kbp
DNA
Ladder

# Figure 3

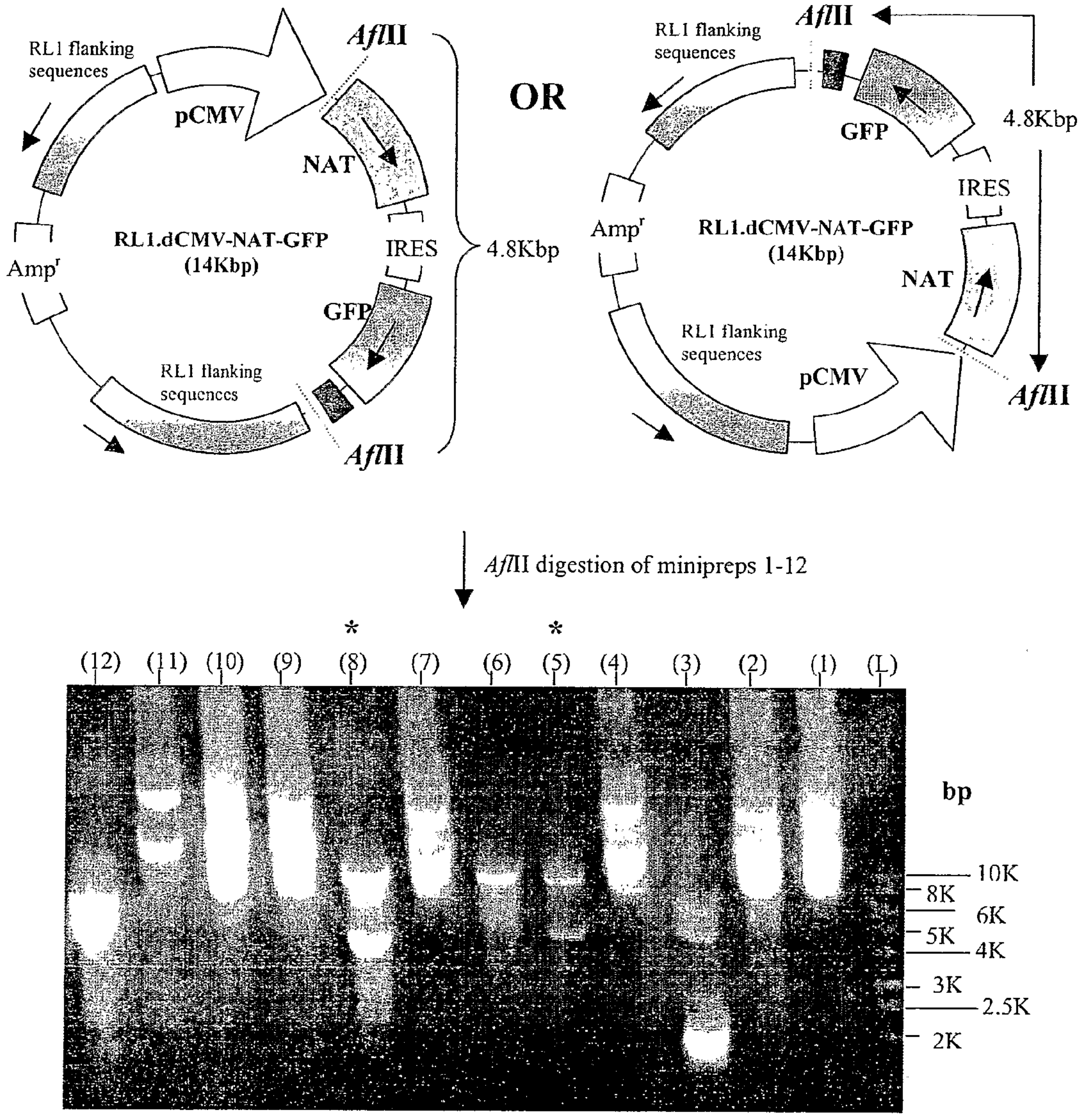

RL1 flanking sequences
pCMV
AflII
NAT
RL1.dCMV-NAT-GFP (14Kbp)
Amp^r
IRES
GFP
RL1 flanking sequences
AflII
4.8Kbp
OR
RL1 flanking sequences
AflII
GFP
4.8Kbp
IRES
Amp^r
RL1.dCMV-NAT-GFP (14Kbp)
NAT
RL1 flanking sequences
pCMV
AflII
AflII digestion of minipreps 1-12
*
*
(12) (11) (10) (9) (8) (7) (6) (5) (4) (3) (2) (1) (L)
bp
10K
8K
6K
5K
4K
3K
2.5K
2K


**Figure 4**

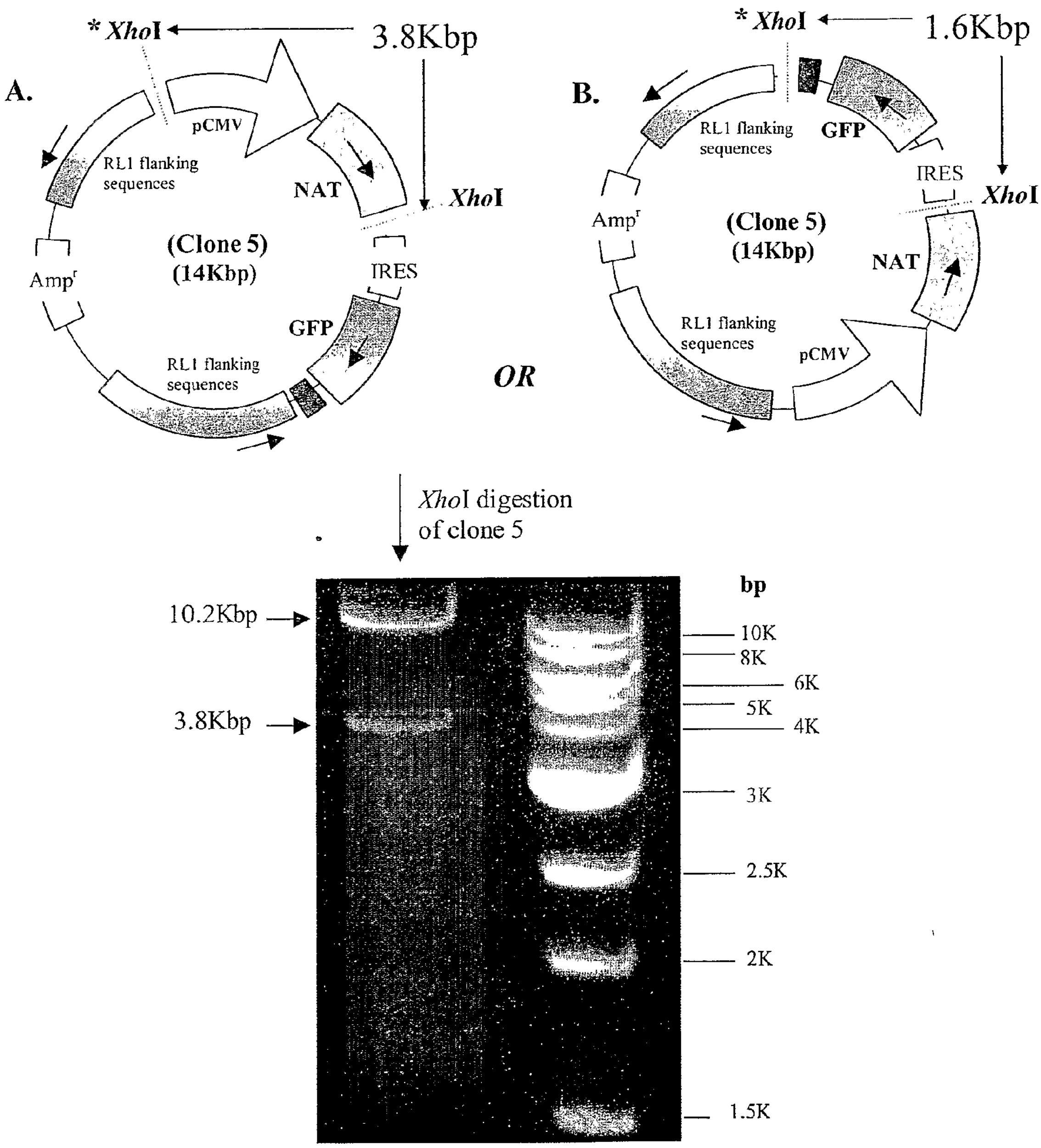
*XhoI
3.8Kbp
A.
pCMV
RL1 flanking sequences
NAT
XhoI
(Clone 5) (14Kbp)
Amp^r
IRES
GFP
RL1 flanking sequences
OR
*XhoI
1.6Kbp
B.
RL1 flanking sequences
GFP
IRES
XhoI
Amp^r
(Clone 5) (14Kbp)
NAT
RL1 flanking sequences
pCMV
XhoI digestion of clone 5
10.2Kbp
3.8Kbp
bp
10K
8K
6K
5K
4K
3K
2.5K
2K
1.5K

**Figure 5**

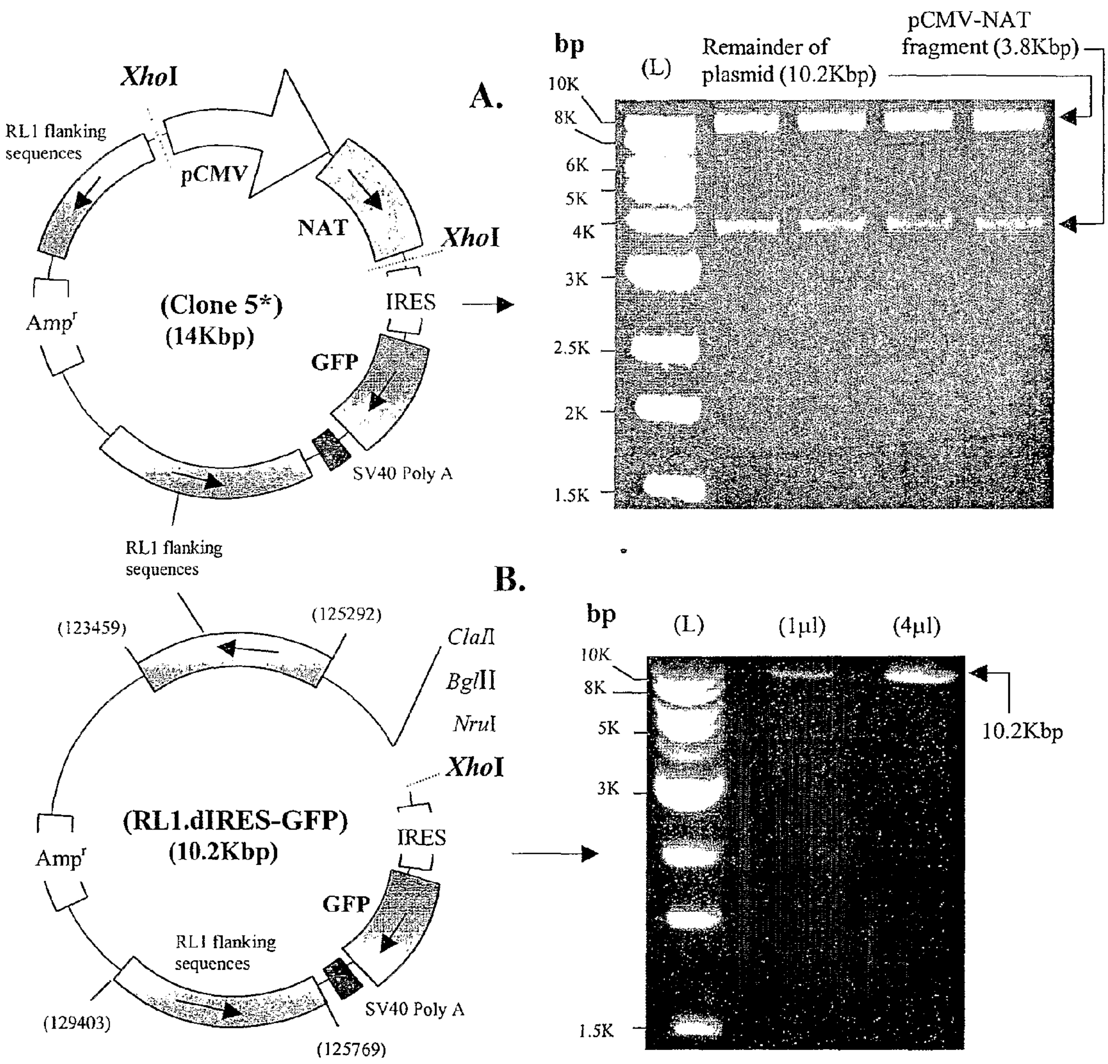
A.
XhoI
RL1 flanking sequences
pCMV
NAT
XhoI
(Clone 5*)
(14Kbp)
Amp^r
IRES
GFP
SV40 Poly A
RL1 flanking sequences
bp
(L)
Remainder of plasmid (10.2Kbp)
pCMV-NAT fragment (3.8Kbp)
10K
8K
6K
5K
4K
3K
2.5K
2K
1.5K
B.
(123459)
(125292)
ClaII
BglII
NruI
XhoI
(RL1.dIRES-GFP)
(10.2Kbp)
Amp^r
IRES
GFP
RL1 flanking sequences
SV40 Poly A
(129403)
(125769)
bp
(L)
(1μl)
(4μl)
10K
8K
5K
3K
1.5K
10.2Kbp

# Figure 6

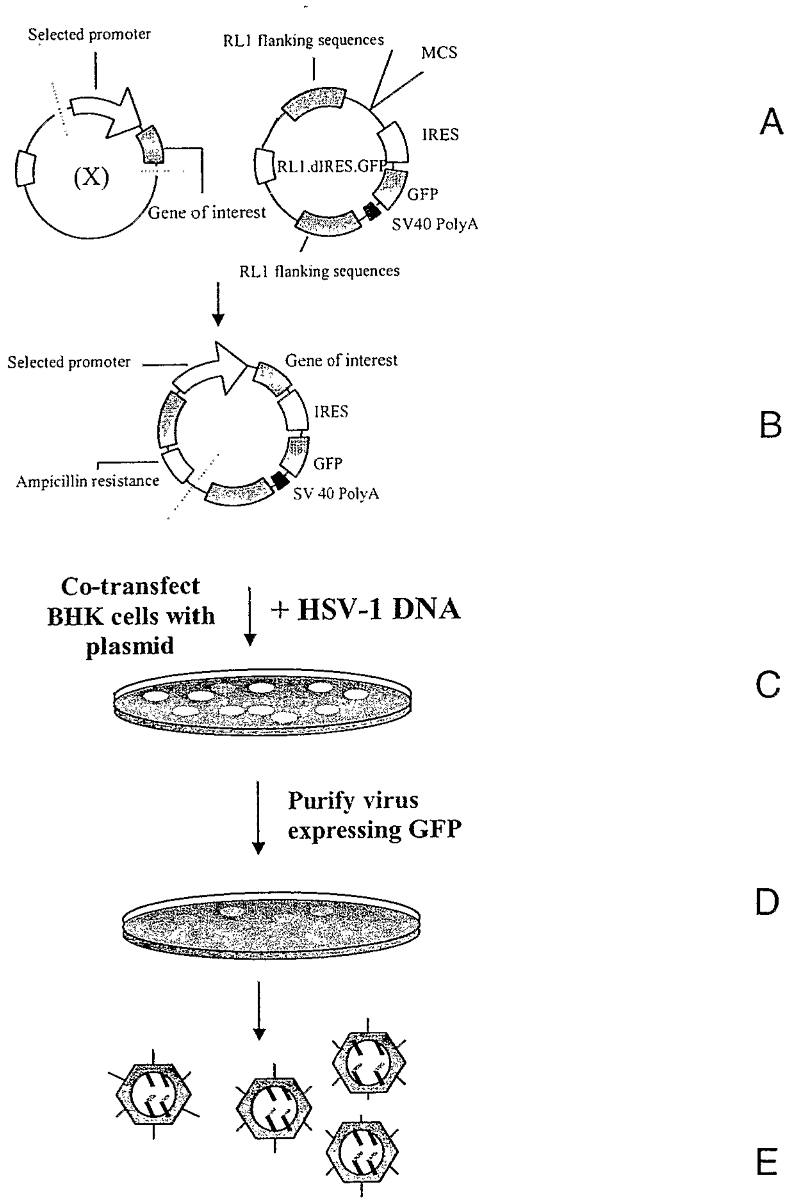
Selected promoter
RL1 flanking sequences
MCS
IRES
(X)
RL1.dIRES.GFP
GFP
Gene of interest
SV40 PolyA
RL1 flanking sequences
A
Selected promoter
Gene of interest
IRES
GFP
Ampicillin resistance
SV 40 PolyA
B
Co-transfect BHK cells with plasmid
+ HSV-1 DNA
C
Purify virus expressing GFP
D
E

Figure 7

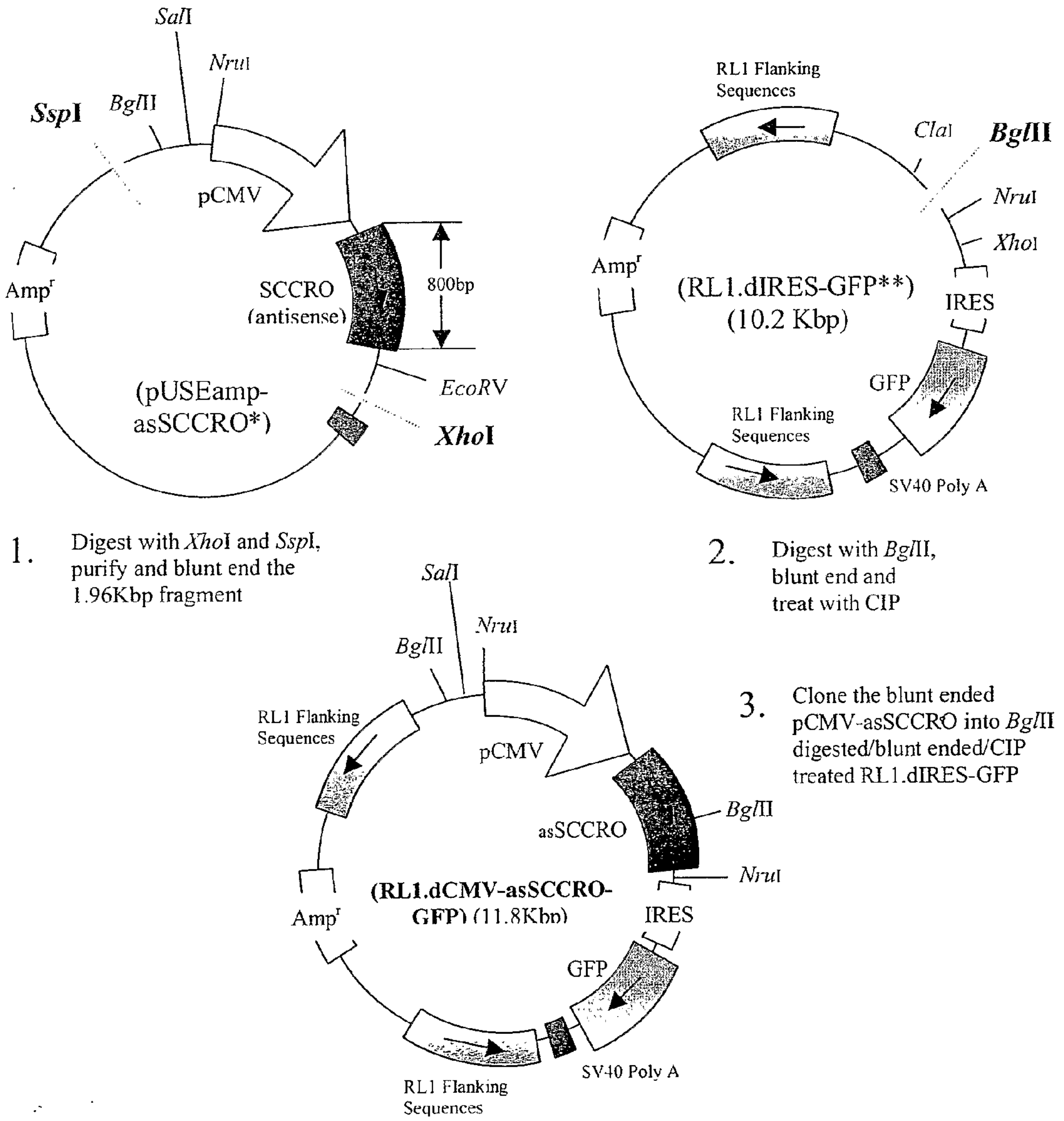
SalI
NruI
SspI
BglII
pCMV
Amp^r
SCCRO
(antisense)
800bp
(pUSEamp-
asSCCRO*)
EcoRV
XhoI
RL1 Flanking
Sequences
ClaI
BglII
NruI
XhoI
Amp^r
(RL1.dIRES-GFP**)
(10.2 Kbp)
IRES
GFP
RL1 Flanking
Sequences
SV40 Poly A
1. Digest with XhoI and SspI,
purify and blunt end the
1.96Kbp fragment
2. Digest with BglII,
blunt end and
treat with CIP
SalI
NruI
BglII
RL1 Flanking
Sequences
pCMV
asSCCRO
BglII
NruI
Amp^r
(RL1.dCMV-asSCCRO-
GFP) (11.8Kbp)
IRES
GFP
SV40 Poly A
RL1 Flanking
Sequences
3. Clone the blunt ended
pCMV-asSCCRO into BglII
digested/blunt ended/CIP
treated RL1.dIRES-GFP

**Figure 8**

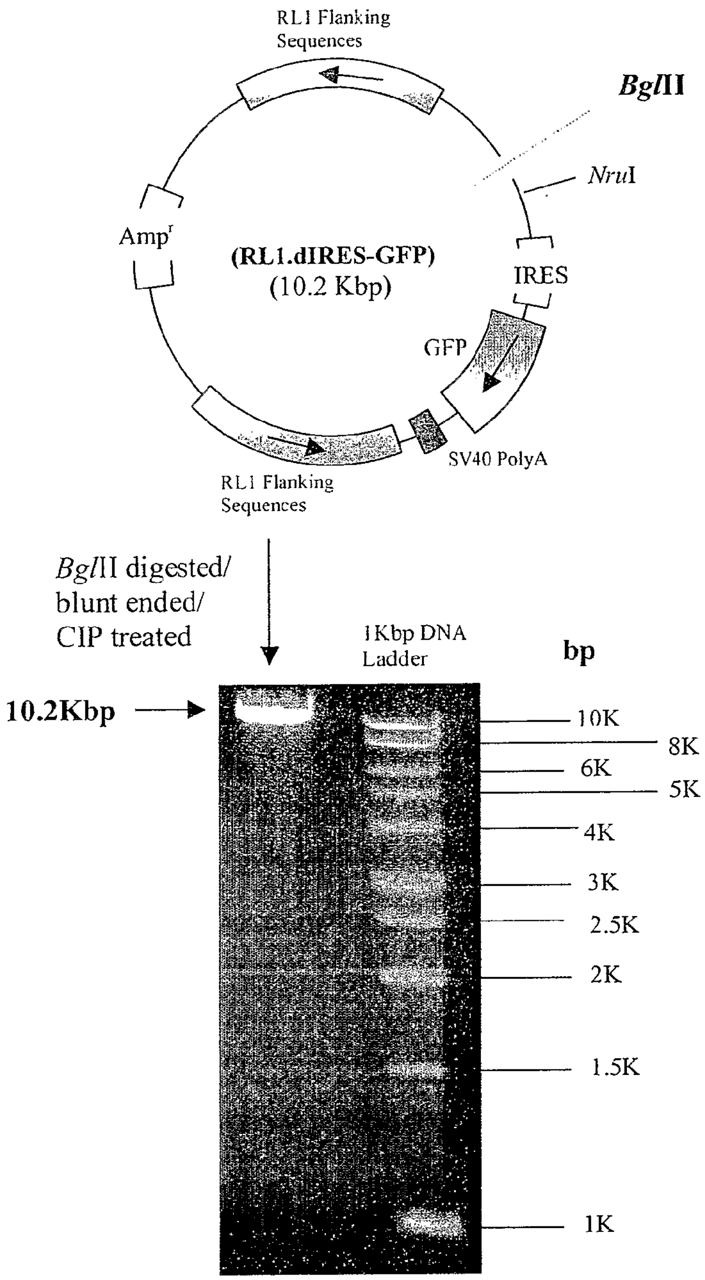
RL1 Flanking Sequences
BglII
NruI
Amp^r
(RL1.dIRES-GFP)
(10.2 Kbp)
IRES
GFP
SV40 PolyA
RL1 Flanking Sequences
BglII digested/ blunt ended/ CIP treated
1Kbp DNA Ladder
bp
10.2Kbp
10K
8K
6K
5K
4K
3K
2.5K
2K
1.5K
1K

**Figure 9**

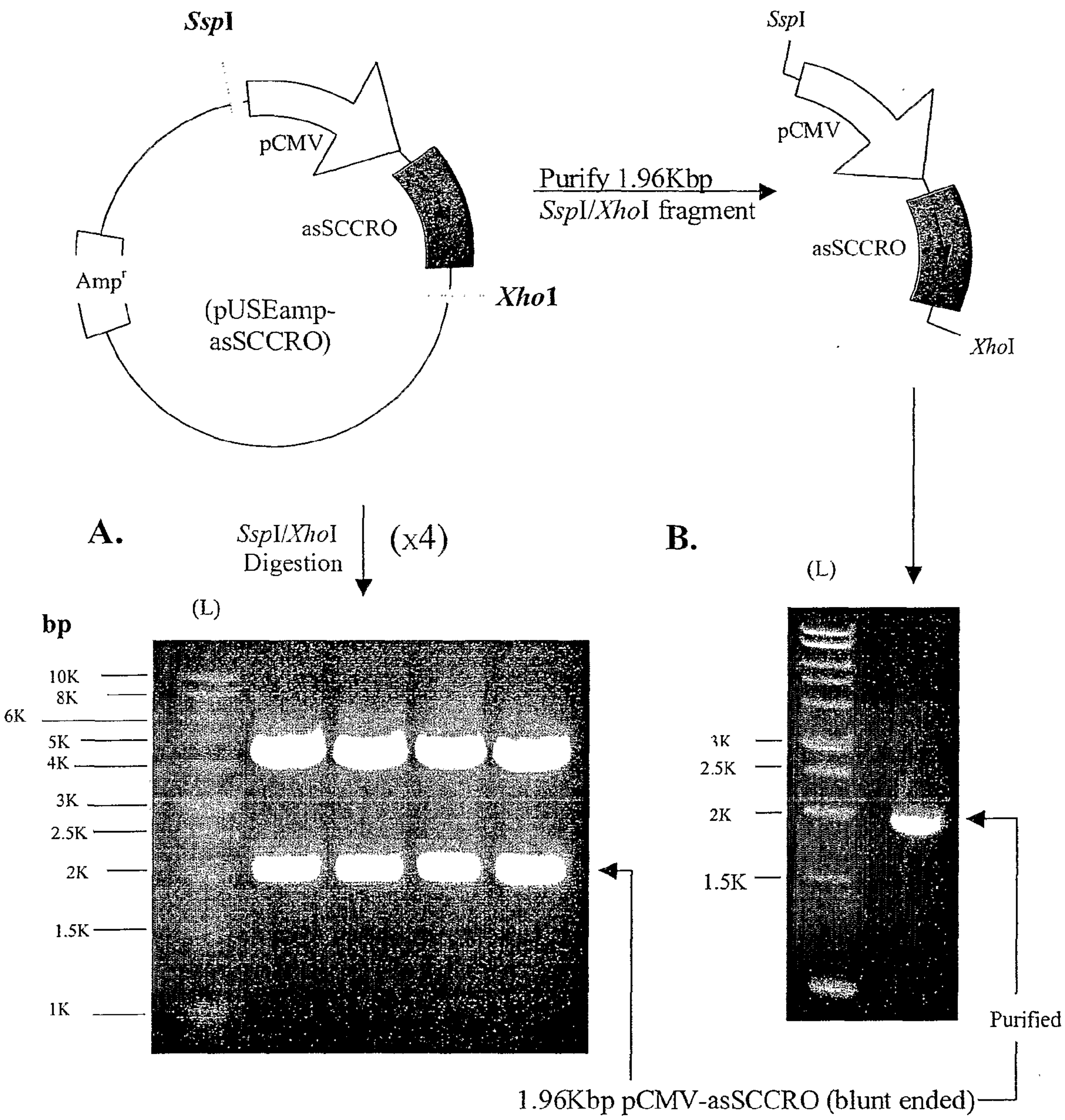
SspI
pCMV
asSCCRO
Ampr
(pUSEamp-asSCCRO)
XhoI
Purify 1.96Kbp SspI/XhoI fragment
SspI
pCMV
asSCCRO
XhoI
A.
SspI/XhoI Digestion
(x4)
B.
(L)
bp
10K
8K
6K
5K
4K
3K
2.5K
2K
1.5K
1K
(L)
3K
2.5K
2K
1.5K
Purified
1.96Kbp pCMV-asSCCRO (blunt ended)

**Figure 10**

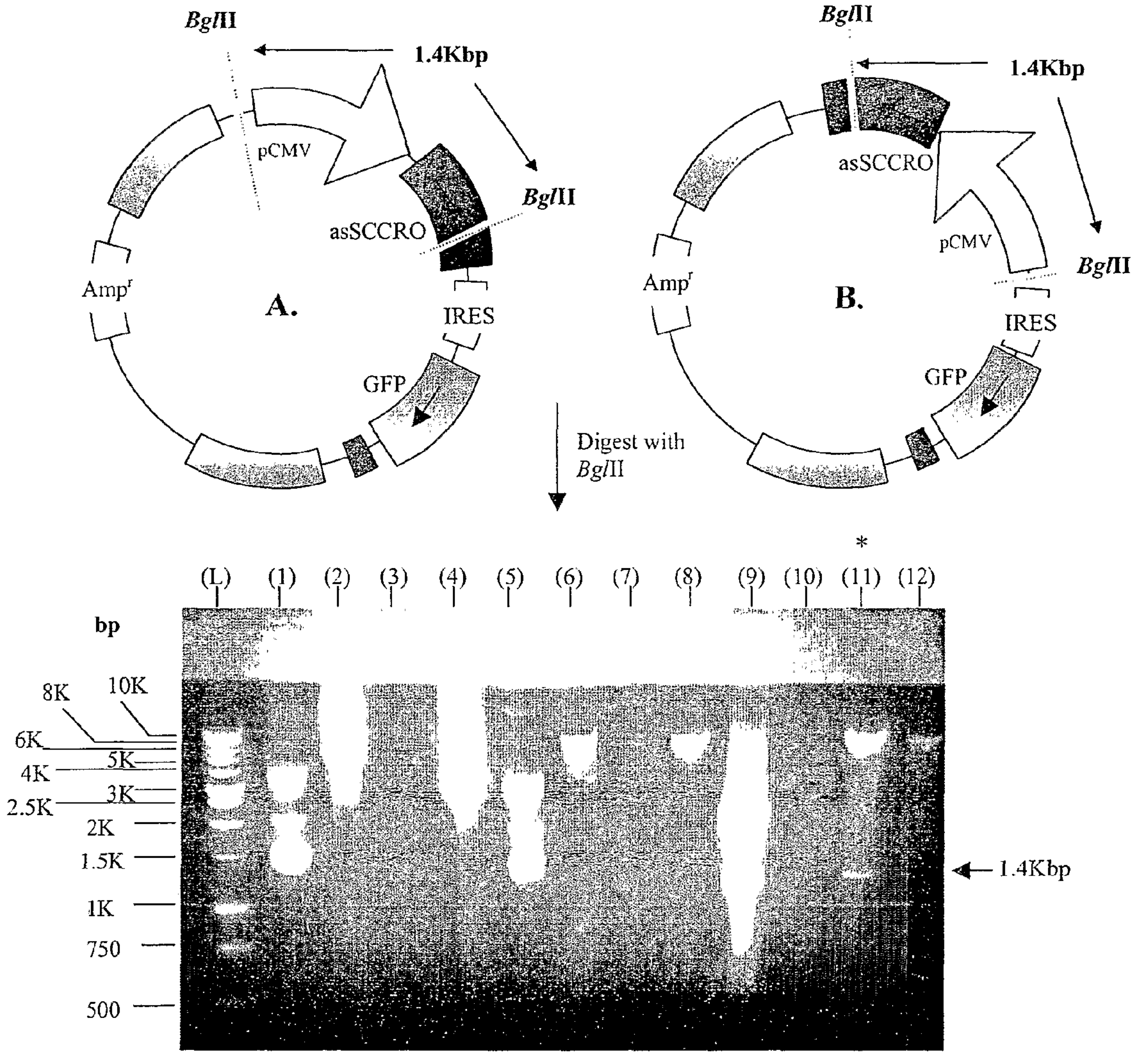
BglII
1.4Kbp
pCMV
asSCCRO
BglII
Amp^r
A.
IRES
GFP
BglII
1.4Kbp
asSCCRO
pCMV
BglII
Amp^r
B.
IRES
GFP
Digest with
BglII
*
(L) (1) (2) (3) (4) (5) (6) (7) (8) (9) (10) (11) (12)
bp
8K
10K
6K
5K
4K
3K
2.5K
2K
1.5K
1K
750
500
1.4Kbp

Figure 11

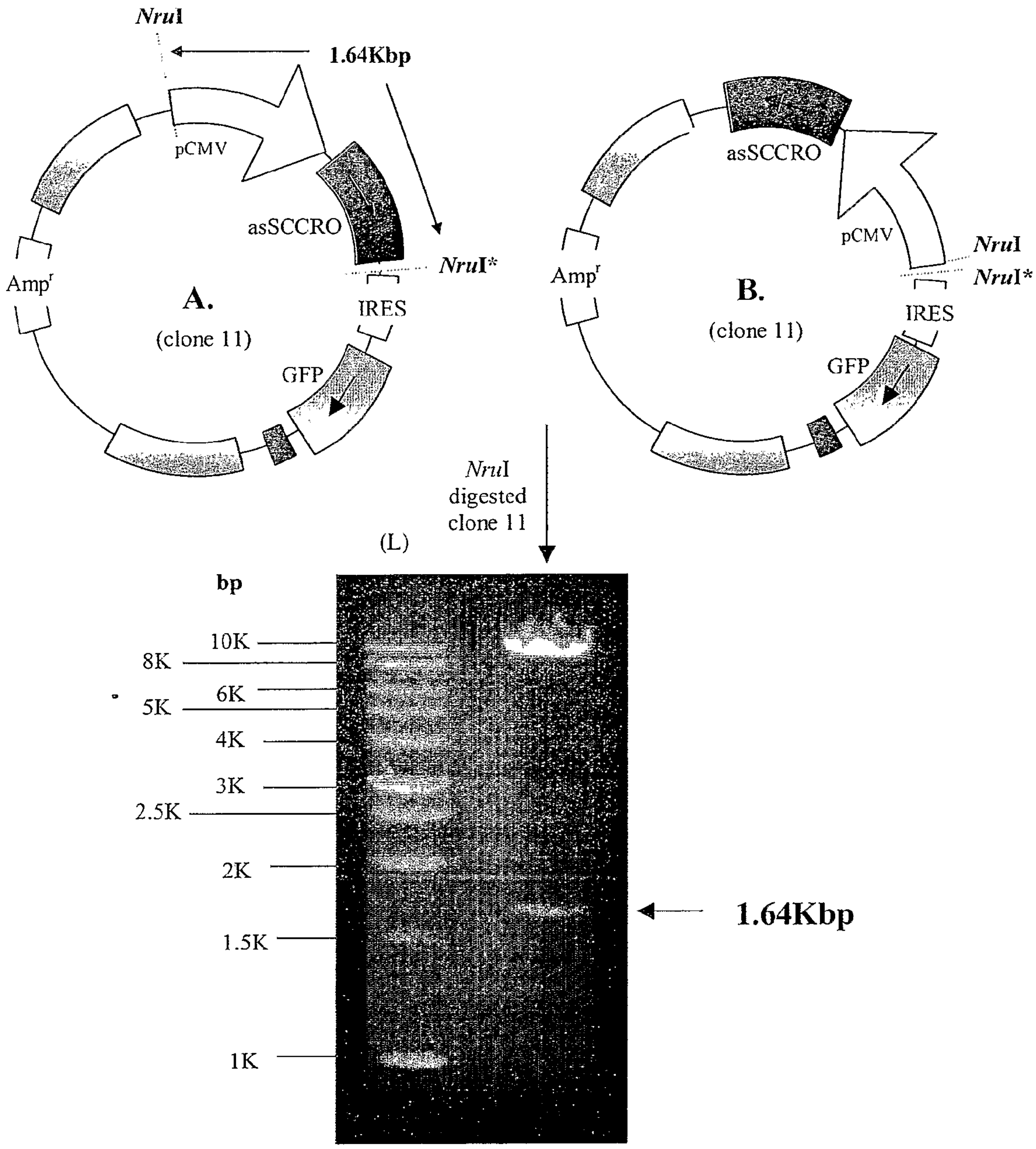
NruI
1.64Kbp
pCMV
asSCCRO
NruI*
Amp^r
A.
(clone 11)
IRES
GFP
asSCCRO
pCMV
NruI
NruI*
Amp^r
B.
(clone 11)
IRES
GFP
NruI
digested
clone 11
(L)
bp
10K
8K
6K
5K
4K
3K
2.5K
2K
1.5K
1K
1.64Kbp

# Figure 12

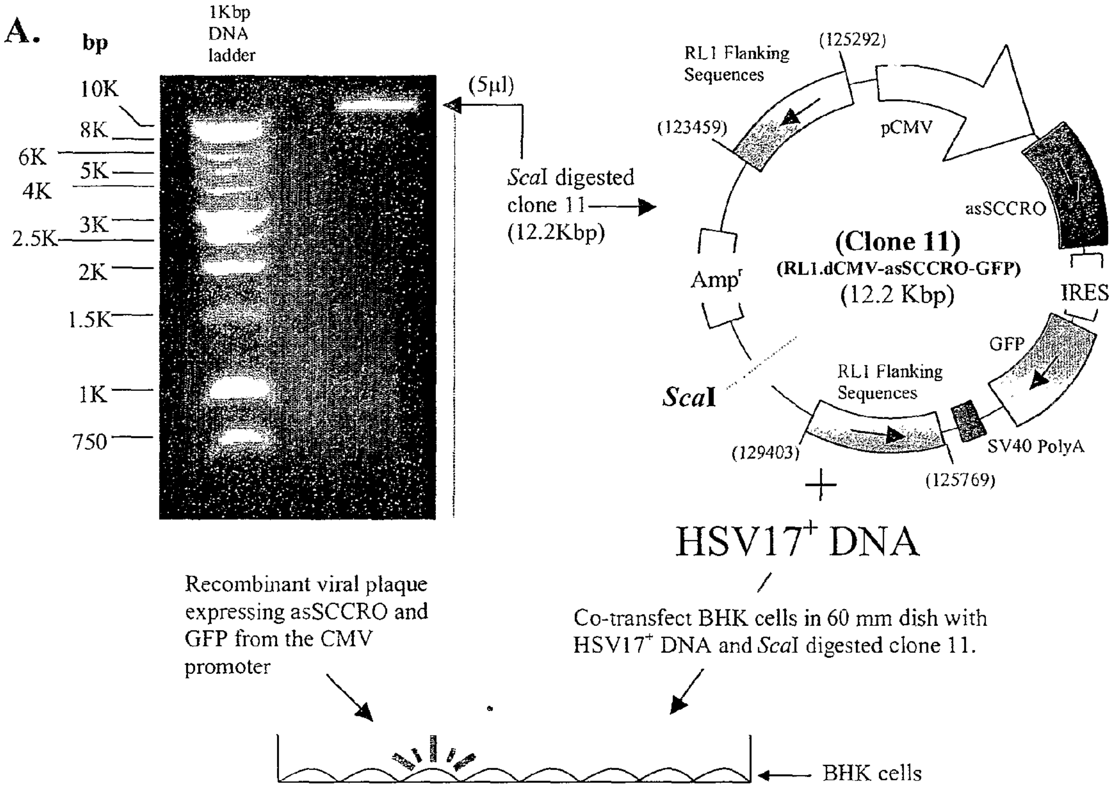


**B.**

| HSV1716/CMV-asSCCRO/GFP Fraction | Titre |
|---|---|
| Combined | $1.2 \times 10^{10}$ pfu/ml |

**Figure 13**

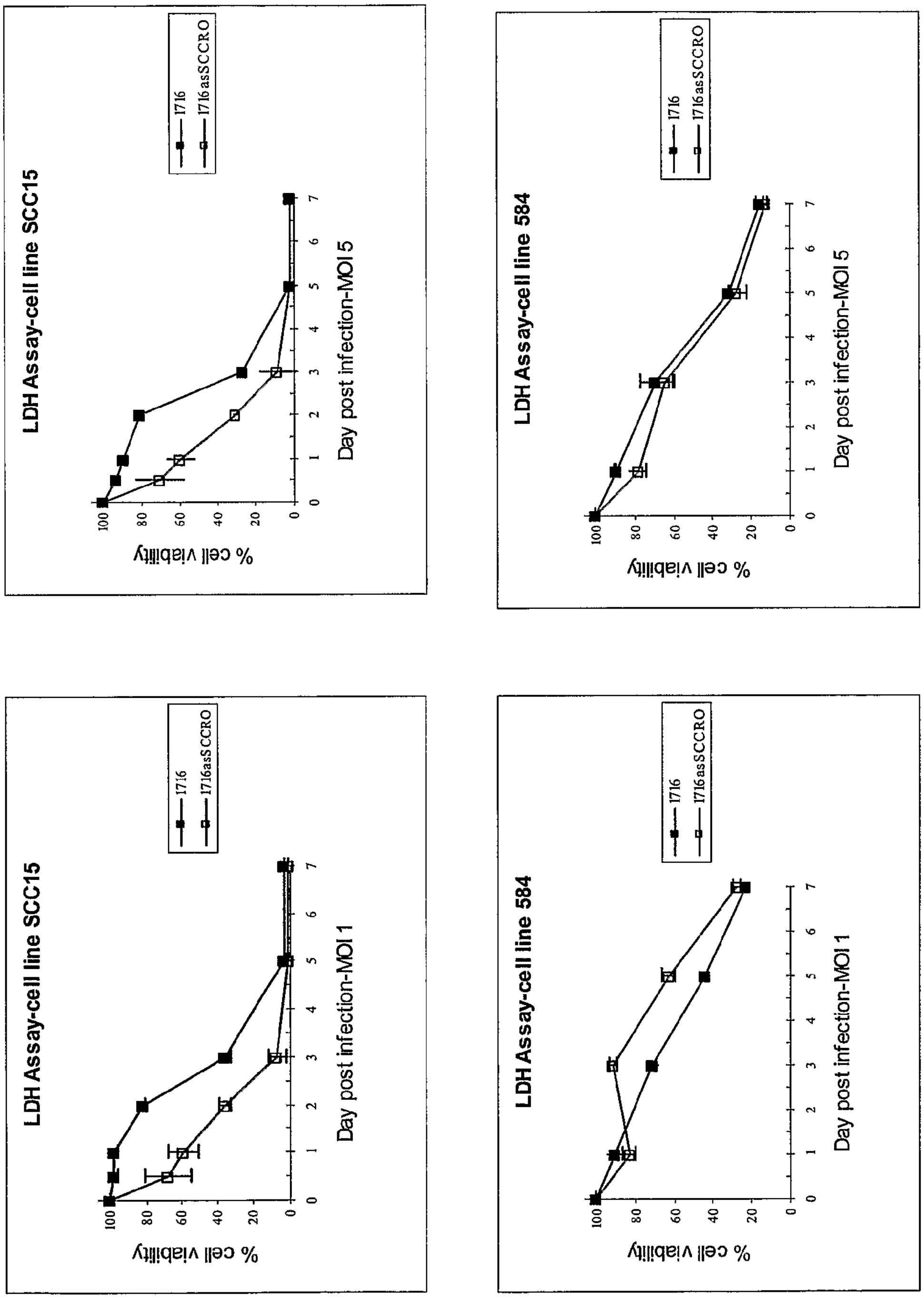


SCC15

584

**Figure 14**

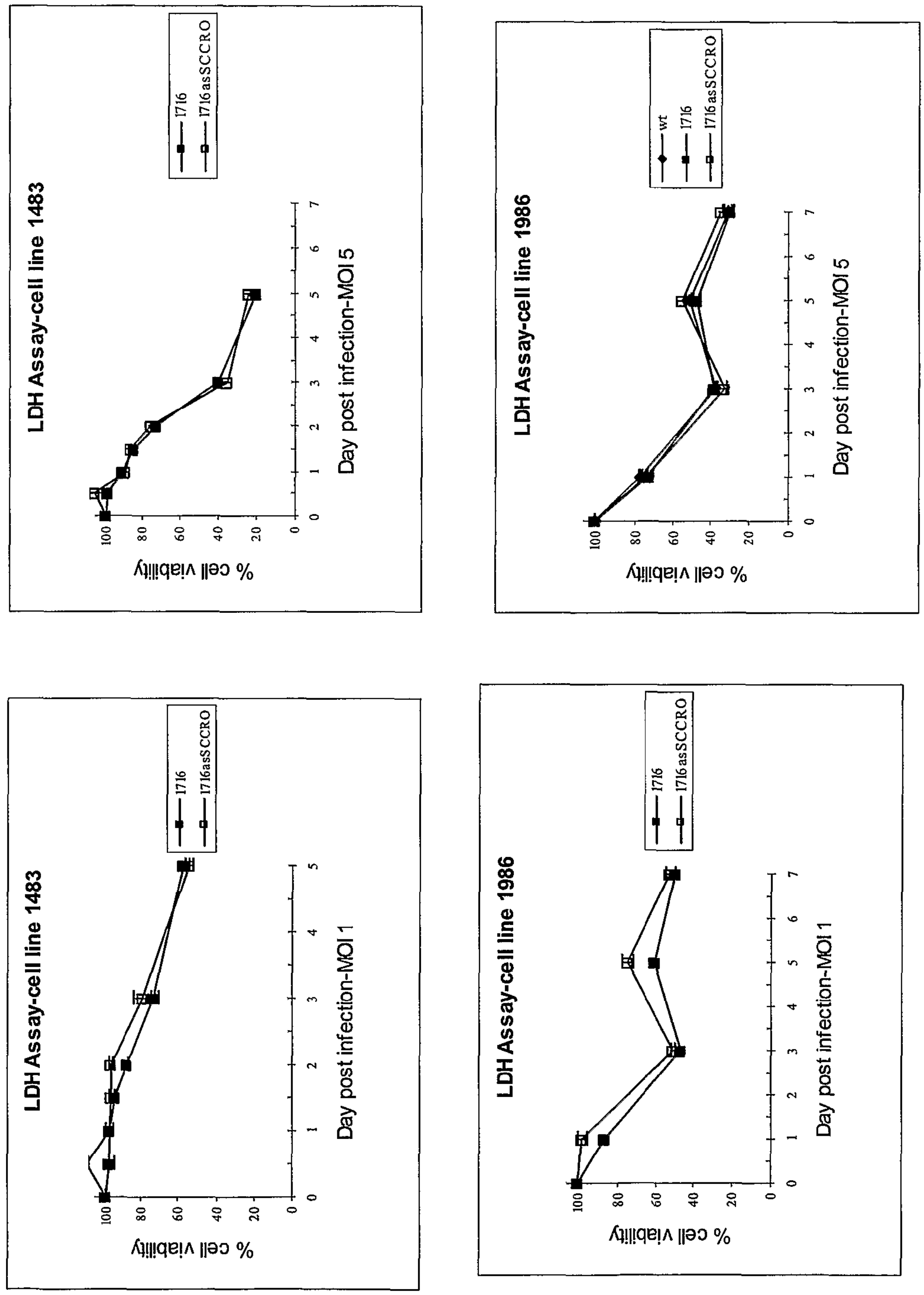


1483

1986

# Figure 15

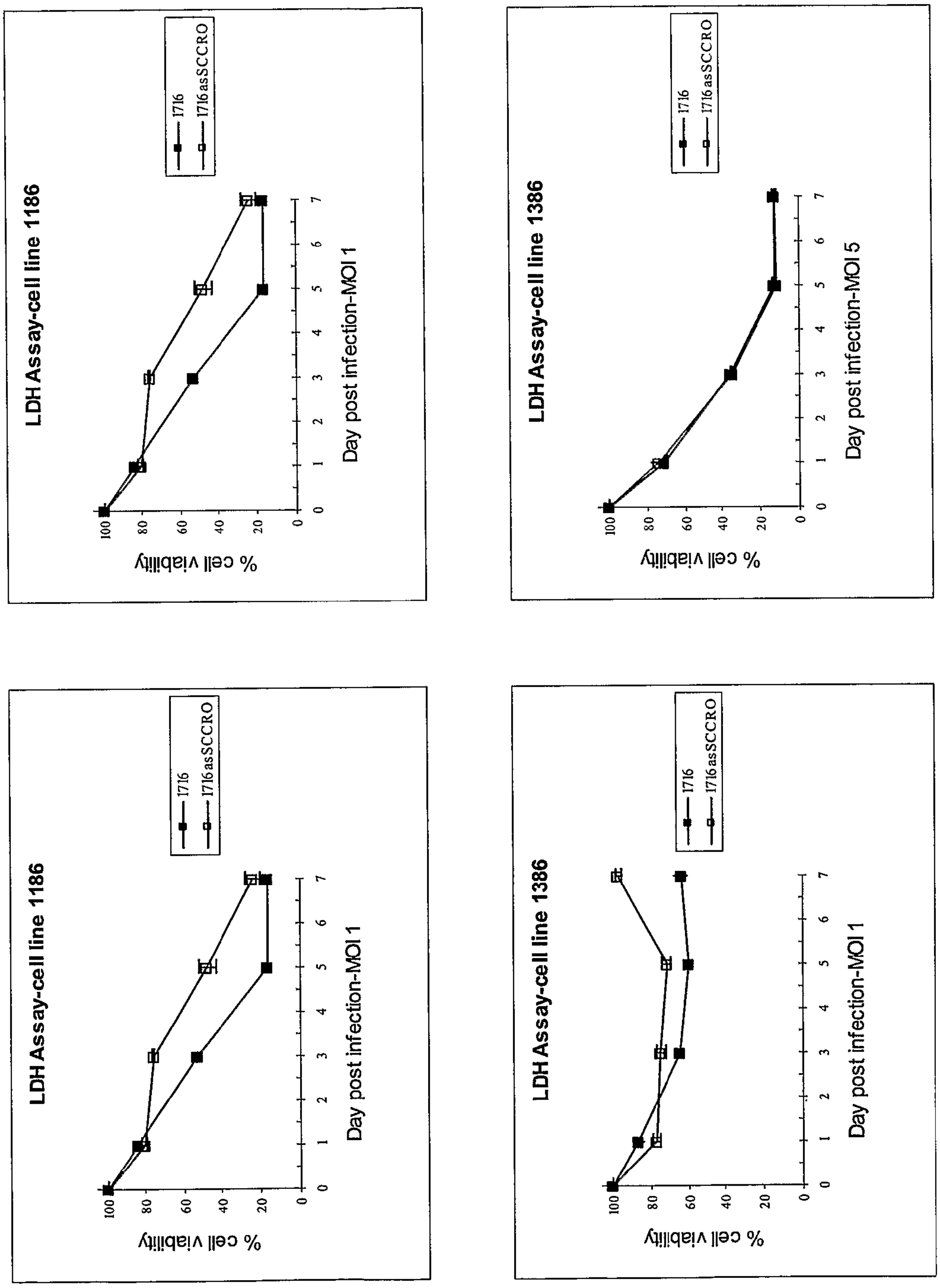


1186

1386

**Figure 16**

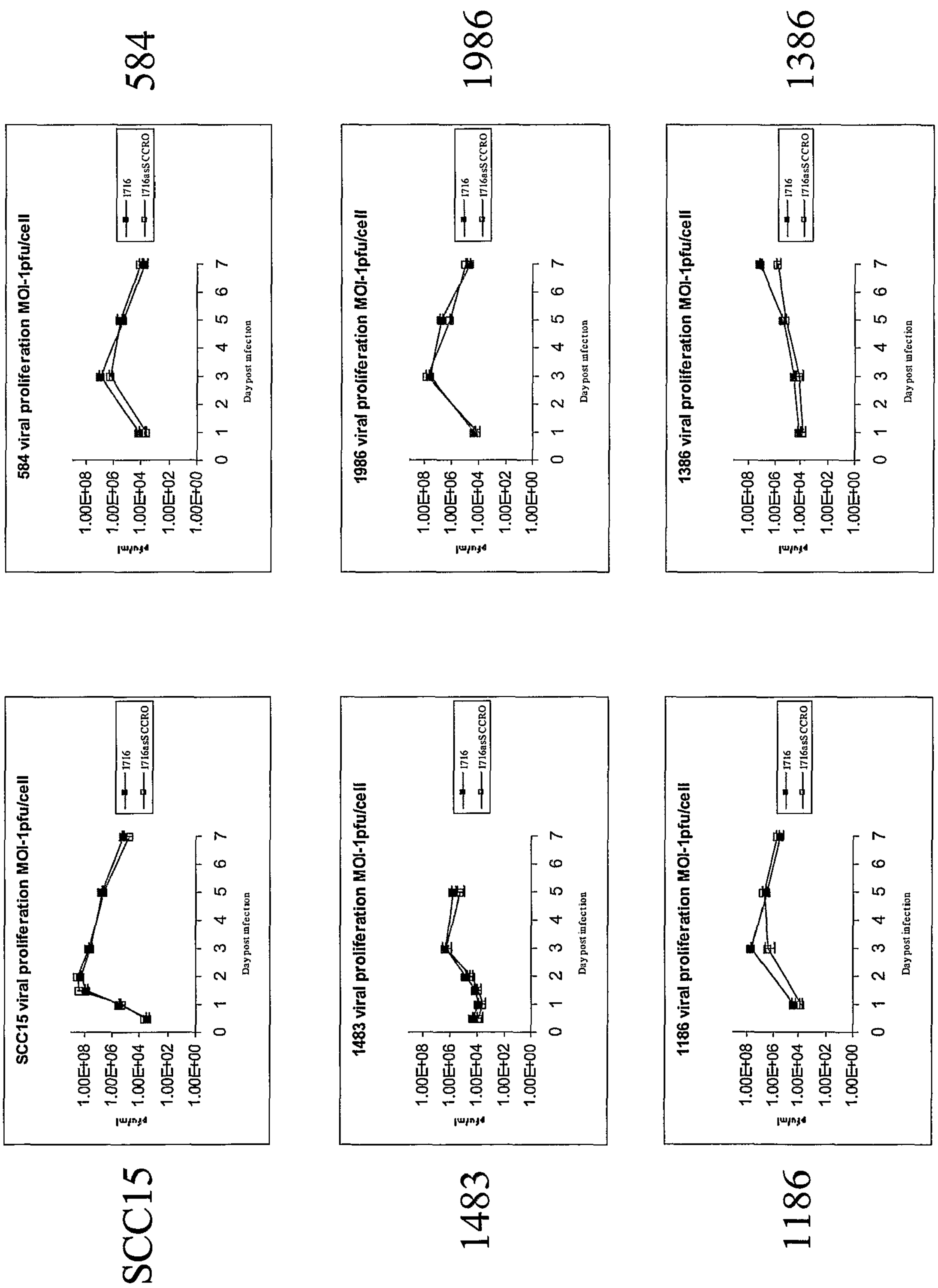
584
584 viral proliferation MOI-1pfu/cell
1986
1986 viral proliferation MOI-1pfu/cell
1386
1386 viral proliferation MOI-1pfu/cell
SCC15
SCC15 viral proliferation MOI-1pfu/cell
1483
1483 viral proliferation MOI-1pfu/cell
1186
1186 viral proliferation MOI-1pfu/cell
pfu/ml
1.00E+08
1.00E+06
1.00E+04
1.00E+02
1.00E+00
0 1 2 3 4 5 6 7
Day post infection
1716
1716asSCCRO

# Figure 17

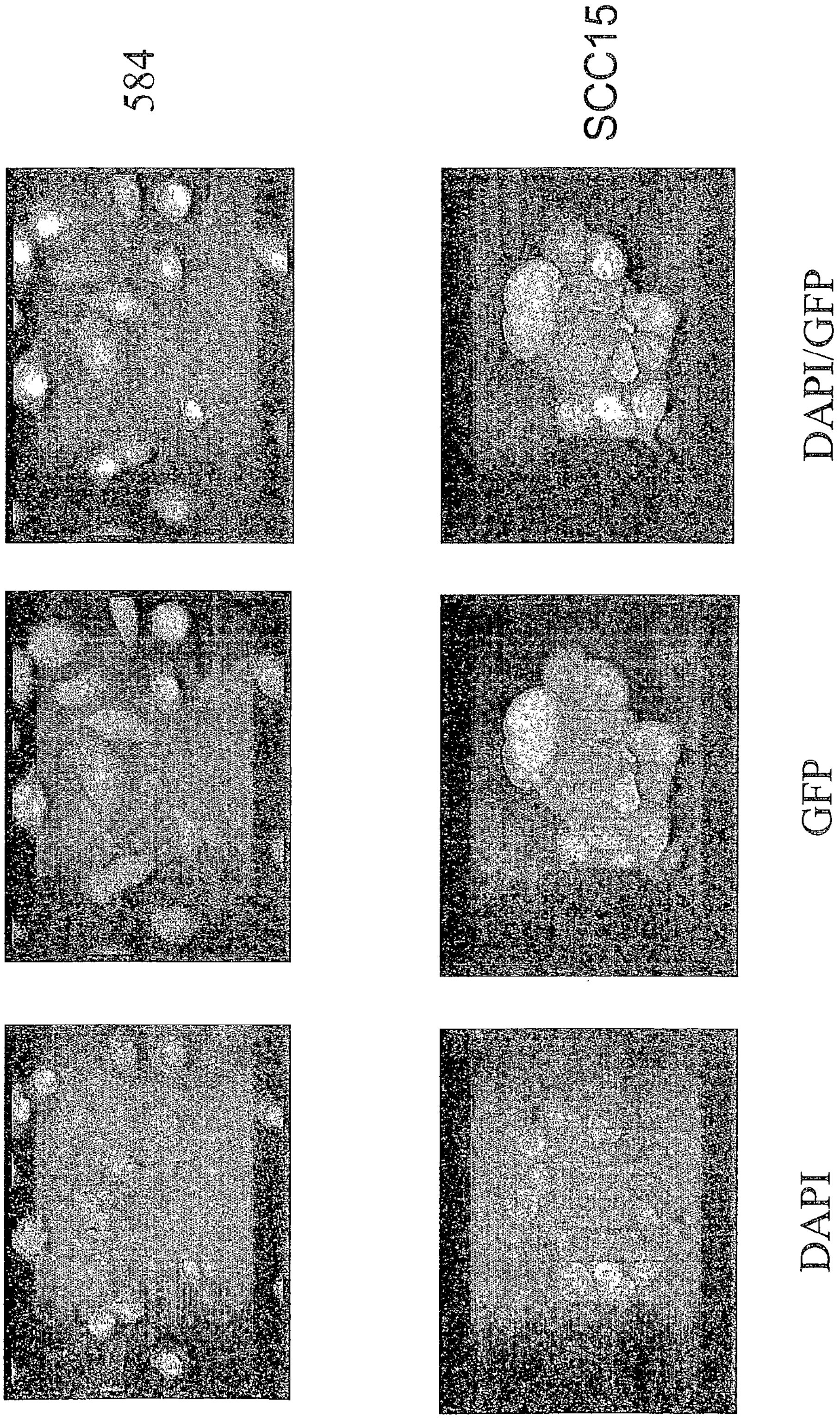


Figure 18

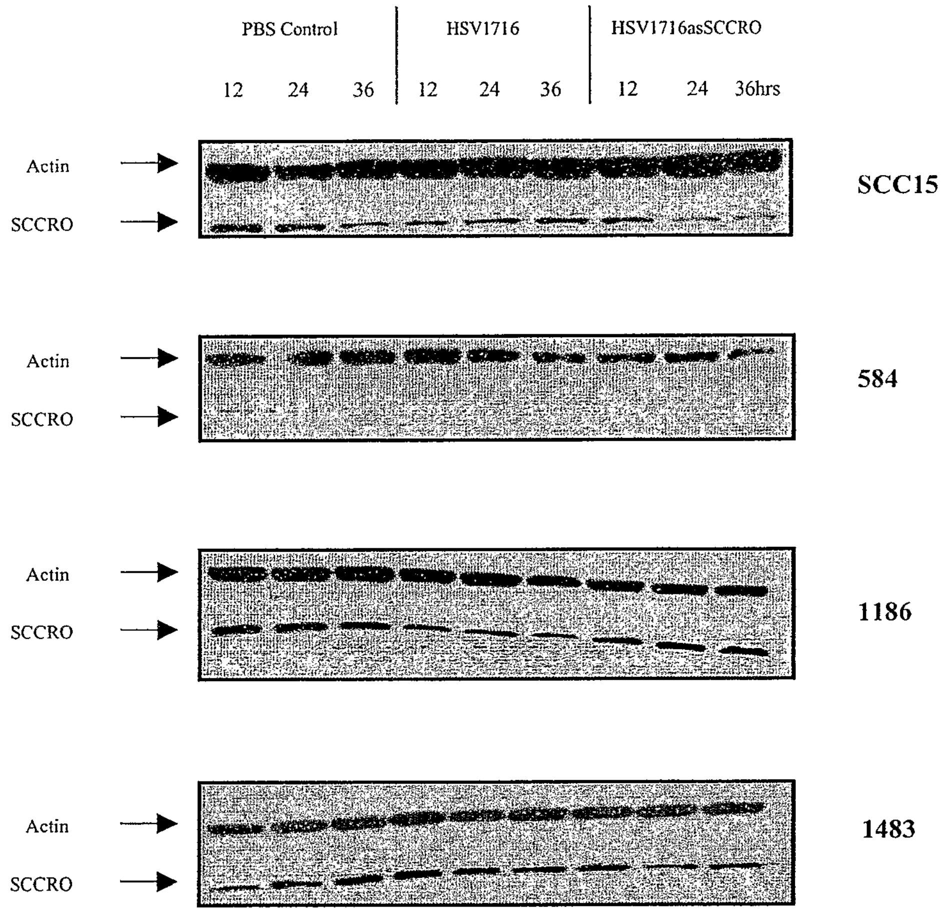
PBS Control
HSV1716
HSV1716asSCCRO
12
24
36
12
24
36
12
24
36hrs
Actin
SCCRO
SCC15
Actin
SCCRO
584
Actin
SCCRO
1186
Actin
SCCRO
1483

# Figure 19

SCC15

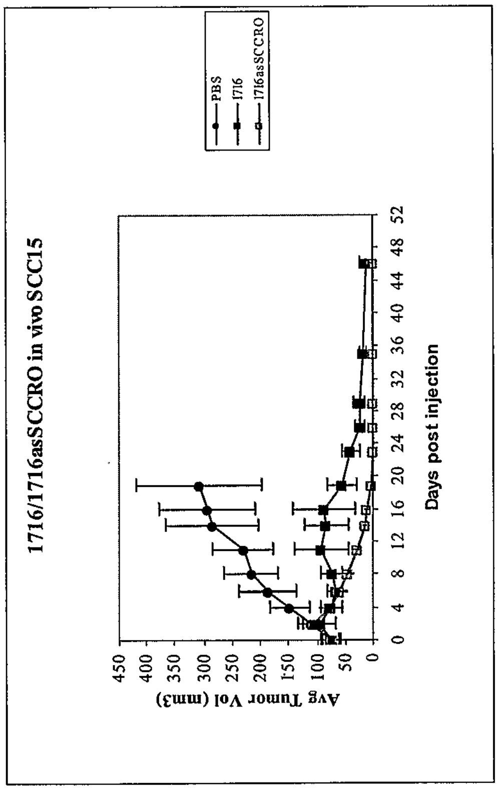


584

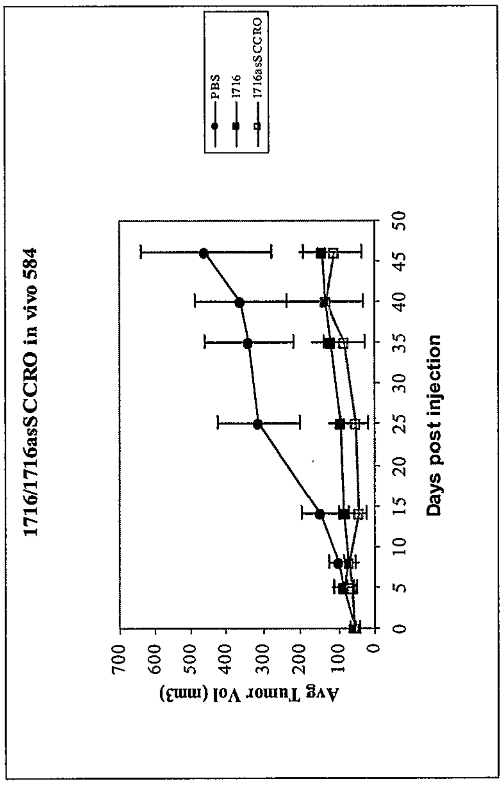


**Figure 20**

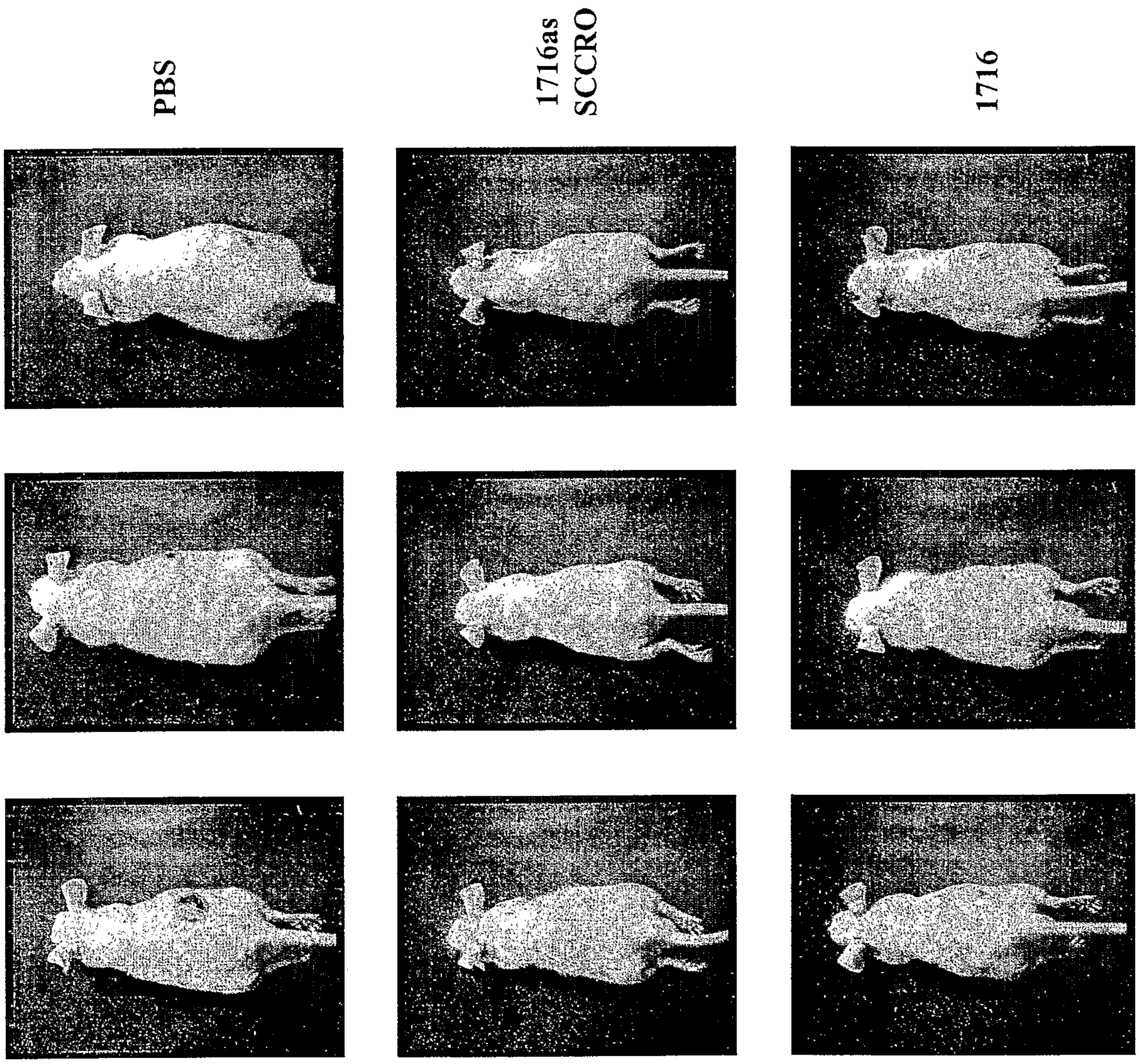


# Figure 21

# SEQ ID No. 01

```
cgccgtccat tcgctgcgga gccggaggag gaggggagag gcctggagga caccaac atg     60
                                                              Met
                                                               1

aac aag ttg aaa tca tcg cag aag gat aaa gtt cgt cag ttt atg atc       108
Asn Lys Leu Lys Ser Ser Gln Lys Asp Lys Val Arg Gln Phe Met Ile
            5                  10                  15

ttc aca caa tct agt gaa aaa aca gca gta agt tgt ctt tct caa aat       156
Phe Thr Gln Ser Ser Glu Lys Thr Ala Val Ser Cys Leu Ser Gln Asn
        20                  25                  30

gac tgg aag tta gat gtt gca aca gat aat ttt ttc caa aat cct gaa       204
Asp Trp Lys Leu Asp Val Ala Thr Asp Asn Phe Phe Gln Asn Pro Glu
    35                  40                  45

ctt tat ata cga gag agt gta aaa gga tca ttg gac agg aag aag tta       252
Leu Tyr Ile Arg Glu Ser Val Lys Gly Ser Leu Asp Arg Lys Lys Leu
 50                  55                  60                  65

gaa cag ctg tac aat aga tac aaa gac cct caa gat gag aat aaa att       300
Glu Gln Leu Tyr Asn Arg Tyr Lys Asp Pro Gln Asp Glu Asn Lys Ile
                70                  75                  80

gga ata gat ggc ata cag cag ttc tgt gat gac ctg gca ctc gat cca       348
Gly Ile Asp Gly Ile Gln Gln Phe Cys Asp Asp Leu Ala Leu Asp Pro


gcc agc att agt gtg ttg att att gca tgg aag ttc aga gca gca aca       396
Ala Ser Ile Ser Val Leu Ile Ile Ala Trp Lys Phe Arg Ala Ala Thr
        100                 105                 110

cag tgc gag ttc tcc aaa cag gag ttc atg gat ggc atg aca gaa tta       444
Gln Cys Glu Phe Ser Lys Gln Glu Phe Met Asp Gly Met Thr Glu Leu
    115                 120                 125

gga tgt gac agc ata gaa aaa cta aag gcc cag ata ccc aag atg gaa       492
Gly Cys Asp Ser Ile Glu Lys Leu Lys Ala Gln Ile Pro Lys Met Glu
130                 135                 140                 145

caa gaa ttg aaa gaa cca gga cga ttt aag gat ttt tac cag ttt act       540
Gln Glu Leu Lys Glu Pro Gly Arg Phe Lys Asp Phe Tyr Gln Phe Thr
                150                 155                 160

ttt aat ttt gca aag aat cca gga caa aaa gga tta gat cta gaa atg       588
Phe Asn Phe Ala Lys Asn Pro Gly Gln Lys Gly Leu Asp Leu Glu Met
            165                 170                 175

gcc att gcc tac tgg aac tta gtg ctt aat gga aga ttt aaa ttc tta       636
Ala Ile Ala Tyr Trp Asn Leu Val Leu Asn Gly Arg Phe Lys Phe Leu
        180                 185                 190

gac tta tgg aat aaa ttt ttg ttg gaa cat cat aaa cga tca ata cca       684
Asp Leu Trp Asn Lys Phe Leu Leu Glu His His Lys Arg Ser Ile Pro
    195                 200                 205

aaa gac act tgg aat ctt ctt tta gac ttc agt acg atg att gca gat       732
Lys Asp Thr Trp Asn Leu Leu Leu Asp Phe Ser Thr Met Ile Ala Asp
210                 215                 220                 225

gac atg tct aat tat gat gaa gaa gga gca tgg cct gtt ctt att gat       780
Asp Met Ser Asn Tyr Asp Glu Glu Gly Ala Trp Pro Val Leu Ile Asp
                230                 235                 240

gac ttt gtg gaa ttt gca cgc cct caa att gct ggg aca aaa agt aca       828
Asp Phe Val Glu Phe Ala Arg Pro Gln Ile Ala Gly Thr Lys Ser Thr
            245                 250                 255

aca gtg tag cactaaagga accttctaga atgtacatag tctgtacaat               877
Thr Val  *

aaatacaaca gaaaattgca cagtcaattt ctgctggctg g                          918
```

Figure 22a

SEQ ID No. 02

```
Met Asn Lys Leu Lys Ser Ser Gln Lys Asp Lys Val Arg Gln Phe Met
1               5                   10                  15

Ile Phe Thr Gln Ser Ser Glu Lys Thr Ala Val Ser Cys Leu Ser Gln
            20                  25                  30

Asn Asp Trp Lys Leu Asp Val Ala Thr Asp Asn Phe Phe Gln Asn Pro
        35                  40                  45

Glu Leu Tyr Ile Arg Glu Ser Val Lys Gly Ser Leu Asp Arg Lys Lys
    50                  55                  60

Leu Glu Gln Leu Tyr Asn Arg Tyr Lys Asp Pro Gln Asp Glu Asn Lys
65                  70                  75                  80

Ile Gly Ile Asp Gly Ile Gln Gln Phe Cys Asp Asp Leu Ala Leu Asp
                85                  90                  95

Pro Ala Ser Ile Ser Val Leu Ile Ile Ala Trp Lys Phe Arg Ala Ala
            100                 105                 110

Thr Gln Cys Glu Phe Ser Lys Gln Glu Phe Met Asp Gly Met Thr Glu
        115                 120                 125

Leu Gly Cys Asp Ser Ile Glu Lys Leu Lys Ala Gln Ile Pro Lys Met
    130                 135                 140

Glu Gln Glu Leu Lys Glu Pro Gly Arg Phe Lys Asp Phe Tyr Gln Phe
145                 150                 155                 160

Thr Phe Asn Phe Ala Lys Asn Pro Gly Gln Lys Gly Leu Asp Leu Glu
                165                 170                 175

Met Ala Ile Ala Tyr Trp Asn Leu Val Leu Asn Gly Arg Phe Lys Phe
            180                 185                 190

Leu Asp Leu Trp Asn Lys Phe Leu Leu Glu His His Lys Arg Ser Ile
        195                 200                 205

Pro Lys Asp Thr Trp Asn Leu Leu Leu Asp Phe Ser Thr Met Ile Ala
    210                 215                 220

Asp Asp Met Ser Asn Tyr Asp Glu Glu Gly Ala Trp Pro Val Leu Ile
225                 230                 235                 240

Asp Asp Phe Val Glu Phe Ala Arg Pro Gln Ile Ala Gly Thr Lys Ser
                245                 250                 255

Thr Thr Val
```

Figure 22b

## SEQ ID No.03

```
ctggaggaca ccaac atg aac aag ttg aaa tca tcg cag aag gat aaa gtt       51
                 Met Asn Lys Leu Lys Ser Ser Gln Lys Asp Lys Val
                  1               5                  10

cgt cag ttt atg atc ttc aca caa tct agt gaa aaa aca gca gta agt        99
Arg Gln Phe Met Ile Phe Thr Gln Ser Ser Glu Lys Thr Ala Val Ser
        15                  20                  25

tgt ctt tct caa aat gac tgg aag tta gat gtt gca aca gat aat ttt       147
Cys Leu Ser Gln Asn Asp Trp Lys Leu Asp Val Ala Thr Asp Asn Phe
    30                  35                  40

ttc caa aat cct gaa ctt tat ata cga gag agt gta aaa gga tca ttg       195
Phe Gln Asn Pro Glu Leu Tyr Ile Arg Glu Ser Val Lys Gly Ser Leu
45                  50                  55                  60

gac agg aag aag tta gaa cag ctg tac aat aga tac aaa gac cct caa       243
Asp Arg Lys Lys Leu Glu Gln Leu Tyr Asn Arg Tyr Lys Asp Pro Gln
                65                  70                  75

gat gag aat aaa att gga ata gat ggc ata cag cag ttc tgt gat gac       291
Asp Glu Asn Lys Ile Gly Ile Asp Gly Ile Gln Gln Phe Cys Asp Asp
            80                  85                  90

ctg gca ctc gat cca gcc agc att agt gtg ttg att att gcg tgg aag       339
Leu Ala Leu Asp Pro Ala Ser Ile Ser Val Leu Ile Ile Ala Trp Lys
        95                 100                 105

ttc aga gca gca aca cag tgc gag ttc tcc aaa cag gag ttc atg gat       387
Phe Arg Ala Ala Thr Gln Cys Glu Phe Ser Lys Gln Glu Phe Met Asp
    110                 115                 120

ggc atg aca gaa tta gga tgt gac agc aca gaa aaa cta aag gcc cag       435
Gly Met Thr Glu Leu Gly Cys Asp Ser Thr Glu Lys Leu Lys Ala Gln
125                 130                 135                 140

ata ccc aag atg gaa caa gaa ttg aaa gaa cca gga cga ttt aag gat       483
Ile Pro Lys Met Glu Gln Glu Leu Lys Glu Pro Gly Arg Phe Lys Asp
                145                 150                 155

ttt tac cag ttt act ttt aat ttt gca aag aat cca gga caa aaa gga       531
Phe Tyr Gln Phe Thr Phe Asn Phe Ala Lys Asn Pro Gly Gln Lys Gly
            160                 165                 170

tta gat cta gaa atg gcc att gcc tac tgg aac tta gtg ctt aat gga       579
Leu Asp Leu Glu Met Ala Ile Ala Tyr Trp Asn Leu Val Leu Asn Gly
        175                 180                 185

aga ttt aga ctc tta gac tta tgg aat aaa ttt ttg ttg gaa cat cat       627
Arg Phe Arg Leu Leu Asp Leu Trp Asn Lys Phe Leu Leu Glu His His
    190                 195                 200

aaa cga tca ata cca aaa gac act tgg aat ctt ctt tta gac ttc agt       675
Lys Arg Ser Ile Pro Lys Asp Thr Trp Asn Leu Leu Leu Asp Phe Ser
205                 210                 215                 220

acg atg att gca gat gac atg tct aat tat gat gaa gaa gga gca tgg       723
Thr Met Ile Ala Asp Asp Met Ser Asn Tyr Asp Glu Glu Gly Ala Trp
                225                 230                 235

cct gtt ctt att gat gac ttt gtg gaa ttt gca cgc cct caa att gct       771
Pro Val Leu Ile Asp Asp Phe Val Glu Phe Ala Arg Pro Gln Ile Ala
            240                 245                 250

ggg aca aaa agt aca aca gtg tag cactaaagga accttctaga atgtacatag      825
Gly Thr Lys Ser Thr Thr Val  *
        255

tctgtacaat aaatacaaca gaaaattgca cagtcaattt ctgctggctg g              876
```

Figure 22c

SEQ ID No. 04

Met Asn Lys Leu Lys Ser Ser Gln Lys Asp Lys Val Arg Gln Phe Met
1               5                   10                  15

Ile Phe Thr Gln Ser Ser Glu Lys Thr Ala Val Ser Cys Leu Ser Gln
            20                  25                  30

Asn Asp Trp Lys Leu Asp Val Ala Thr Asp Asn Phe Phe Gln Asn Pro
        35                  40                  45

Glu Leu Tyr Ile Arg Glu Ser Val Lys Gly Ser Leu Asp Arg Lys Lys
    50                  55                  60

Leu Glu Gln Leu Tyr Asn Arg Tyr Lys Asp Pro Gln Asp Glu Asn Lys
65                  70                  75                  80

Ile Gly Ile Asp Gly Ile Gln Gln Phe Cys Asp Asp Leu Ala Leu Asp
                85                  90                  95

Pro Ala Ser Ile Ser Val Leu Ile Ile Ala Trp Lys Phe Arg Ala Ala
            100                 105                 110

Thr Gln Cys Glu Phe Ser Lys Gln Glu Phe Met Asp Gly Met Thr Glu
        115                 120                 125

Leu Gly Cys Asp Ser Thr Glu Lys Leu Lys Ala Gln Ile Pro Lys Met
    130                 135                 140

Glu Gln Glu Leu Lys Glu Pro Gly Arg Phe Lys Asp Phe Tyr Gln Phe
145                 150                 155                 160

Thr Phe Asn Phe Ala Lys Asn Pro Gly Gln Lys Gly Leu Asp Leu Glu
                165                 170                 175

Met Ala Ile Ala Tyr Trp Asn Leu Val Leu Asn Gly Arg Phe Arg Leu

Leu Asp Leu Trp Asn Lys Phe Leu Leu Glu His His Lys Arg Ser Ile
        195                 200                 205

Pro Lys Asp Thr Trp Asn Leu Leu Leu Asp Phe Ser Thr Met Ile Ala
    210                 215                 220

Asp Asp Met Ser Asn Tyr Asp Glu Glu Gly Ala Trp Pro Val Leu Ile
225                 230                 235                 240

Asp Asp Phe Val Glu Phe Ala Arg Pro Gln Ile Ala Gly Thr Lys Ser
                245                 250                 255

Thr Thr Val

Figure 22d

(A) 339isiRNA (SEQ ID No. 05)

gatcCCCGTTCAGAGCAGCAACACAGTTCAAGAGACTGTGTTGCTGCTCTGAA
CTTTTTGGAAA

(B) ConisiRNA (SEQ ID No.06)

gatcCCCCGTCTACCTACACTCCCTCTTCAAGAGAGAGGGAGTGTAGGTAGAC
GTTTTTA

Figure 23

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0571410 A
- WO 9213943 A
- US 10361725 B
- US 20040009541 A


### Non-patent literature cited in the description


- **Sambrook et al.** Molecular Cloning, A Laboratory Manual. Cold Spring Harbor Laboratory Press, 1989
- **BL Liu ; M Robinson ; Z-Q Han ; RH Branston ; C English ; Preay ; Y McGrath ; SK Thomas ; M Thornton ; P Bullock.** *Gene Therapy,* 2003, vol. 10, 292-303
- **A Dolan ; E Mckie ; AR Maclean ; DJ McGeoch.** *Journal of General Virology,* 1992, vol. 73, 971-973
- **Aidan Dolan ; Fiona E Jamieson ; Charles Cunnigham ; Barbara C Barnett ; Duncan J McGeoch.** *Journal of Virology,* March 1998, 2010-2021
- **Joany Chou ; Earl R Kern ; Richard J Whitley ; Bernard Roizman.** *Science,* 1990, vol. 250, 1262-1265
- **Coffin RS ; MacLean AR ; Latchman DS ; Brown SM.** *gene therapy,* October 1996, vol. 3 (10), 886-91
- **McKie EA ; Hope RG ; Brown SM ; Maclean AR.** *Journal of General Virology,* April 1994, vol. 75, 733-41
- **McKay EM ; McVey B ; Marsden HS ; Brown SM ; MacLean AR.** *Journal of general Virology,* November 1993, vol. 74, 2493-7
- **Joany Chou ; Bernard Roizman.** *Journal of Virology,* March 1990, 1014-1020
- **Green, N.K. ; Youngs, D.J ; J.P. Neoptolemos ; F. Friedlos ; R.J. Knox ; C.J. Springer ; G.M. Anlezark ; N.P. Michael ; R.G. Melton ; M.J. Ford.** *Cancer Gene Therapy,* 1997, vol. 4, 229-238
- **Cherry L. Estilo ; Pornchai O-charoenrat ; Ivan Nagai ; Snehal G. Patel ; Pabbathi G. Reddy ; Su Dao ; Ashok R. Shaha ; Dennis H. Kraus ; Jay O. Boyle ; Richard J. Wong.** *Clinical Cancer Research,* June 2003, vol. 9, 2300-2306
- **Ganly I ; Kaye SB.** Recurrent head and neck cancer-current therapy and future prospects. *Annals of Oncology,* 2000, vol. 11, 1-6
- **Ganly I ; Soutar DS ; Kaye SB.** Current role of gene therapy in head and neck cancer. *European J of Surgical Oncology,* 2000, vol. 26, 338-343
- **Ganly I ; Kirn D ; Soutar D ; Eckardt G ; Otto R ; Robertson AG ; Park O ; Heise C ; Von Hoff DD ; Kaye SB.** A phase I study of Onyx-015, an E1B attenuated adenovirus, administered intratumourally in patients with recurrent tumours of the head and neck. *Clinical Cancer Research,* 2000, vol. 6, 798-806
- **Khuri F ; Nemunaitis J ; Ganly I ; Arseneau J ; Tannock I ; Romel L ; Gore M ; Ironside J ; Heise C ; Randley B.** Controlled trial of intratumoural ONYX-015, a selectively replicating adenovirus, in combination with cisplatin and 5-fluorouracil in patients with recurrent head and neck cancer. *Nature Medicine,* 2000, vol. 6 (8), 879-885
- **Heise C ; Sampson-Johannes A ; Williams A ; McCormick F ; Von Hoff D ; Kirn DH.** Onyx -015 an E1B gene attenuated adenovirus causes tumour specific cytolysis and antitumoural dfficacy that canb e augmented by standard chemotherpeutic agents. *Nature Medicine,* 1991, vol. 3, 639-645
- **MacLean AR ; Fareed MU ; Robertson L ; Harland J ; Brown SM.** Herpes simplex virus type 1 deletion variants 1714 and 1716 pinpoint neurovirulence related sequences in Glasgow strain 17+ between immediate early gene 1 and the 'a' sequence. *Journal of General Virology,* 1991, vol. 72, 631-639
- **Brown SM ; Harland J ; MacLean AR ; Podlech J ; Clements JB.** Cell type and cell state determine differentiated in vitro growth of non-neurovirulent ICP34.5 negative herpes simplex virus. *Journal of General Virology,* 1994, vol. 75, 2367-2377
- **McKie EA ; MacLean AR ; Lewis AD ; Cruickshank G ; Rampling R ; Barnett SC ; Kennedy PGE ; Brown SM.** Selective in vitro replication of herpes simplex virus type 1 (HSV1), ICP34.5 null mutants in primary human CNS tumours-evaluation of a potentially effective clinical therapy. *British Journal of Cancer,* 1996, vol. 74, 745-752
- **Randazzo B ; Kesari S ; MacLean AR ; Brown SM ; Fraser NW.** Treatment of experimental intracranial murine melanoma with the neuroattenuated HSV1 mutant 1716. *Virology,* 1995, vol. 211, 94-101

- **Kesari S ; Randazzo BP ; Valyi-Nagy T ; Huang QS ; Brown SM ; MacLean AR ; Lee VM-Y ; Trojanowski JQ ; Fraser NW.** A mutant herpes simplex virus replicates in brain tumours but not in neurons derived from a human embryonal carcinoma cell line. *Laboratory Investigation,* 1995, vol. 73, 636-648
- **Randazzo BP ; Kucharczuk JC ; Litzky LA ; Kaiser LR ; Brown SM ; MacLean AR ; Albelda SM ; Fraser NW.** Herpes simplex 1716 - an ICP34.5 mutant is severely replication restricted in human skin xenografts in vivo. *Virology,* 1996, vol. 223, 392-396
- **Lasner TM ; Kesari S ; Brown SM ; Lee VM-Y ; Fraser NW ; Trojanowski JQ.** Herpes simplex virus type 1 (HSV1) mutants for the treatment of childhood brain tumours. *Journal of Neuropathology and Experimental Neurology,* 1996, vol. 55, 1259-1269
- **Kucharczuk JC ; Randazzo B ; Chang MY ; Amin KM ; Elshami AA ; Sterman DH ; Rizk NP ; Molnar-Kimber KL ; Brown SM ; MacLean AR.** Use of a "replication restricted" herpes virus to treat experimental human malignant mesothelioma. *Cancer Research,* 1997, vol. 57, 466-471
- **Randazzo BP ; Bhat MG ; Kesari S ; Fraser NW ; Brown SM.** Treatment of experimental subcutaneous human melanoma with a replication restricted herpes simplex virus mutant. *Journal of Investigative Dermatology,* 1997, vol. 108, 933-937
- **Rampling R ; Cruickshank G ; Papanastassiou V ; Nicoll J ; Hadley D ; Petty R ; Maclean A ; Harland J ; McKie E ; Mabbs R.** Toxicity evaluation of replication competent herpes simplex virus (ICP 34.5 null mutant 1716) in patients with recurrent malignant glioma. *Gene Therapy,* 2000, vol. 7, 859-866